# EUROPEAN PATENT SPECIFICATION

(11) **EP 4 125 825 B1**
(45) Date of publication and mention of the grant of the patent: **31.01.2024**
(21) Application number: 21712892.5
(22) Date of filing: 24.03.2021
(51) Int. Cl.: A61K 9/50, A61K 31/4402, A61K 31/4415, A61K 9/48

(54) **A MODIFIED RELEASE MULTIPLE UNIT ORAL DOSAGE FORM OF DOXYLAMINE SUCCINATE AND PYRIDOXINE HYDROCHLORIDE AND A PROCESS FOR ITS PREPARATION**
ORALE MEHRFACHEINHEITSDOSIERUNGSFORM MIT MODIFIZIERTER FREISETZUNG VON DOXYLAMINSUCCINAT UND PYRIDOXINHYDROCHLORID UND VERFAHREN ZU IHRER HERSTELLUNG
FORME POSOLOGIQUE ORALE À UNITÉS MULTIPLES ET À LIBÉRATION MODIFIÉE DE SUCCINATE DE DOXYLAMINE ET D'HYDROCHLORURE DE PYRIDOXINE ET SON PROCÉDÉ DE PRÉPARATION

(30) Priority: 25.03.2020 EP 20382227
(43) Date of publication of application: 08.02.2023
(73) Proprietor: Italfarmaco SpA, 20126 Milano (IT)
(72) Inventor: SAURA I VALLS, Marc, 08024 BARCELONA (ES); NEBOT TROYANO, Joaquín, 08750 MOLINS DE REI (ES); ROCA I JUANES, Ramon M., 08014 BARCELONA (ES); MALDONADO VILLEGAS, Adrià, 08470 SANT CELONI (ES); COLOMBO, Giuseppe, 20831 SEREGNO (IT)
(74) Representative: Dragotti & Associati S.R.L.
(86) International application number: PCT/EP2021/057555
(87) International publication number: WO 2021/191268

(56) References cited:
- EP-A1- 3 628 311
- WO-A1-2016/029290
- US-A1- 2007 141 147
- INIBSA GINECOLOGÍA: "SPC Cariban 10mg/10mg modified release capsules", INTERNET CITATION, 1 March 2016 (2016-03-01), XP002789526, Retrieved from the Internet: URL:https://inibsa.com/wp-content/uploads/ 2016/10/En-spc-V1.pdf [retrieved on 2019-03-07] cited in the application

## Description

This application claims the benefit of European Patent Application EP20382227.5 filed March 25th, 2020.

The present invention relates to a modified release multiple unit oral dosage form of modified release pellets of doxylamine or a pharmaceutically acceptable salt thereof and modified release pellets of pyridoxine or a pharmaceutically acceptable salt thereof, and a process for its preparation.

### Background Art

Doxylamine is the International Non-proprietary Name of (*RS*)-*N,N*-dimethyl-2-(1-phenyl-1-pyridin-2-yl-ethoxy)-ethanamine having the CAS number 469-21-6. Doxylamine is the first-generation of antihistamine that competitively, reversibly and non-specifically blocks H1 receptors and is also a non-specific antagonist that can block other receptors such as central or peripheral muscarinic receptors, with marked anticholinergic activity. It is commonly used in form of a salt and particularly in form of its succinate salt (cf. European Pharmacopoeia 10.0. doxylamine hydrogen succinate monograph pp.2476-2477). The structure of doxylamine corresponds to the formula (I):

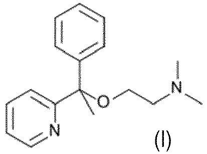

On the other hand, pyridoxine also known as vitamin B₆ is the International Non-proprietary Name of 4.5-Bis(hydroxymethyl)-2-methylpyridin-3-ol having the CAS number 65-23-6. Pyridoxine is a water-soluble vitamin factor whose active form is pyridoxal phosphate. It acts as an enzyme co-factor in numerous biochemical reactions involved in the digestive breakdown of proteins and amino acids and, to a lesser extent, lipids, and carbohydrates. It is also involved in the metabolism of unsaturated fatty acids and it is also a coenzyme for transaminases and decarboxylases allowing the conversion of tryptophan into nicotinic acid. Pyridoxine is commonly used in form of a salt and particularly in form of its hydrochloride salt (cf. European Pharmacopoeia 10.0. pyridoxine hydrochloride monograph pp.3676-3677). The structure of pyridoxine corresponds to the formula (II):

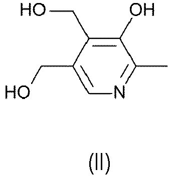

Doxylamine is commonly used by itself as a short-term sedative and also in combination with other drugs to provide night-time allergy and cold relief. Doxylamine is also used in combination with the analgesics paracetamol (acetaminophen) and codeine as an analgesic/calmative preparation, and is prescribed in combination with pyridoxine to prevent morning sickness in pregnant women.

Modified release oral dosage forms of doxylamine succinate and pyridoxine hydrochloride with different pharmacokinetic and pharmacological properties have been disclosed in the state of the art.

Several dual release oral dosage forms of doxylamine succinate and pyridoxine hydrochloride and processes for their preparation have been disclosed in the state of the art. These dual release oral dosage forms are formed by at least one immediate release composition and at least one modified release composition having each one of composition one or more of the active ingredients. This dosage system allows having an immediate release of one of the active ingredients and a modified release of the other active ingredients separately (cf. WO2013123569 and WO2016029290).

On the other hand, hard capsules filled with modified release pellets of doxylamine succinate and modified release pellets of pyridoxine hydrochloride are marketed with the name of Cariban (INIBSA GINECOLOGIA: "SPC Cariban 10mg/10mg modified release capsules", internet citation, 1 March 2016, available at the following URL address: https://inibsa.com/wp-content/uploads/2016/10/En-spc-V1.pdf and retrieved on 2019-03-07)). Cariban is used for the symptomatic treatment of nausea and vomiting. In fact, Cariban is indicated for the symptomatic treatment of nausea and vomiting during pregnancy (NVP) who do not respond to conservative management. In particular, the effect of doxylamine and pyridoxine begins to be noted five hours after ingestion, which is advantageous because allows a prolonged therapeutic effect and a reduction of the drug intakes. The therapeutic effect of the multiple unit dosage forms is conditioned by the dissolution profile of the active ingredients from each one of the pellets and in turn from the inter-pellets' homogeneity.

It is known in the state of the art that a lack of inter-pellets homogeneity can affect the dissolution profile of the active ingredients and also compromise the dosification of these pellets in the final multiple unit oral dosage form and its uniformity.

Therefore, from what is known in the art it is derived that there is still the need of providing a modified release multiple unit oral dosage form comprising homogeneous plurality of modified release pellets of both active ingredients doxylamine and pyridoxine, exhibiting the appropriate dissolution profile for being used in therapy.

### Summary of Invention

Inventors have found that the multiple unit oral dosage form of the present invention which comprises the claimed first plurality of modified release pellets of doxylamine or a pharmaceutically acceptable salt thereof; and the claimed second plurality of modified release pellets of pyridoxine or a pharmaceutically acceptable salt thereof allows having a modified sustained release of both active ingredients for at least 8 hours after administration. It is advantageous because the modified sustained release composition of the present invention has a dual effect, one immediate just after the administration and a prolonged release for the entire day, particularly after getting up.

Without being bound to any theory, the inventors surprisingly found that the use of pharmaceutically acceptable inert nucleus having a particle size such that at least 90% of the inert nuclei have a particle size from 300 µm to 1700 µm, and that at least the 90% of the pharmaceutically acceptable inert nuclei have a particle size variability of not more than 200 µm allows obtaining each one of the pellets of the first and the second plurality of pellets with a determinate particle size. It implies that a high inter-pellet homogeneity of the content of the active ingredient is maintained without compromising the target dissolution profile. It also implies that variability of the dissolution profile among different unit dosages of the multiple unit oral dosage form of the present invention is reduced. Also, it implies that the use of the claimed pharmaceutically acceptable inert nucleus also minimize the formation of aggregates and/or powdered mixtures.

In particular, the inventors have surprisingly observed the above-mentioned effects by the use of pharmaceutically acceptable inert nucleus having a particle size such that at least 90% of the inert nuclei have a particle size from 300 µm to 1700 µm, and that at least the 90% of the pharmaceutically acceptable inert nuclei have a particle size variability of not more than 150 µm, of not more than 100 µm and also of not more than 75 µm.

Furthermore, the high homogeneity of the particle size (expressed by the low particle size variability of the pharmaceutically inert nucleus) of both plurality of pellets implies that these pellets are easier to handle allowing a high uniformity in dosification and reducing the stucking of dosing machine and other problems related to the dosification (for example reducing the frequency of need to re-adjust within acceptable range the filling weight with consequent less production interruptions). It means that the machine occupation is reduced and therefore, the cost for the preparation of the multiple unit dosage form containing them is also reduced.

As it is mentioned above, the multiple unit oral dosage form of the present invention comprises a two plurality of pellets having a high homogeneity of the content of active ingredient. It means that each pellet has about the same amount of active ingredient and then, it assures that the multiple unit oral dosage form comprising those pellets always have the same therapeutically effective amount of doxylamine or a pharmaceutically acceptable salt thereof, and pyridoxine or a pharmaceutically acceptable salt thereof. It means that infra- and supra dosification of active ingredients in the multiple unit oral dosage is minimized by using the process of the present invention.

Furthermore, inventors have found that the process for the preparation of the homogeneous batches of the multiple unit oral dosage of the present invention is cheaper, more robust, reproducible and ease to scale-up in comparison with the processes of the state of the art. In fact, the process of the invention allows obtaining the homogenous batches of both plurality of pellets of both active ingredients and the preparation of the multiple unit oral dosage form in a high yield without the need of intermediate sieving steps during the process of preparation of both pluralities of pellets to remove undesired powderish material and aggregates, reducing to a minimum level also the loss of yield in an optional final sieving step, or even being able to perform the process of preparation of both pluralities of pellets without the need of any of these additional undesirable sieving steps. Finally, the inventors have found that this process allows preparing the modified release coating of the both plurality of pellets of the present invention which exhibits the target dissolution profile.

Then, the first aspect of the invention relates to a modified release multiple unit oral dosage form comprising:
a first plurality of modified release pellets of doxylamine or a pharmaceutically acceptable salt thereof comprising:
   - a pharmaceutically acceptable inert nucleus;
   - an inner active coating layer comprising a therapeutically effective amount of doxylamine or a pharmaceutically acceptable salt thereof, one or more coating agents, one or more anticaking agents, optionally one or more pore-forming agent; and optionally one or more pharmaceutically acceptable excipients;
   - optionally an intermediate enteric release coating layer comprising one or more enteric coating agents, one or more anticaking agents, optionally one or more pore-forming agent; and optionally one or more pharmaceutically acceptable excipients, and
   - an external modified release coating layer comprising one or more enteric coating agents, one or more modified release coating agents, one or more anticaking agents, optionally one or more pore-forming agents, and optionally one or more pharmaceutically acceptable excipients; and
a second plurality of modified release pellets of pyridoxine or a pharmaceutically acceptable salt thereof comprising:
   - a pharmaceutically acceptable inert nucleus;
   - an inner active coating layer comprising a therapeutically effective amount of pyridoxine or a pharmaceutically acceptable salt thereof, one or more coating agents, and optionally one or more pharmaceutically acceptable excipients; and
   - an external modified release coating layer comprising one or more enteric coating agents, one or more modified release coating agents, optionally one or more pore-forming agents, and optionally one or more pharmaceutically acceptable excipients;
wherein:
   the particle size of the pharmaceutically acceptable inert nucleus of the first and the second plurality of pellets is such that at least 90% of the inert nuclei have a particle size from 300 µm to 1700 µm measured by analytical sieving and at least the 90% of inert nuclei have a particle size variability of not more than 200 µm from a given value comprised from 500 µm and 1400 µm
   measured by analytical sieving; wherein the particle size variability means that from a given value, at least 90% of the pharmaceutically acceptable inert nuclei of the first and second plurality of pellets have a particle size comprised from ±200 µm from the given value.

Optionally at least 90% of the pharmaceutically acceptable inert nuclei of the first and second plurality of pellets have a particle size variability of not more than ±150 µm; particularly of not more than 100 µm and particularly of not more than 75 µm.

Particularly, the present invention relates to a modified release multiple unit oral dosage form comprising:
a first plurality of modified release pellets of doxylamine or a pharmaceutically acceptable salt thereof comprising:
   - a pharmaceutically acceptable inert nucleus;
   - an inner active coating layer comprising a therapeutically effective amount of doxylamine or a pharmaceutically acceptable salt thereof, one or more coating agents, one or more anticaking agents, optionally one or more pore-forming agent; and optionally one or more pharmaceutically acceptable excipients;
   - an intermediate enteric release coating layer comprising one or more enteric coating agents, one or more anticaking agents, optionally one or more pore-forming agent; and optionally one or more pharmaceutically acceptable excipients, and
   - an external modified release coating layer comprising one or more enteric coating agents, one or more modified release coating agents, one or more anticaking agents, optionally one or more pore-forming agents, and optionally one or more pharmaceutically acceptable excipients; and
a second plurality of modified release pellets of pyridoxine or a pharmaceutically acceptable salt thereof comprising:
   - a pharmaceutically acceptable inert nucleus;
   - an inner active coating layer comprising a therapeutically effective amount of pyridoxine or a pharmaceutically acceptable salt thereof, one or more coating agents, and optionally one or more pharmaceutically acceptable excipients; and
   - an external modified release coating layer comprising one or more enteric coating agents, one or more modified release coating agents, optionally one or more pore-forming agents, and optionally one or more pharmaceutically acceptable excipients;
wherein:
   the particle size of the pharmaceutically acceptable inert nucleus of the first and the second plurality of pellets is such that at least 90% of the inert nuclei have a particle size from 300 µm to 1700 µm measured by analytical sieving and at least the 90% of inert nuclei have a particle size variability of not more than 200 µm from a given value comprised from 500 µm and 1400 µm
   measured by analytical sieving; wherein the particle size variability means that from a given value, at least 90% of the pharmaceutically acceptable inert nuclei of the first and second plurality of pellets have a particle size comprised from ±200 µm from the given value. Optionally at least 90% of the pharmaceutically acceptable inert nuclei of the first and second plurality of pellets have a particle size variability of not more than ±150 µm;
   particularly of not more than 100 µm and particularly of not more than 75 µm.

The second aspect of the invention relates to a process for the preparation of the modified release multiple unit oral dosage form as defined in the first aspect of the invention comprising:
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by adding the one or more enteric coating agents, the one or more modified release coating agents, the one or more anticaking agents, optionally one or more pore-forming agent, and optionally one or more pharmaceutically acceptable excipients, wherein the particle size of the pharmaceutically acceptable inert nucleus is such that at least 90% of the inert nuclei have a particle size from 300 µm to 1700 µm measured by analytical sieving and at least the 90% of the pharmaceutically acceptable inert nuclei have a particle size variability of not more than 200 µm measured by analytical sieving; particularly of not more than 150 µm; particularly of not more than 100 µm and particularly of not more than 75 µm;
   and
(b1) preparing the second plurality of modified release pellets of pyridoxine or a pharmaceutically acceptable salt thereof by coating the pellets of pyridoxine or a pharmaceutically acceptable salt thereof having the inner active coating layer by adding the one or more enteric coating agents, the one or more modified release coating agents, optionally the one or more pore-forming agents, and optionally one or more pharmaceutically acceptable excipients, wherein the particle size of the pharmaceutically acceptable inert nucleus is such that at least 90% of the inert nuclei have a particle size from 300 µm to 1700 µm measured by analytical sieving and at least the 90% of the pharmaceutically acceptable inert nuclei have a particle size variability of not more than 200 µm measured by analytical sieving; particularly of not more than 150 µm; particularly of not more than 100 µm and particularly of not more than 75 µm.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

For the purposes of the present invention, any ranges given include both the lower and the upper end-points of the range. Ranges and values given, such as temperatures, times, and the like, should be considered approximate, unless specifically stated.

The terms "percentage (%) by weight" or "percentage (%) w/w" have the same meaning and are used interchangeable. This term refers to the percentage of a component in relation to the total weight.

The term "about" or "around" as used herein refers to a range of values ± 10% of a specified value. For example, the expression "about 10" or "around 10" includes ± 10% of 10, i.e., from 9 to 11.

The terms "relation" and "relationship" have the same meaning and are used interchangeable. This term is used in the present invention for having the relation between the spray average flow rate of the mixture comprising the coating agents and the average of the solid addition rate of the mixture in solid form.

The term "multiple unit dosage form" defines a dosage form which consists of more than one unit which contains the effective amount of doxylamine and pyridoxine. Usually, the multiple unit dosage forms are based on subunits such as granules, pellets or minitablets. They are usually delivered in hard gelatine capsules or transformed into tablets.

As it is known in the state of the art, the term "administration" refers to a method of delivering a formulation to a desired site. The terms "oral" and "oral administration" have the same meaning and they are used interchangeable. Specifically, they refer to ingesting a drug by swallowing or chewing. Preferably, by swallowing.

As it is disclosed above, the multiple unit dosage form of the present invention including two plurality of pellets. The term "pellet" refers to small particles with approximately uniform shapes and sizes produced by an extrusion process or by coating of pharmaceutically acceptable inert nucleus. A "small particle" refers to a particle of which diameter, length, height, width, or the like is from 100 µm to 3000 µm, particularly from 300 µm to 1700. Small particles have approximately uniform sizes of the diameter, length, height, width, or the like of the smallest particle is at least about one half of the average diameter, length, height, width, or the like of the particles and if the diameter, length, height, width, or the like of the largest particle is at most about twice the average diameter, length, height, width, or the like of the particles. Then, the term "pellet", "spherical pellet", "beads", "beadlets", "spherical particles", "spheroids" and "microspheres" have the same meaning and are used interchangeable. The term "granule" refers to small particles without approximately uniform shapes and sizes obtained by a granulation process. Generally, granules are less uniform in size or shape than pellets. Thus, granules have a lower uniformity due to their irregular surfaces and afford unacceptable dose uniformity and an inappropriate dissolution profile. Therefore, for the purpose of the invention the term "pellet" and "granule" are not the same and they are not interchangeable.

The term "modified release" dosage form and "modified delivery dosage form" as well as "modified release" pellet and "modified delivery" pellet have the same meaning and are interchangeable. Both terms are to be understood as a dosage form or pellet that exhibits a slower release of the active agents than that of a conventional immediate release pharmaceutical composition administered by the same route. In general, the term "modified release" refers that the active ingredient is released from the pharmaceutical dosage form in a controlled, sustained, prolonged or extended release.

For the purposes of the present invention, the term "modified release" refers to a multiple unit oral dosage form that exhibits a dissolution profile according to which: from 5% to 35% by weight of doxylamine content is dissolved at 1sth in 0.1 N HCl medium (pH = 1); then, the medium is replaced by a pH = 4.5 medium (0.05 M acetate buffer) and at 4th h from an accumulated more than 35% to 75% by weight of doxylamine initial content is dissolved; then, the medium is replaced by a pH = 6.8 medium (0.05 M phosphate buffer) and at 7th h at least an accumulated more than 75% by weight of doxylamine initial content is dissolved; and from 5% to 35% by weight of pyridoxine content is dissolved at 1sth in 0.1 N HCl medium (pH = 1); then, the medium is replaced by a pH = 4.5 medium (0.05 M acetate buffer) and at 4th h from an accumulated more than 35% to 75% by weight of pyridoxine initial content is dissolved; then, the medium is replaced by a pH = 6.8 medium (0.05 M phosphate buffer) and at 7th h at least an accumulated more than 75% by weight of pyridoxine initial content is dissolved; wherein the dissolution profile is measured using an appropriate method. Commonly an appropriate method is a USP method such as for example by using a USP type 2 apparatus (basket), placing the composition in 900mL of the corresponding media / buffered 37°C ± 0.5 °C and 100 rpm (revolution per minute) or using a USP type 3 apparatus (reciprocating cylinder), placing the composition in 250mL of the corresponding media / buffered at 37°C ± 0.5 °C and 15 dpm (dipping per minute). In the present invention, the measurement of the dissolution profile of the multiple unit oral dosage form is performed by USP type 2 apparatus (basket), placing the composition in 900mL of the corresponding media / buffered 37°C ± 0.5 °C and 100 rpm (revolution per minute).

In an embodiment, the multiple unit oral dosage form that exhibits a dissolution profile according to which: from 10% to 35% by weight of doxylamine content is dissolved at 1sth in 0.1 N HCl medium (pH = 1); then, the medium is replaced by a pH = 4.5 medium (0.05 M acetate buffer) and at 4th h from an accumulated 45% to 70% by weight of doxylamine initial content is dissolved; then, the medium is replaced by a pH = 6.8 medium (0.05 M phosphate buffer) and at 7th h at least an accumulated 80% by weight of doxylamine initial content is dissolved; and from 10% to 35% by weight of pyridoxine content is dissolved at 1sth in 0.1 N HCl medium (pH = 1); then, the medium is replaced by a pH = 4.5 medium (0.05 M acetate buffer) and at 4th h from an accumulated 40% to 65% by weight of pyridoxine initial content is dissolved; then, the medium is replaced by a pH = 6.8 medium (0.05 M phosphate buffer) and at 7th h at least an accumulated 80% by weight of pyridoxine initial content is dissolved; wherein the dissolution profile is measured using an appropriate method.

In the context of the invention, the term "coating agents" and "film-forming coating agents" have the same meaning and are used interchangeable. Both terms are to be understood as an agent capable of forming a thin coat to a solid dosage form or dosage form intermediate such as tablet and pellets. Examples of each one of the types of coating agents are disclosed below.

In an embodiment, the multiple unit oral dosage form of the present invention is one wherein the coating agent of the inner active coating layer of the first and the second plurality of pellets are independently selected from the group consisting of polyvinylpyrrolidone, hydroxypropyl cellulose, microcrystalline cellulose, calcium carbonate, carboxymethylcellulose calcium, carboxymethylcellulose sodium, carboxymethylcellulose sodium enzymatically-hydrolyzed, cellaburate, cellacefate, cellulose acetate, cetyl alcohol, chitosan, coconut oil, hydrogenated, copovidone, corn syrup solids, ethylcellulose, ethylcellulose aqueous dispersion, ethylcellulose dispersion type b, ethylene glycol and vinyl alcohol graft copolymer, gelatin, glaze pharmaceutical, glucose liquid, glyceryl behenate, glyceryl dibehenate, hydroxyethyl cellulose, hydroxypropyl cellulose, hypromellose, isomalt, alpha-lactalbumin, maltitol, maltodextrin, methacrylic acid polymers and copolymers commercially available under the trade name of Eudragit^{®} (Rohm Pharma; Westerstadt, Germany) such as Eudragit^{®} E , Eudragit^{®} NE, Eudragit^{®} NM, Eudragit^{®} RL and Eudragit^{®} RS, methylcellulose, palm kernel oil, palm oil, palm oil hydrogenated, polydextrose, polydextrose hydrogenated, polyethylene glycol, polyethylene glycol 3350, polyethylene oxide, polyvinyl alcohol, pullulan, rapeseed oil fully hydrogenated, rapeseed oil superglycerinated fully hydrogenated, shellac, starch pregelatinized modified, sucrose, sugar confectioner's, sunflower oil, titanium dioxide, wax carnauba, wax microcrystalline, xylitol and zinc oxide, alginic acid, copovidone, dibutyl phthalate, diethyl phthalate, pyroxylin, sodium alginate, and a mixture thereof. In an embodiment, the multiple unit oral dosage form of the present invention is one wherein the coating agent of the inner active coating layer of the first and the second plurality of pellets are independently selected from the group consisting of polyvinylpyrrolidone, shellac, hypromellose, hydroxypropyl cellulose, microcrystalline cellulose, and a mixture thereof.

In particular, the term "modified release coating agent" refers to an agent capable of forming films which allow the delivery of the drug at a predetermined rate and/or location according to the needs of the body and disease states for a definite time of period. Illustrative but non-limitative examples of "modified release polymers" and "modified delivery polymers" are polymers which provide a controlled release, a sustained release, a prolonged release or an extended release. Examples of modified release coating agent include without limitation acrylic polymers, celluloses and their derivatives, shellac, hydrogenated vegetable oil, hydrogenated castor oil, and their mixtures. Examples of suitable acrylic polymers include methacrylic acid polymers and copolymers commercially available under the trade name of Eudragit^{®} (Rohm Pharma; Westerstadt, Germany) such as Eudragit^{®} E, Eudragit^{®} NE, Eudragit^{®} NM, Eudragit^{®} RL and Eudragit^{®} RS. Examples of modified release coating agent include without limitation alginic acid, carbomer copolymer, carbomer homopolymer, carbomer interpolymer, carboxymethylcellulose sodium, carrageenan, cellaburate, ethylcellulose, ethylcellulose aqueous dispersion, ethylcellulose dispersion type b, glyceryl monooleate, glyceryl monostearate, guar gum, hydroxypropyl betadex, hydroxypropyl cellulose, hypromellose, polyethylene oxide, shellac, sodium alginate, starch, pregelatinized, starch, pregelatinized modified or xanthan gum. Preferably, the modified release coating agent is shellac, particularly dewaxed shellac. The modified release polymers can be accompanied by plasticizers such as triethyl citrate (TEC), polyethylene glycol (PEG), cetyl and stearyl alcohol, acetyltributyl citrate, acetyltriethyl citrate, benzyl benzoate, castor oil, chlorobutanol, diacetylated monoglycerides, dibutyl sebacate, diethyl phthalate, glycerin, mannitol, polyethylene glycol, polyethylene glycol 3350, polyethylene glycol monomethyl ether, propylene glycol, pullulan, sorbitol, sorbitol sorbitan solution, sucrose diacetate hexaisobutyrate, triacetin, tributyl citrate, triethyl citrate and vitamin E; surface-active agents such as sodium lauryl sulphate, polysorbate and poloxamer; pigments such as titanium dioxide, iron sesquioxide; lubricants such as talc, magnesium stearate,glyceryl monostearate, behenoyl polyoxylglycerides, calcium stearate, castor oil hydrogenated, coconut oil hydrogenated, glyceryl behenate, glyceryl dibehenate, glyceryl mono and dicaprylate, glyceryl mono and dicaprylocaprate, glyceryl monocaprylate, glyceryl monocaprylocaprate, glyceryl monostearate, glyceryl tricaprylate, glyceryl tristearate, lauric acid, magnesium stearate, mineral oil light, myristic acid, palm oil hydrogenated, palmitic acid, poloxamer, polyethylene glycol, polyethylene glycol 3350, polyoxyl 10 oleyl ether, polyoxyl 15 hydroxystearate, polyoxyl 20 cetostearyl ether, polyoxyl 35 castor oil, polyoxyl 40 castor oil hydrogenated, polyoxyl 40 stearate, polysorbate 20, polysorbate 40, polysorbate 60, polysorbate 80, potassium benzoate, sodium benzoate, sodium lauryl sulfate, sodium stearate, sodium stearyl fumarate, sorbitan monolaurate, sorbitan monooleate, sorbitan monopalmitate, sorbitan monostearate, sorbitan sesquioleate, sorbitan trioleate, stearic acid, stearic acid purified, sucrose stearate, talc, vegetable oil hydrogenated, type I, zinc stearate and mixtures thereof.

In an embodiment, the multiple unit oral dosage form of the present invention is one wherein the modified release coating agents are independently selected from the group consisting of polyvinylpyrrolidone and shellac, particularly polyvinylpyrrolidone and dewaxed shellac and a mixture thereof.

The term "enteric release" refers to a composition or layer of a dosage form that is formulated to release the active ingredient(s) upon exposure to a characteristic aspect of the gastrointestinal tract. In an embodiment, the enteric material is pH-sensitive and is affected by changes in pH encountered within the gastrointestinal tract (pH-sensitive release). The enteric material typically remains insoluble at gastric pH, then allows for release of the active ingredient in the higher pH environment of the downstream gastrointestinal tract (e.g., often the duodenum, or sometimes the colon). In another embodiment, the enteric material comprises enzymatically degradable polymers that are degraded by bacterial enzymes present in the lower gastrointestinal tract, particularly in the colon. Optionally, the unit dosage form is formulated with a pH-sensitive enteric material designed to result in a release within about appropriate hours when at or above a specific pH. In various embodiments, the specific pH can for example be from about 4 to about 7, such as about 4.5, 5, 5.5, 6, 6.5, 6.8 or 7. In the context of the invention, the term "enteric release coating agent" refers to an agent capable of forming films which allow the delivery of doxylamine and pyridoxine upon exposure to a characteristic aspect of the gastrointestinal tract as defined above. Materials used for enteric release formulations, for example as coatings, are well known in the art and include, but are not limited to, cellulosic polymers such as, hydroxypropyl methyl cellulose acetate succinate, hydroxypropyl methyl cellulose phthalate, cellulose acetate phthalate and cellulose acetate trimellitate;, preferably formed from acrylic acid, methacrylic acid, methyl acrylate, ethyl acrylate, methyl methacrylate and/or ethyl methacrylate, and other methacrylic resins that are commercially available under the trade-name Acryl-EZE^{®} (Colorcon, USA), Eudragit^{®} (Rohm Pharma; Westerstadt, Germany), including Eudragit^{®} L30D-55 and L100-55 (soluble at pH 5.5 and above), Eudragit^{®} L100 and L12.5 (soluble at pH 6.0 and above), Eudragit^{®} S, S12.5 and FS 30D (soluble at pH 7.0 and above, as a result of a higher degree of esterification); vinyl polymers and copolymers formed from vinyl acetate, vinyl acetate phthalate, vinyl acetate crotonic acid copolymer, and ethylene-vinyl acetate copolymer; enzymatically degradable polymers such as azo polymers; and zein. Combinations of different enteric materials may also be used. Multi-layer coatings using different polymers may also be applied. The properties, manufacture and design of enteric delivery systems are well known to those of ordinary skill in the art. In a particular embodiment, the enteric coating agent is selected from the group consisting of copolymer of methacrylic acid and methyl methacrylate, copolymer of methacrylic acid and methyl acrylate, cellulose acetate phthalate, hydroxypropyl methyl cellulose phthalate, polyvinyl acetate phthalate, cellulose acetate trimellitate and a mixture thereof. More particularly, Eudragit L^{®} such as for example Eudragit L100 (sold by Evonik). In an embodiment, the multiple unit oral dosage form of the present invention is one wherein the enteric coating agents are independently selected from the group consisting of methacrylic acid-methyl methacrylate copolymer, methacrylic acid-methyl acrylate copolymer, cellulose acetate phthalate, hydroxypropyl methyl cellulose phthalate, polyvinyl acetate phthalate, cellulose acetate trimellitate and a mixture thereof. In an embodiment, the multiple unit oral dosage form of the present invention is one wherein the enteric coating agent is a methacrylic acid-methyl methacrylate copolymer, particularly Eudragit L100.

As it is defined above, the multiple unit oral dosage form of the present invention comprises a first plurality of pellets which comprises a therapeutically effective amount of doxylamine or a pharmaceutically acceptable salt thereof and a second plurality of pellets which comprises a therapeutically effective amount of pyridoxine or a pharmaceutically acceptable salt thereof.

The term "therapeutically effective amount" as used herein, refers to the amount of an active ingredient per multiple unit dosage form that, when administered, is sufficient to prevent development of, or alleviate to some extent, one or more of the symptoms of the disease which is addressed. The particular dose of compound administered according to this invention will be determined by the particular circumstances surrounding the case, including the compound administered, the route of administration, the particular condition being treated, and the similar considerations.

The term "pharmaceutically acceptable salt(s)" used herein encompasses any salt formed from pharmaceutically acceptable non-toxic acids including inorganic or organic acids. There is no limitation regarding the salts, except that if used for therapeutic purposes, they must be pharmaceutically acceptable. As doxylamine and pyridoxine are basic compounds, salts may be prepared from pharmaceutically acceptable non-toxic acids, including inorganic and organic acids. Such acids include among others acetic, benzene sulfonic, benzoic, camphor sulfonic, citric, ethansulfonic, fumaric, gluconic, glutamic, hydrobromic, hydrochloric, lactic, maleic, malic, mandelic, methanesulfonic, phosphoric, succinic, sulphuric, tartaric, or p-toluensulfonic acid. The preparation of pharmaceutically acceptable salts of doxylamine and pyridoxine can be carried out by methods known in the art. For instance, they can be prepared from the parent compound, which contains a basic or acidic moiety, by conventional chemical methods. Generally, such salts are, for example, prepared by reacting the free acid or base forms of these compounds with a stoichiometric amount of the appropriate pharmaceutically acceptable base or acid in water or in an organic solvent or in a mixture of them. In an embodiment, the multiple unit oral dosage form of the present invention is one which comprises a pharmaceutically acceptable salt of doxylamine and a pharmaceutically acceptable salt of pyridoxine. In an embodiment, the multiple unit oral dosage form of the present invention is one which comprises doxylamine succinate and pyridoxine hydrochloride.

As it is mentioned above, the particle size of the pharmaceutically acceptable inert nucleus of the first and the second plurality of pellets is such that at least 90% of the inert nuclei have a particle size from 300 µm to 1700 µm measured by analytical sieving and at least the 90% of inert nuclei have a particle size variability of not more than 200 µm from a given value comprised from 500 µm and 1400 µm measured by analytical sieving; particularly of not more than 150 µm; particularly of not more than 100 µm; and particularly of not more than 75 µm. It means that the pharmaceutically acceptable inert nucleus of both plurality of pellets have a particle size value and a particle size variability value falling within the scope of the present invention. It encompasses that the values of the particle size and particle size variability of the first plurality of pellets and the second plurality of pellets can be equal or different, but both falling within the ranges of the present invention.

The multiple unit oral dosage form of the present invention comprises pharmaceutically acceptable inert nucleus having a particle size such that at least 90% of the inert nuclei have a particle size from 300 µm to 1700 µm measured by analytical sieving, and at least the 90% of the pharmaceutically acceptable inert nuclei have a particle size variability of not more than 200 µm from a given value comprised from 500 µm and 1400 µm measured by analytical sieving. In an embodiment, the multiple unit oral dosage form of the present invention comprises pharmaceutically acceptable inert nucleus having a particle size such that at least 90% of the inert nuclei have a particle size from 300 µm to 1400 µm measured by analytical sieving, and at least the 90% of the pharmaceutically acceptable inert nuclei have a particle size variability of not more than 150 µm from a given value comprised from 450 µm and 1250 µm measured by analytical sieving. In an embodiment, the multiple unit oral dosage form of the present invention comprises pharmaceutically acceptable inert nucleus having a particle size such that at least 90% of the inert nuclei have a particle size from 600 µm to 1180 µm measured by analytical sieving and at least the 90% of the pharmaceutically acceptable inert nuclei have a particle size variability of not more than 100 µm from a given value comprised from 710 µm and 1000 µm measured by analytical sieving. In an embodiment, the multiple unit oral dosage form of the present invention comprises pharmaceutically acceptable inert nucleus having a particle size such that at least 90% of the inert nuclei have a particle size from 710 µm to 1000 µm measured by analytical sieving and at least the 90% of the pharmaceutically acceptable inert nuclei have a particle size variability of not more than 75 µm from a given value comprised from 800 µm and 900 µm measured by analytical sieving.

The term "inert nucleus" refers to neutral microspheres which comprises in their composition at least one of the following substances: sorbitol, mannitol, sucrose, saccharose, starch, microcrystalline cellulose, lactose, glucose, trehalose, maltitol, fructose, colloidal silicon dioxide. In an embodiment, the pharmaceutically acceptable inert nucleus are neutral microspheres of a mixture of sucrose and starch.

In an embodiment, the multiple unit oral dosage form is one wherein the particle size of the pellets of the first plurality of modified release pellets of doxylamine or a pharmaceutically acceptable salt thereof is such that at least 90% of the pellets have a particle size from 600 µm to 1600 µm measured by analytical sieving and at least the 90% of the pellets have a particle size variability of not more than 150 µm from a given value comprised from 800 µm and 1400 µm measured by analytical sieving. In an embodiment, the multiple unit oral dosage form is one wherein the particle size of the pellets of the first plurality of modified release pellets of doxylamine or a pharmaceutically acceptable salt thereof is such that at least 90% of the pellets have a particle size from 710 µm to 1400 µm measured by analytical sieving and at least the 90% of the pellets have a particle size variability of not more than 100 µm from a given value comprised from 850 µm and 1250 µm measured by analytical sieving. In an embodiment, the multiple unit oral dosage form is one wherein the particle size of the pellets of the first plurality of modified release pellets of doxylamine or a pharmaceutically acceptable salt thereof is such that at least 90% of the pellets have a particle size from 800 µm to 1400 µm measured by analytical sieving and at least the 90% of the pellets have a particle size variability of not more than 75 µm from a given value comprised from 900 µm and 1180 µm measured by analytical sieving.

In an embodiment, the multiple unit oral dosage form is one wherein the particle size of the pellets of the second plurality of modified release pellets of pyridoxine or a pharmaceutically acceptable salt thereof is such that at least 90% of the pellets have a particle size from 600 µm to 1600 µm measured by analytical sieving and at least the 90% of the pellets have a particle size variability of not more than 150 µm from a given value comprised from 800 µm and 1400 µm measured by analytical sieving. In an embodiment, the multiple unit oral dosage form is one wherein the particle size of the pellets of the second plurality of modified release pellets of pyridoxine or a pharmaceutically acceptable salt thereof is such that at least 90% of the pellets have a particle size from 710 µm to 1400 µm measured by analytical sieving and at least the 90% of the pellets have a particle size variability of not more than 100 µm from a given value comprised from 850 µm and 1250 µm measured by analytical sieving. In an embodiment, the multiple unit oral dosage form is one wherein the particle size of the pellets of the second plurality of modified release pellets of pyridoxine or a pharmaceutically acceptable salt thereof is such that at least 90% of the pellets have a particle size from 800 µm to 1400 µm measured by analytical sieving and at least the 90% of the pellets have a particle size variability of not more than 75 µm from a given value comprised from 900 µm and 1180 µm measured by analytical sieving.

In an embodiment, the multiple unit oral dosage form of the present invention comprises pharmaceutically acceptable inert nucleus having a particle size such that at least 90% of the inert nuclei have a particle size from 300 µm to 1700 µm measured by analytical sieving, and at least the 90% of the pharmaceutically acceptable inert nuclei have a particle size variability of not more than 200 µm from a given value comprised from 500 µm and 1400 µm measured by analytical sieving; the particle size of the pellets of the first plurality of modified release pellets of doxylamine or a pharmaceutically acceptable salt thereof is such that at least 90% of the pellets have a particle size from 400 µm to 2000 µm measured by analytical sieving and at least the 90% of the pellets have a particle size variability of not more than 200 µm from a given value comprised from 600 µm and 1800 µm measured by analytical sieving and the particle size of the pellets of the second plurality of modified release pellets of pyridoxine or a pharmaceutically acceptable salt thereof is such that at least 90% of the pellets have a particle size from 400 µm to 2000 µm measured by analytical sieving and at least the 90% of the pellets have a particle size variability of not more than 200 µm from a given value comprised from 600 µm and 1800 µm measured by analytical sieving.

In an embodiment, the multiple unit oral dosage form of the present invention comprises pharmaceutically acceptable inert nucleus having a particle size such that at least 90% of the inert nuclei have a particle size from 300 µm to 1400 µm measured by analytical sieving and at least the 90% of the pharmaceutically acceptable inert nuclei have a particle size variability of not more than 150 µm from a given value comprised from 450 µm and 1250 µm measured by analytical sieving; the particle size of the pellets of the first plurality of modified release pellets of doxylamine or a pharmaceutically acceptable salt thereof is such that at least 90% of the pellets have a particle size from 600 µm to 1600 µm measured by analytical sieving and at least the 90% of the pellets have a particle size variability of not more than 150 µm from a given value comprised from 800 µm and 1400 µm measured by analytical sieving; and the particle size of the pellets of the second plurality of modified release pellets of pyridoxine or a pharmaceutically acceptable salt thereof is such that at least 90% of the pellets have a particle size from 600 µm to 1600 µm measured by analytical sieving and at least the 90% of the pellets have a particle size variability of not more than 150 µm from a given value comprised from 800 µm and 1400 µm measured by analytical sieving.

In an embodiment, the multiple unit oral dosage form of the present invention comprises pharmaceutically acceptable inert nucleus having a particle size such that at least 90% of the inert nuclei have a particle size from 600 µm to 1180 µm measured by analytical sieving and at least the 90% of the pharmaceutically acceptable inert nuclei have a particle size variability of not more than 100 µm from a given value comprised from 710 µm and 1000 µm measured by analytical sieving at least the 90% of the pharmaceutically acceptable inert nuclei have ; the particle size of the pellets of the first plurality of modified release pellets of doxylamine or a pharmaceutically acceptable salt thereof is such that at least 90% of the pellets have a particle size from 710 µm to 1400 µm measured by analytical sieving and at least the 90% of the pellets have a particle size variability of not more than 100 µm from a given value comprised from 850 µm and 1250 µm measured by analytical sieving; and the particle size of the pellets of the second plurality of modified release pellets of pyridoxine or a pharmaceutically acceptable salt thereof is such that at least 90% of the pellets have a particle size from 710 µm to 1400 µm measured by analytical sieving and at least the 90% of the pellets have a particle size variability of not more than 100 µm from a given value comprised from 850 µm and 1250 µm measured by analytical sieving.

In an embodiment, the multiple unit oral dosage form of the present invention comprises pharmaceutically acceptable inert nucleus having a particle size such that at least 90% of the inert nuclei have a particle size from 710 µm to 1000 µm measured by analytical sieving and at least the 90% of the pharmaceutically acceptable inert nuclei have a particle size variability of not more than 75 µm from a given value comprised from 800 µm and 900 µm measured by analytical sieving; the particle size of the pellets of the first plurality of modified release pellets of doxylamine or a pharmaceutically acceptable salt thereof is such that at least 90% of the pellets have a particle size from 800 µm to 1400 µm measured by analytical sieving and at least the 90% of the pellets have a particle size variability of not more than 75 µm from a given value comprised from 900 µm and 1180 µm measured by analytical sieving; and the particle size of the pellets of the second plurality of modified release pellets of pyridoxine or a pharmaceutically acceptable salt thereof is such that at least 90% of the pellets have a particle size from 800 µm to 1400 µm measured by analytical sieving and at least the 90% of the pellets have a particle size variability of not more than 75 µm from a given value comprised from 900 µm and 1180 µm measured by analytical sieving.

The particle size of the pharmaceutically acceptable inert nucleus, the pellets, the active ingredients and the excipients can be measured by any method disclosed in the state of the art. Examples of methods commonly used for measuring the particle size is Dynamic light scattering (DLS) reporting the number average diameter value, Atomic force microscopy (AFM) or transmission electron microscopy (TEM) to measure dried particles; laser diffraction (Laser Mastersizer, Mie Theory; ISO 13320-1) and by sedimentation analysis (Sedigraph-Stoke's Law; ISO 13317-3) and by analytical sieving. For the purpose of the present invention, the particle size of the pharmaceutically acceptable inert nucleus and of the pellets of the first and the second plurality as defined herein is measured by analytical sieving, particularly as directed by European Pharmacopoeia (cf. European Pharmacopoeia chapter 2.9.38).

The terms "variability" and "dispersion" have the same meaning and are used interchangeable. They refer to how spread out a set of data is. Variability gives you a way to describe how much data sets vary. In particular, the term "particle size variability" refers to how spread out the particle size of a given value is.

The expression "at least the 90% [...] have a particle size from X to Y µm means that the 90% of the totality of the population of particles has a particle size comprised from X and Y µm. For the purpose of the invention, the expression "at least the 90% of the pharmaceutically acceptable inert nuclei or pellets have a particle size variability of not more than 200 µm" means that from a given value, the 90% of the population of pharmaceutically acceptable inert nuclei or pellets have a particle size comprised from ± 200 µm from a given value. For instance, for the given value 500 µm, at least the 90% of the population of pharmaceutically acceptable inert nuclei have a particle size from 300 µm to 700 µm (i.e. ± 200 µm). The particle size variability of the pharmaceutically acceptable inert nuclei or the pellets can be measured by any method disclosed in the state of the art. Examples of methods commonly used for measuring the particle size variability is Dynamic light scattering (DLS) reporting the number average diameter value, Atomic force microscopy (AFM) or transmission electron microscopy (TEM) to measure dried particles; laser diffraction (Laser Mastersizer, Mie Theory; ISO 13320-1), by sedimentation analysis (Sedigraph-Stoke's Law; ISO 13317-3), by analytical sieving and optical microscopy. For the purpose of the present invention, the particle size variability is measured by analytical sieving.

In an embodiment, the multiple unit oral dosage form is one wherein the particle size of doxylamine or a pharmaceutically acceptable salt thereof is characterized for having a D90 equal to or below than 250 µm. In an embodiment, the multiple unit oral dosage form is one wherein the particle size of pyridoxine or a pharmaceutically acceptable salt thereof is characterized for having a D90 equal to or below than 250 µm. In an embodiment, the multiple unit oral dosage form is one wherein: the particle size of doxylamine or a pharmaceutically acceptable salt is characterized for having a D90 equal to or below than 250 µm; and the particle size of pyridoxine or a pharmaceutically acceptable salt thereof is characterized for having a D90 equal to or below than 250 µm.

In an embodiment, the multiple unit oral dosage form is one wherein the particle size of the one or more anticaking agent is characterized for having a D90 equal to or below than 250 µm. In an embodiment, the multiple unit oral dosage form is one wherein the particle size of the one or more optionally present pore-forming agents is characterized for having a D90 equal to or below than 250 µm.

The DX value indicates that a certain percentage X of the particles has a weight/volume/number equal to or below a certain limit. A D90 indicates that the 90% of the particles has a weight/volume/number equal to or below a certain limit. The D90 may be expressed by volume, by weight or by number. For the purpose of the present invention, the D90 of the active ingredients (doxylamine and pyridine) are expressed by volume. And, the D90 of the anticaking agents, such as talc, is expressed by weight. For instance, a D90 value of doxylamine or pyridoxine equal to or below than 250 µm means that 90 % by volume of the particles have a diameter equal to or below than 250 µm. And a D90 value of talc equal to or below 250 µm means that 90 % by weight of the particles have a diameter equal to or below than 250 µm. The DX (and particularly the D90) can be measured using any appropriate method disclosed above for the measurement of the particle size of the pharmaceutically acceptable inert nucleus, the coated pellets, the active ingredients, and the excipients of the present invention, such as for example analytical sieving. Particularly, among the suitable methods above mentioned, a Malvern method (Laser Mastersizer, Mie Theory; ISO 13320-1) is the preferable option for determining the D90 and the particle size distribution of doxylamine or a pharmaceutically acceptable salt, and also the D90 of pyridoxine or a pharmaceutically acceptable salt thereof.

In an embodiment, the multiple unit oral dosage form comprises a first plurality of pellets which comprises a therapeutically effective amount of a pharmaceutically acceptable salt of doxylamine, particularly doxylamine succinate. In an embodiment, the multiple unit oral dosage form comprises the first plurality of pellets which comprises from 5 mg to 50 mg per oral dosage form of doxylamine or a pharmaceutically acceptable salt thereof per multiple unit dosage form, particularly from 6 mg to 40 mg, from 7 mg to 30 mg, more particularly from 8 mg to 22 mg. In an embodiment, the multiple unit oral dosage form comprises the first plurality of pellets having particularly from 5 mg to 50 mg per oral dosage form of doxylamine succinate per multiple unit dosage form, particularly from 6 mg to 40 mg, from 7 mg to 30 mg, more particularly from 8 mg to 22 mg; much more particularly from 9 mg to 11 mg of doxylamine succinate. In a more particular embodiment, the multiple unit oral dosage form comprises the first plurality of pellets having 10 mg of doxylamine or a pharmaceutically acceptable salt thereof; particularly doxylamine succinate per multiple unit dosage form. In another embodiment, the multiple unit oral dosage form comprises the first plurality of pellets having particularly from 19 mg to 21 mg of doxylamine or a pharmaceutically acceptable salt thereof; particularly doxylamine succinate. In another more particular embodiment, the multiple unit oral dosage form comprises the first plurality of pellets having 20 mg of doxylamine or a pharmaceutically acceptable salt thereof; particularly doxylamine succinate per multiple unit dosage form.

In an embodiment, the multiple unit oral dosage form comprises a second plurality of pellets which comprises a therapeutically effective amount of a pharmaceutically acceptable salt of pyridoxine, particularly pyridoxine hydrochloride. In an embodiment, the multiple unit oral dosage form comprises the second plurality of pellets which comprises from 5 mg to 50 mg per oral dosage form of pyridoxine or a pharmaceutically acceptable salt thereof, per multiple unit dosage form, particularly from 6 mg to 40 mg, from 7 mg to 30 mg, more particularly from 8 mg to 22 mg. In an embodiment, the multiple unit oral dosage form comprises the second plurality of pellets having particularly from 5 mg to 50 mg per oral dosage form of pyridoxine hydrochloride per multiple unit dosage form, particularly from 6 mg to 40 mg, particularly from 7 mg to 30 mg, more particularly from 8 mg to 22 mg; much more particularly from 9 mg to 11 mg of pyridoxine hydrochloride. In a more particular embodiment, the multiple unit oral dosage form comprises the second plurality of pellets having 10 mg of pyridoxine or a pharmaceutically acceptable salt thereof; particularly pyridoxine hydrochloride per multiple unit dosage form. In another embodiment, the multiple unit oral dosage form comprises the second plurality of pellets having particularly from 19 mg to 21 mg of pyridoxine or a pharmaceutically acceptable salt thereof; particularly pyridoxine hydrochloride. In another more particular embodiment, the multiple unit oral dosage form comprises the second plurality of pellets having 20 mg of pyridoxine or a pharmaceutically acceptable salt thereof; particularly pyridoxine hydrochloride per multiple unit dosage form.

In an embodiment, the multiple unit oral dosage form comprises the first plurality of pellets which comprises doxylamine or a therapeutically effective amount of a pharmaceutically acceptable salt thereof, particularly doxylamine succinate; and the second plurality of pellets which comprises a therapeutically effective amount of pyridoxine or a pharmaceutically acceptable salt thereof, particularly the pyridoxine hydrochloride. In an embodiment, the multiple unit oral dosage form comprises the first plurality of pellets which comprises from 5 mg to 50 mg of doxylamine or a pharmaceutically acceptable salt thereof; particularly doxylamine succinate per multiple unit dosage form, particularly 10 mg or 20mg of doxylamine or a pharmaceutically acceptable salt thereof; particularly doxylamine succinate per multiple unit dosage form; and the second plurality of pellets which comprises from 5 mg to 50 mg of pyridoxine or a pharmaceutically acceptable salt thereof; particularly pyridoxine hydrochloride per multiple unit dosage form, particularly 10 mg or 20mg of pyridoxine or a pharmaceutically acceptable salt thereof; particularly pyridoxine hydrochloride per multiple unit dosage form. In a particular embodiment, the multiple unit oral dosage form comprises the first plurality of pellets which comprises 10 mg of doxylamine or a pharmaceutically acceptable salt thereof; particularly doxylamine succinate per multiple unit dosage form and the second plurality of pellets comprising 10 mg of pyridoxine or a pharmaceutically acceptable salt thereof; particularly pyridoxine hydrochloride per multiple unit dosage form. In another particular embodiment, the multiple unit oral dosage form comprises the first plurality of pellets which comprises 20 mg of doxylamine or a pharmaceutically acceptable salt thereof; particularly doxylamine succinate per multiple unit dosage form and the second plurality of pellets comprising 20 mg of pyridoxine or a pharmaceutically acceptable salt thereof; particularly pyridoxine hydrochloride per multiple unit dosage form.

In an embodiment, the multiple unit dosage form comprises from 20 mg to 220 mg of the first plurality of pellets which comprises a therapeutically effective amount of doxylamine or a pharmaceutically acceptable salt thereof, particularly doxylamine succinate. In an embodiment, the multiple unit dosage form comprises from 40 mg to 140 mg of the first plurality of pellets which comprises a therapeutically effective amount of doxylamine or a pharmaceutically acceptable salt thereof, particularly doxylamine succinate.

In an embodiment, the multiple unit dosage form comprises about 60 mg of the first plurality of pellets which comprises a therapeutically effective amount of doxylamine or a pharmaceutically acceptable salt thereof, particularly doxylamine succinate.

In another embodiment, the multiple unit dosage form comprises about 120 mg of the first plurality of pellets which comprises a therapeutically effective amount of doxylamine or a pharmaceutically acceptable salt thereof, particularly doxylamine succinate.

In an embodiment, the multiple unit dosage form comprises from 20 mg to 220 mg of the second plurality of pellets which comprises a therapeutically effective amount of pyridoxine or a pharmaceutically acceptable salt thereof, particularly pyridoxine hydrochloride. In an embodiment, the multiple unit dosage form comprises from 40 mg to 140 mg of the second plurality of pellets which comprises a therapeutically effective amount of pyridoxine or a pharmaceutically acceptable salt thereof, particularly pyridoxine hydrochloride. In an embodiment, the multiple unit dosage form comprises about 60 mg of the second plurality of pellets which comprises a therapeutically effective amount of pyridoxine or a pharmaceutically acceptable salt thereof, particularly pyridoxine hydrochloride. In another embodiment, the multiple unit dosage form comprises about 120 mg of the second plurality of pellets which comprises a therapeutically effective amount of pyridoxine or a pharmaceutically acceptable salt thereof, particularly pyridoxine hydrochloride.

In an embodiment, the multiple unit dosage form comprises from 20 mg to 220 mg of the first plurality of pellets which comprises a therapeutically effective amount of doxylamine or a pharmaceutically acceptable salt thereof, particularly doxylamine succinate; and from 20 mg to 220 mg of the second plurality of pellets which comprises a therapeutically effective amount of pyridoxine or a pharmaceutically acceptable salt thereof, particularly pyridoxine hydrochloride. In an embodiment, the multiple unit dosage form comprises from 40 mg to 140 mg, more particularly about 60mg, of the first plurality of pellets which comprises from 5 mg to 50 mg of doxylamine succinate, particularly 10 mg of doxylamine succinate; and from 40 mg to 140 mg, more particularly 60mg, of the second plurality of pellets which comprises from 5 mg to 50 mg of pyridoxine hydrochloride, particularly 10 mg of pyridoxine hydrochloride.

In another embodiment, the multiple unit dosage form comprises from 40 mg to 140 mg, more particularly about 120 mg, of the first plurality of pellets which comprises from 5 mg to 50 mg of doxylamine succinate, particularly 20 mg of doxylamine succinate; and from 40 mg to 140 mg, more particularly 120 mg, of the second plurality of pellets which comprises from 5 mg to 50 mg of pyridoxine hydrochloride, particularly 20 mg of pyridoxine hydrochloride.

In an embodiment, the multiple unit dosage form comprises the first plurality of pellets which comprises from 5 mg to 50 mg of doxylamine succinate, particularly 10 mg of doxylamine succinate; and the second plurality of pellets which comprises from 5 mg to 50 mg of pyridoxine hydrochloride, particularly 10 mg of pyridoxine hydrochloride. In a particular embodiment, the multiple unit dosage form comprises the first plurality of pellets which comprises 10 mg of doxylamine succinate and the second plurality of pellets comprising 10 mg of pyridoxine hydrochloride.

In another embodiment, the multiple unit dosage form comprises the first plurality of pellets which comprises from 5 mg to 50 mg of doxylamine succinate, particularly 20 mg of doxylamine succinate; and the second plurality of pellets which comprises from 5 mg to 50 mg of pyridoxine hydrochloride, particularly 20 mg of pyridoxine hydrochloride. In a particular embodiment, the multiple unit dosage form comprises the first plurality of pellets which comprises 20 mg of doxylamine succinate and the second plurality of pellets comprising 20 mg of pyridoxine hydrochloride.

The term "anticaking agent" or "anticaking agents" refers to any pharmaceutically acceptable agent capable to reduce surface tackiness and the incidence of nucleus or pellets sticking together during coating and consequently improve process efficiency, coating uniformity, and appearance, preventing the formation of lumps (aggregates) and allows easing packaging, transport, flowability, filling into final dosage form and consumption. Examples of anticaking agents include, but are not limited to, calcium stearate, magnesium stearate, silica, silicates, talc, flour, starch calcium phosphate tribasic, calcium silicate, cellulose powdered, magnesium oxide, magnesium silicate, magnesium trisilicate, silica dental-type, silica hydrophobic colloidal, silicon dioxide colloidal, sodium stearate and a mixture thereof. In an embodiment, the multiple unit dosage form comprises one or more anticaking agents selected from the group consisting of talc and silica (colloidal silicone dioxide; particularly Aerosil) and a mixture thereof. In an embodiment, the multiple unit dosage form comprises talc as anticaking agent. In an embodiment, the multiple unit dosage form comprises one or more anticaking agents having a particle size distribution characterized for having a D90 equal to or below than 250 µm; particularly lower than 150 µm; more particularly lower than 100 µm. In an embodiment, the multiple unit dosage form comprises one or more anticaking agents having a particle size distribution characterized for having a D90 equal to or below than 75 µm. In an embodiment, the multiple unit dosage form comprises talc as anticaking agent having a D90 particle diameter lower than 250 µm; particularly lower than 150 µm; more particularly lower than 100 µm. In an embodiment, the multiple unit dosage form comprises talc as anticaking agent having a D90 particle diameter lower than 75 µm.

The term "pore-forming agent" refers to any pharmaceutically acceptable agent capable of forming one or more pores in the shell/coating to allow the modified release of the active ingredients. The pore-forming agent can be organic or inorganic, or any combination thereof. Examples of pore-forming agent include, but are not limited to, polyethylene glycol (PEG), propylene glycol, isopropyl alcohol, glycerol, lactose, glucose, sucrose, mannitol, sorbitol, sodium chloride, potassium chloride, talc, hydroxypropyl cellulose, micronized sugar, hydroxypropyl methyl cellulose (HPMC), polyvinyl alcohols, methacrylic acid copolymers, or a mixture therefore. In an embodiment, the pore-forming agent is selected from the group consisting of talc, micronized sugar, sodium or potassium chloride and a mixture thereof. As it is mentioned above, the modified release multiple unit oral dosage form of the present invention optionally comprises one or more pore-forming agents. In an embodiment, the one or more pore-forming agents as defined above are present in the multiple unit oral dosage form. In an embodiment, the one or more pore-forming agents as defined above are absent in the multiple unit oral dosage form.

The term "pharmaceutically acceptable" refers to any composition, compound, or material suitable for use in the pharmaceutical technology. For the purpose of the present invention, the term "pharmaceutically acceptable excipients or carriers" refers to that excipients or carriers for preparing compositions with medical use. The appropriate excipients and/or carriers, and their amounts, can readily be determined by those skilled in the art according to the type of formulation being prepared. In an embodiment, the modified release multiple unit dosage form of the present invention further comprises one or more binders, glidants, fillers, lubricants, wicking agents, and mixtures thereof. In an embodiment, the process comprises preparing a multiple unit dosage form which can comprise one or more pharmaceutically acceptable excipients or carriers.

As it is mentioned above, the modified release multiple unit oral dosage form of the present invention optionally comprises one or more pharmaceutically acceptable excipients or carriers. In an embodiment, the modified release multiple unit oral dosage form comprises one or more pharmaceutically acceptable excipients or carriers.

In an embodiment, the multiple unit oral dosage form comprises: one or more pore-forming agents as defined above; and one or more pharmaceutically acceptable excipients or carriers.

The term "binder" refers to any pharmaceutically acceptable compound having binding properties. Materials commonly used as binders include microcrystalline cellulose, polyvinylpyrrolidone (also called povidone or PVP), methylcellulose polymers, hydroxyethyl cellulose, hydroxypropyl cellulose, L-hydroxypropyl cellulose (low substituted), hydroxypropyl methyl cellulose (HPMC), sodium carboxymethyl cellulose, carboxymethylene, carboxymethyl hydroxyethyl cellulose and other cellulose derivatives, starches or modified starches, polyethylene glycol (PEG) 6000, guar gum, starch or shellac and mixture thereof. Examples of binders include acacia, agar, alginic acid, calcium carbonate, calcium lactate, carboxymethylcellulose sodium, cellulose, microcrystalline cellulose, copovidone, dextrates, dextrin, ethylcellulose, gelatin, glucose liquid, glyceryl behenate, glyceryl dibehenate, guar gum, hydroxyethyl cellulose, hydroxypropyl cellulose, hydroxypropyl cellulose low-substituted, hypromellose, hypromellose acetate succinate, inulin, invert sugar, lactose monohydrate, maltodextrin, maltose, methylcellulose, polydextrose hydrogenated, polyethylene oxide, polyvinylpyrrolidone (also called povidone or PVP), pullulan, shellac, sodium alginate, starch pregelatinized, starch pregelatinized modified, starch corn, starch hydroxypropyl corn, starch pregelatinized hydroxypropyl corn, starch pea, starch hydroxypropyl pea, starch pregelatinized hydroxypropyl pea, starch potato, starch hydroxypropyl potato, starch pregelatinized hydroxypropyl potato, starch tapioca, starch wheat, starch hydrolysate hydrogenated, sucrose, sunflower oil, syrup, trehalose, vegetable oil hydrogenated, vitamin E polyethylene glycol succinate.

In an embodiment, the multiple unit oral dosage form is one wherein the pharmaceutically acceptable excipients or carriers comprises one or more binder; preferably comprises shellac and polyvinylpyrrolidone (PVP), and more particular embodiment are dewaxed shellac and PVP-K30.

The term "glidant" refers to a pharmaceutically acceptable substance which improves the flow characteristics of powder mixtures in the dry state. Materials commonly used as a glidant include magnesium stearate, silica (colloidal silicon dioxide; particularly Aerosil) or talc. In an embodiment, the multiple unit oral dosage form is one wherein the pharmaceutically acceptable excipients or carriers comprises one or more glidant; preferably comprises colloidal silicon dioxide, and more preferably Aerosil 200 Pharma.

The term "lubricant" refers to a pharmaceutically acceptable substance that prevents composition ingredients from clumping together and from sticking to the tablet punches or capsule filling machine and improves flowability of the composition mixture. Materials commonly used as a lubricant include sodium oleate, sodium stearate, sodium benzoate, sodium stearate, sodium chloride, stearic acid, sodium stearyl fumarate, calcium stearate, magnesium stearate, magnesium lauryl sulfate, sodium stearyl fumarate, sucrose esters or fatty acid, zinc, polyethylene glycol, talc, and mixtures thereof. Examples of lubricants include behenoyl polyoxylglycerides, calcium stearate, castor oil hydrogenated, coconut oil hydrogenated, glyceryl behenate, glyceryl dibehenate, glyceryl mono and dicaprylate, glyceryl mono and dicaprylocaprate, glyceryl monocaprylate, glyceryl monocaprylocaprate, glyceryl monostearate, glyceryl tricaprylate, glyceryl tristearate, lauric acid, magnesium stearate, mineral oil light, myristic acid, palm oil hydrogenated, palmitic acid, poloxamer, polyethylene glycol, polyethylene glycol 3350, polyoxyl 10 oleyl ether, polyoxyl 15 hydroxystearate, polyoxyl 20 cetostearyl ether, polyoxyl 35 castor oil, polyoxyl 40 castor oil hydrogenated, polyoxyl 40 stearate, polysorbate 20, polysorbate 40, polysorbate 60, polysorbate 80, potassium benzoate, sodium benzoate, sodium lauryl sulfate, sodium stearate, sodium stearyl fumarate, sorbitan monolaurate, sorbitan monooleate, sorbitan monopalmitate, sorbitan monostearate, sorbitan sesquioleate, sorbitan trioleate, stearic acid, stearic acid purified, sucrose stearate, talc, vegetable oil hydrogenated type I and zinc stearate. The presence of a lubricant is particularly preferred when the composition is a tablet to improve the tableting process.

The terms "filler" and "diluent" have the same meaning and are used interchangeably. They refer to any pharmaceutically acceptable excipient or carrier (material) that fill out the size of a composition, making it practical to produce and convenient for the consumer to use. Materials commonly used as filler include calcium carbonate, calcium phosphate, dibasic calcium phosphate, tribasic calcium sulfate, calcium carboxymethyl cellulose, cellulose, cellulose products such as microcrystalline cellulose and its salts, dextrin derivatives, dextrin, dextrose, fructose, lactitol, lactose, starches or modified starches, magnesium carbonate, magnesium oxide, maltitol, maltodextrins, maltose, mannitol, sorbitol, starch, sucrose, sugar, xylitol, erythritol and mixtures thereof. In an embodiment, the multiple unit oral dosage form is one wherein the pharmaceutically acceptable excipients or carriers comprises one or more fillers; preferably comprises sucrose, starch, or microcrystalline cellulose. Examples of fillers include amino methacrylate copolymer; ammonio methacrylate copolymer; ammonio methacrylate copolymer dispersion; calcium carbonate; calcium phosphate, dibasic, anhydrous; calcium phosphate, dibasic, dihydrate; calcium phosphate, tribasic; calcium sulfate; cellaburate; cellulose, microcrystalline; cellulose, silicified microcrystalline; cellulose, powdered; cellulose acetate; corn syrup; corn syrup solids; dextrates; dextrin; dextrose; dextrose excipient; erythritol; ethyl acrylate and methyl methacrylate copolymer dispersion; fructose; invert sugar; isomalt; kaolin; alpha-lactalbumin; lactitol; lactose, anhydrous; lactose, monohydrate; magnesium carbonate; magnesium oxide; maltitol; maltodextrin; maltose; mannitol; methacrylic acid and ethyl acrylate copolymer; methacrylic acid and ethyl acrylate copolymer dispersion; methacrylic acid and methyl methacrylate copolymer; polydextrose; polyethylene glycol; polyethylene glycol 3350; propylene glycol monocaprylate; pullulan; simethicone; sodium chloride; sorbitol; starch, pregelatinized; starch, pregelatinized modified; starch, corn; starch, hydroxypropyl corn; starch, pregelatinized hydroxypropyl corn; starch, pea; starch, hydroxypropyl pea; starch, pregelatinized hydroxypropyl pea; starch, potato; starch, hydroxypropyl potato; starch, pregelatinized hydroxypropyl potato; starch, tapioca; starch, wheat; starch hydrolysate, hydrogenated; sucrose; sugar, compressible; sugar, confectioner's; sugar spheres; sunflower oil; talc; trehalose; and xylitol.

The term "wicking agents" refers to the pharmaceutically acceptable material that has the ability to draw water into the porous network of a delivery device. It has the ability to undergo physisorption with water. The role of a wicking agent is to act like a carrier and facilitate the entry of water to the inner surfaces of the core. Materials commonly used as wicking agent include sodium lauryl sulfate, kaolin, titanium dioxide, alumina, bentonite, magnesium aluminium silicate, povidone, and colloidal silicon dioxide (Aerosil). In an embodiment, the multiple unit oral dosage form of the invention is one wherein the pharmaceutically acceptable excipients or carriers comprises one or more wicking agents; preferably kaolin, titanium dioxide, alumina, bentonite, magnesium aluminium silicate, povidone, and colloidal silicon dioxide (Aerosil). In an embodiment, the multiple unit oral dosage form of the invention is one wherein the pharmaceutically acceptable excipients or carriers comprises one or more wicking agents; preferably comprises povidone or colloidal silicon dioxide (Aerosil) or a mixture thereof.

In an embodiment, the multiple unit oral dosage form of the invention is one wherein the modified release multiple unit oral dosage form comprises: a first plurality of modified release pellets of doxylamine or a pharmaceutically acceptable salt thereof comprising an inner active coating layer comprising from 6 to 20 % by weight of one or more coating agents in relation to the total weight of the inner active coating layer.

In an embodiment, the multiple unit oral dosage form of the invention is one wherein the modified release multiple unit oral dosage form comprises: a first plurality of modified release pellets of doxylamine or a pharmaceutically acceptable salt thereof comprising an inner active coating layer comprising from 15 to 30 % by weight of one or more anticaking agent in relation to the total weight of the inner active coating layer.

In an embodiment, the multiple unit oral dosage form of the invention is one wherein the modified release multiple unit oral dosage form comprises: a first plurality of modified release pellets of doxylamine or a pharmaceutically acceptable salt thereof comprising an inner active coating layer comprising: from 6 to 20 % by weight of one or more coating agents in relation to the total weight of the inner active coating layer; from 15 to 30 % by weight of one or more anticaking agent in relation to the total weight of the inner active coating layer and optionally one or more pore forming agent, being the sum of the components up to 100% by weight in relation to the weight of the inner active coating layer.

In an embodiment, the multiple unit oral dosage form of the invention is one wherein the modified release multiple unit oral dosage form comprises a first plurality of modified release pellets of doxylamine or a pharmaceutically acceptable salt thereof comprising an intermediate enteric release coating layer comprising from 45 to 65 % by weight of one or more enteric coating agents in relation to the total weight of the intermediate enteric release coating layer.

In an embodiment, the multiple unit oral dosage form of the invention is one wherein the modified release multiple unit oral dosage form comprises a first plurality of modified release pellets of doxylamine or a pharmaceutically acceptable salt thereof comprising an intermediate enteric release coating layer comprising from 35 to 55 % by weight of one or more anticaking agent in relation to the total weight of the intermediate enteric release coating layer and optionally one or more pore forming agent.

In an embodiment, the multiple unit oral dosage form of the invention is one wherein the modified release multiple unit oral dosage form comprises a first plurality of modified release pellets of doxylamine or a pharmaceutically acceptable salt thereof comprising an intermediate enteric release coating layer comprising: from 45 to 65 % by weight of one or more enteric coating agents in relation to the total weight of the intermediate enteric release coating layer; from 35 to 55 % by weight of one or more anticaking agent in relation to the total weight of the intermediate enteric release coating layer and optionally one or more pore forming agent; and optionally one or more pharmaceutically acceptable excipients being the sum of the components up to 100% by weight in relation to the weight of the intermediate enteric release coating layer.

In an embodiment, the multiple unit oral dosage form of the invention is one wherein the modified release multiple unit oral dosage form comprises a first plurality of modified release pellets of doxylamine or a pharmaceutically acceptable salt thereof comprising an external modified release coating layer comprising from 7 to 14 % by weight of one or more enteric coating agents in relation to the total weight of the external modified release coating layer.

In an embodiment, the multiple unit oral dosage form of the invention is one wherein the modified release multiple unit oral dosage form comprises a first plurality of modified release pellets of doxylamine or a pharmaceutically acceptable salt thereof comprising an external modified release coating layer comprising from 38 to 46 % by weight of one or more modified release coating agents in relation to the total weight of the external modified release coating layer.

In an embodiment, the multiple unit oral dosage form of the invention is one wherein the modified release multiple unit oral dosage form comprises a first plurality of modified release pellets of doxylamine or a pharmaceutically acceptable salt thereof comprising an external modified release coating layer comprising from 42 to 52 % by weight of one or more anticaking agent in relation to the total weight of the external modified release coating layer and optionally one or more pore forming agent, being the sum of the components up to 100% by weight in relation to the weight of the external modified release coating layer.

In an embodiment, the multiple unit oral dosage form of the invention is one wherein the modified release multiple unit oral dosage form comprises a first plurality of modified release pellets of doxylamine or a pharmaceutically acceptable salt thereof comprising an external modified release coating layer comprising: from 7 to 14 % by weight of one or more enteric coating agents; from 38 to 46 % by weight of one or more modified release coating agents in relation to the total weight of the external modified release coating layer; from 42 to 52 % by weight of one or more anticaking agent in relation to the total weight of the external modified release coating layer and optionally one or more pore forming agent; and optionally one or more pharmaceutically acceptable excipients, being the sum of the components up to 100% by weight in relation to the weight of the external modified release coating layer.

In an embodiment, the multiple unit oral dosage form of the invention is one wherein the modified release multiple unit oral dosage form comprises a first plurality of modified release pellets of doxylamine or a pharmaceutically acceptable salt thereof comprising:
- an inner active coating layer comprising:
   a therapeutically effective amount of doxylamine or a pharmaceutically acceptable salt thereof;
   from 6 to 20 % by weight of one or more coating agents in relation to the total weight of the inner active coating layer and
   from 15 to 30 % by weight of one or more anticaking agent in relation to the total weight of the inner active coating layer and optionally one or more pore forming agents;
   optionally one or more pharmaceutically acceptable excipients; and
   being the sum of the components up to 100% by weight in relation to the weight of the inner active coating layer;
- optionally an intermediate enteric release coating layer comprising:
   from 45 to 65 % by weight of one or more enteric coating agents in relation to the total weight of the intermediate enteric release coating layer; and
   from 35 to 55 % by weight of one or more anticaking agents in relation to the total weight of the intermediate enteric release coating layer and optionally one or more pore forming agents, optionally one or more pharmaceutically acceptable excipients; and
   being the sum of the components up to 100% by weight in relation to the weight of the intermediate enteric release coating layer;
- an external modified release coating layer comprising:
   from 7 to 14 % by weight of one or more enteric coating agents in relation to the total weight of the external modified release coating layer;
   from 38 to 46 % by weight of one or more modified release coating agents in relation to the total weight of the external modified release coating layer; and
   from 42 to 52 % by weight of one or more anticaking agent in relation to the total weight of the external modified release coating layer and optionally one or more pore-forming agent;
   optionally one or more pharmaceutically acceptable excipients; and
   being the sum of the components up to 100% by weight in relation to the weight of the external modified release coating layer.

In an embodiment, the multiple unit oral dosage form of the invention is one wherein the modified release multiple unit oral dosage form comprises a second plurality of modified release pellets of pyridoxine or a pharmaceutically acceptable salt thereof comprising an inner active coating layer comprising from 13 to 25 % by weight of one or more coating agents in relation to the total weight of the inner active coating layer; and optionally one or more pharmaceutically acceptable excipients; being the sum of the components up to 100% by weight in relation to the weight of the inner active coating layer.

In an embodiment, the multiple unit oral dosage form of the invention is one wherein the modified release multiple unit oral dosage form comprises a second plurality of modified release pellets of pyridoxine or a pharmaceutically acceptable salt thereof comprising an external modified release coating layer comprising from 2 to 8% by weight of one or more enteric coating agents in relation to the total weight of the external modified release coating layer.

In an embodiment, the multiple unit oral dosage form of the invention is one wherein the modified release multiple unit oral dosage form comprises a second plurality of modified release pellets of pyridoxine or a pharmaceutically acceptable salt thereof comprising an external modified release coating layer comprising from 30 to 49 % by weight of one or more modified release coating agents in relation to the total weight of the external modified release coating layer.

In an embodiment, the multiple unit oral dosage form of the invention is one wherein the modified release multiple unit oral dosage form comprises a second plurality of modified release pellets of pyridoxine or a pharmaceutically acceptable salt thereof comprising an external modified release coating layer comprising from 46 to 65% by weight of one or more anticaking agents in relation to the total weight of the external modified release coating layer and optionally one or more pore forming agents, being the sum of the components up to 100% by weight in relation to the weight of the external modified release coating layer.

In an embodiment, the multiple unit oral dosage form of the invention is one wherein the modified release multiple unit oral dosage form comprises a second plurality of modified release pellets of pyridoxine or a pharmaceutically acceptable salt thereof comprising an external modified release coating layer comprising: from 2 to 8 % by weight of one or more enteric coating agents in relation to the total weight of the external modified release coating layer; from 30 to 49 % by weight of one or more modified release coating agents in relation to the total weight of the external modified release coating layer; from 46 to 65 % by weight of one or more anticaking agents in relation to the total weight of the external modified release coating layer and optionally one or more pore forming agents; optionally one or more pharmaceutically acceptable excipients being the sum of the components up to 100% by weight in relation to the weight of the external modified release coating layer.

In an embodiment, the multiple unit oral dosage form of the invention is one wherein the modified release multiple unit oral dosage form comprises a second plurality of modified release pellets of pyridoxine or a pharmaceutically acceptable salt thereof comprising:
- an inner active coating layer comprising:
   a therapeutically effective amount of pyridoxine or a pharmaceutically acceptable salt thereof; and
   from 13 to 25 % by weight of one or more coating agents in relation to the total weight of the inner active coating layer;
   optionally one or more pharmaceutically acceptable excipients; and
   being the sum of the components up to 100% by weight in relation to the weight of the inner active coating layer;
- an external modified release coating layer comprising:
   from 2 to 8 % by weight of one or more enteric coating agents in relation to the total weight of the external modified release coating layer;
   from 30 to 49 % by weight of one or more modified release coating agents in relation to the total weight of the external modified release coating layer; and
   from 46 to 65 % by weight of one or more anticaking agents in relation to the total weight of the external modified release coating layer and optionally one or more pore forming agents;
   optionally one or more pharmaceutically acceptable excipients; and
   being the sum of the components up to 100% by weight in relation to the weight of the external modified release coating layer.

In an embodiment, the multiple unit oral dosage form of the invention is one wherein the modified release multiple unit oral dosage form comprises:
a first plurality of modified release pellets of doxylamine or a pharmaceutically acceptable salt thereof comprising:
   - a pharmaceutically acceptable inert nucleus;
   - an inner active coating layer comprising:
      a therapeutically effective amount of doxylamine or a pharmaceutically acceptable salt thereof from 6 to 20 % by weight of one or more coating agents in relation to the total weight of the inner active coating layer and
      from 15 to 30 % by weight of one or more anticaking agent in relation to the total weight of the inner active coating layer and optionally one or more pore forming agents;
      optionally one or more pharmaceutically acceptable excipients; and
      being the sum of the components up to 100% by weight in relation to the weight of the inner active coating layer;
   - optionally an intermediate enteric release coating layer comprising:
      from 45 to 65 % by weight of one or more enteric coating agents in relation to the total weight of the intermediate enteric release coating layer; and
      from 35 to 55 % by weight of one or more anticaking agents in relation to the total weight of the intermediate enteric release coating layer and optionally one or more pore forming agents;
      optionally one or more pharmaceutically acceptable excipients; and
      being the sum of the components up to 100% by weight in relation to the weight the intermediate enteric release coating layer;
   - an external modified release coating layer comprising:
      from 7 to 14 % by weight of one or more enteric coating agents in relation to the total weight of the external modified release coating layer;
      from 38 to 46 % by weight of one or more modified release coating agents in relation to the total weight of the external modified release coating layer; and
      from 42 to 52 % by weight of one or more anticaking agent in relation to the total weight of the external modified release coating layer and optionally one or more pore-forming agent;
      optionally one or more pharmaceutically acceptable excipients; and
      being the sum of the components up to 100% by weight in relation to the weight the external modified release coating layer;
         and
a second plurality of modified release pellets of pyridoxine or a pharmaceutically acceptable salt thereof comprising:
   - a pharmaceutically acceptable inert nucleus;
   - an inner active coating layer comprising:
      a therapeutically effective amount of pyridoxine or a pharmaceutically acceptable salt thereof; and
      from 13 to 25 % by weight of one or more coating agents in relation to the total weight of the inner active coating layer;
      optionally one or more pharmaceutically acceptable excipients; and
      being the sum of the components up to 100% by weight in relation to the weight of the inner active coating layer;
   - an external modified release coating layer comprising:
      from 2 to 8 % by weight of one or more enteric coating agents in relation to the total weight of the external modified release coating layer;
      from 30 to 49 % by weight of one or more modified release coating agents in relation to the total weight of the external modified release coating layer; and
      from 46 to 65 % by weight of one or more anticaking agents in relation to the total weight of the external modified release coating layer and optionally one or more pore forming agents;
      optionally one or more pharmaceutically acceptable excipients; and
      being the sum of the components up to 100% by weight in relation to the weight of the external modified release coating layer.

In an embodiment, the modified multiple unit oral dosage form of the present invention is one which exhibits a dissolution profile according to which:
from 5% to 35% by weight of doxylamine content is dissolved at 1^{st}h in 0.1 N HCl medium (pH = 1);
then, the medium is replaced by a pH = 4.5 medium (0.05 M acetate buffer) and at 4^{th} h from an accumulated more than 35% to 75% by weight of doxylamine initial content is dissolved;
then, the medium is replaced by a pH = 6.8 medium (0.05 M phosphate buffer) and at 7^{th} h at least an accumulated more than 75% by weight of doxylamine initial content is dissolved; and
from 5% to 35% by weight of pyridoxine content is dissolved at 1^{st}h in 0.1 N HCl medium (pH = 1);
then, the medium is replaced by a pH = 4.5 medium (0.05 M acetate buffer) and at 4^{th} h from an accumulated more than 35% to 75% by weight of pyridoxine initial content is dissolved;
then, the medium is replaced by a pH = 6.8 medium (0.05 M phosphate buffer) and at 7^{th} h at least an accumulated more than 75% by weight of pyridoxine initial content is dissolved;
wherein the dissolution profile is measured using a USP type 2 apparatus (basket), placing the composition in 900mL of the corresponding media / buffered at 37°C ± 0.5 °C and 100 rpm (revolution per minute).

In an embodiment, the modified multiple unit oral dosage form of the present invention is one which exhibits a dissolution profile according to which:
from 10% to 35% by weight of doxylamine content is dissolved at 1^{st}h in 0.1 N HCl medium (pH = 1);
then, the medium is replaced by a pH = 4.5 medium (0.05 M acetate buffer) and at 4^{th} h from an accumulated 45% to 70% by weight of doxylamine initial content is dissolved;
then, the medium is replaced by a pH = 6.8 medium (0.05 M phosphate buffer) and at 7^{th} h at least an accumulated 80% by weight of doxylamine initial content is dissolved; and
from 10% to 35% by weight of pyridoxine content is dissolved at 1^{st}h in 0.1 N HCl medium (pH = 1);
then, the medium is replaced by a pH = 4.5 medium (0.05 M acetate buffer) and at 4^{th} h from an accumulated 40% to 65% by weight of pyridoxine initial content is dissolved;
then, the medium is replaced by a pH = 6.8 medium (0.05 M phosphate buffer) and at 7^{th} h at least an accumulated 80% by weight of pyridoxine initial content is dissolved;
wherein the dissolution profile is measured using a USP type 2 apparatus (basket), placing the composition in 900mL of the corresponding media / buffered at 37°C ± 0.5 °C and 100 rpm (revolution per minute).

As it is mentioned above, the dosage form of the present invention is a "multiple unit dosage form". In an embodiment, the multiple unit dosage form is a capsule filled with the first and the second plurality pellets of the present invention as subunits having the active ingredient. In an embodiment, the multiple unit dosage form is a hard capsule.

For the purpose of the invention, the hard capsule is understood as a hard capsule suitable to be used in fully automatic capsule filling machine. Commonly, these capsules are made up of two cylindrical halves, wherein one of them is large in diameter but shorter in length called cap and other is shorter in diameter but longer in length called body. In an embodiment, the multiple unit oral dosage form is a hard capsule having a capsule size from size 0 to size 5. In an embodiment, the multiple unit oral dosage form is a hard capsule having a capsule size from size 1 to size 5. In an embodiment, the multiple unit oral dosage form is a hard capsule having a capsule size from size 1 to size 4. In an embodiment, the multiple unit oral dosage form is a hard capsule having a capsule size of 3. In an embodiment, the multiple unit oral dosage form is a hard capsule having a capsule size of 2. In an embodiment, the multiple unit oral dosage form is a hard capsule having a capsule size of 1. In an embodiment, the multiple unit dosage form of the present invention is a hard capsule made of a substance selected from the group consisting of gelatine, hydroxypropyl methylcellulose (hypromellose, HPMC), pullulan and a mixture thereof. In an embodiment, the multiple unit dosage form of the present invention is a gelatine hard capsule. In an embodiment, the multiple unit dosage form of the present invention is a hydroxypropyl methylcellulose hard capsule.

In an embodiment, the multiple unit dosage form of the present invention is a hard capsule and comprises from 20 mg to 220 mg of the first plurality of modified release pellets of doxylamine or a pharmaceutically acceptable salt thereof; and from 20 mg to 220 mg of the second plurality of modified release pellets of pyridoxine or a pharmaceutically acceptable salt thereof. In an embodiment, the multiple unit dosage form of the present invention is a hard capsule and comprises from 40 mg to 140 mg of the first plurality of modified release pellets of doxylamine or a pharmaceutically acceptable salt thereof; and from 40 mg to 140 mg of the second plurality of modified release pellets of pyridoxine or a pharmaceutically acceptable salt thereof.

In an embodiment, the multiple unit dosage form of the present invention is a hard capsule and comprises about 60 mg of the first plurality of modified release pellets of doxylamine or a pharmaceutically acceptable salt thereof; and about 60 mg of the second plurality of modified release pellets of pyridoxine or a pharmaceutically acceptable salt thereof; particularly the hard capsule has a size selected from size 2 or size 3 or size 4, more particular has a size 3.

In an embodiment, the multiple unit dosage form of the present invention is a hard capsule and comprises about 120 mg of the first plurality of modified release pellets of doxylamine or a pharmaceutically acceptable salt thereof; and about 120 mg of the second plurality of modified release pellets of pyridoxine or a pharmaceutically acceptable salt thereof; particularly the hard capsule has a size selected from size 1, size 2 and size 3. In an embodiment, the multiple unit dosage form of the present invention is a hard capsule and comprises about 10 mg per capsule of doxylamine succinate and about 10 mg per capsule of pyridoxine hydrochloride; particularly the hard capsule has a size 3. In an embodiment, the multiple unit dosage form of the present invention is a hard capsule and comprises about 20 mg per capsule of doxylamine succinate and about 20 mg per capsule of pyridoxine hydrochloride; particularly the hard capsule has a size selected from size 1, size 2 and size 3.

For the purpose of the present invention, the multiple unit dosage form of the present invention, particularly hard capsules, can be conditioned in appropriate package. The type of package can readily be determined by those skilled in the art according to the type of formulation being prepared. In an embodiment, the modified release multiple unit dosage form of the present invention is packaged in blisters. In an embodiment, the modified release multiple unit dosage form of the present invention is packaged in bottles with or without desiccant placed inside the bottle or integrated in the closure system of the bottle).

For the purpose of the invention, the multiple unit dosage form of the present invention, particularly hard capsules, is primary packaged in blisters or bottles as defined above and secondary packaged in an outer carton. Materials commonly used for assembling the blisters in which the multiple unit dosage form of the present invention, particularly hard capsules, are primary packaged are PVC (polyvinylchloride), PVdC (polyvinylidene chloride), PE (polyethylene comprising HDPE or high-density polyethylene and LDPE or lowdensity polyethylene), PET (polyethylene terephthalate), PETG (poly-ethylene terephthalate glycol), PCTFE (polychlorotrifluoroethylene, commercially available as polyAclar^{®}) , PVC/PE/PVdC (commercially available as AquaBa^{®}), COC (Cyclic olefin copolymer), Aluminium or a combination thereof. Materials commonly used for making bottles and the relevant closures or caps, in which the multiple unit dosage form of the present invention, particularly hard capsules, are primary packaged are glass, aluminium and plastic materials. Example of plastic material are PE (comprising HDPE and LDPE), PET, PP (polypropylene), PVC, PETG, PS (polystyrene), COC, and/or a mixture of more than one plastic material thereof and/or a mixture of one or more plastic material with further additives. Example of plastic material additives are binders, desiccants, plasticizers, flame retardants, antioxidants, acid scavengers, light and heat stabilizers, lubricants, pigments, antistatic agents, slip compounds and thermal stabilizers. Example of desiccants are moisture barrier materials, molecular sieve such as for example zeolites, calcium oxide, activated charcoal, calcium sulphate, calcium chloride and silica. The desiccant can be either mixed/incorporated with a suitable binder in the plastic material used to make the bottle or placed inside the bottle or integrated in the closure system of the bottle.

The inventors have surprisingly found out that the primary packaging as defined above, is advantageous in terms of stability. Particularly, they allow the storage at or below 25°C and 60% Relative Humidity. This is advantageous as the multiple unit dosage form of the present invention would be suitable for long term storage, even in climatic zone I and II countries (temperate and Mediterranean/subtropical zone countries), without particular restrictions for instance without the need to store in a refrigerator.

In an embodiment, the multiple unit dosage form of the present invention is hard gelatine or HPMC (hydroxypropyl methyl cellulose) capsules having a primary packaged selected from the group consisting of blister made with any of the material mentioned above and plastic bottle made with any plastic material mentioned above without desiccant. They are especially advantageous because are stable allowing storage at or below 25°C and 60% Relative Humidity.

In a particular embodiment, the multiple unit dosage form of the present invention is hard gelatine or HPMC capsules having a primary packaged in blister made of PVC/PVdC (on one side of the blister) and aluminium (on the other side of the blister) being stable allowing storage at or below 25°C and 60% Relative Humidity. This is advantageous as this type of blister is broadly used and easy to handle from a primary packaging manufacturing step and it is therefore costless.

In a particular embodiment, the multiple unit dosage form of the present invention is hard capsule made of gelatine or HPMC having a primary packaged in plastic bottles with a desiccant. They are especially advantageous in terms of stability allowing storage at or below 30°C and 75% Relative Humidity. This is advantageous as the multiple unit dosage form of the present invention would be suitable for long term storage also in climatic zone III and IV countries (hot dry and hot humid / tropical zone countries) without particular restrictions (for example without the need to store in a refrigerator). An additional advantage is the avoidance of the use of glass bottles that are impermeable containers and can offer maximum protection against moisture but are more fragile and heavier when compared to plastic bottles and are therefore much more difficult to handle from a logistic standpoint with increased cost and risk for the manufacturer and the user.

In an embodiment, the multiple unit dosage form of the present invention is a hard capsule made of gelatine or HPMC having a primary packaged in blister made with materials as define above or plastic bottles with desiccant as a primary package is further advantageous in terms of stability allowing storage at or below 30°C and 75% Relative Humidity. This is advantageous as the multiple unit dosage form of the present invention would be suitable for long term storage also in climatic zone III and IV countries (hot dry and hot humid / tropical zone countries) without particular restrictions (for example without the need to store in a refrigerator).

In an embodiment, the multiple unit dosage form of the present invention is hard HPMC capsules having a primary packaged in blister made with materials as defined above and particularly made in AquaBa^{®} or Aluminium (one side of the blister) and Aluminium (the other side of the blister). They are advantageous because they are stable allowing storage at or below 30°C and 75% Relative Humidity. This is further advantageous for the user in climatic zone III and IV countries (hot dry and hot humid / tropical zone countries) because not only it allows storage without particular restrictions (for example without the need to store in a refrigerator) but also because blisters are easier to handle than bottles (for example can be easily kept on hand in case of travelling) and the capsules are individually protected when packed in blister thus allowing a safer and more convenient use.

The second aspect of the present invention relates to a process for the preparation of the multiple unit oral dosage form of the first aspect of the invention. In particular, this process comprises:
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by adding the one or more enteric coating agents, the one or more modified release coating agents, the one or more anticaking agents, optionally one or more pore-forming agent, and optionally one or more pharmaceutically acceptable excipients, wherein the particle size of the pharmaceutically acceptable inert nucleus is such that at least 90% of the inert nuclei have a particle size from 300 µm to 1700 µm measured by analytical sieving and at least the 90% of inert nuclei have a particle size variability of not more than 200 µm; particularly of not more than 150 µm; particularly of not more than 100 µm; and particularly of not more than 75 µm measured by analytical sieving; and
(b1) preparing the second plurality of modified release pellets of pyridoxine or a pharmaceutically acceptable salt thereof by coating the pellets of pyridoxine or a pharmaceutically acceptable salt thereof having the inner active coating layer by adding the one or more enteric coating agents, the one or more modified release coating agents, optionally the one or more pore-forming agents, and optionally one or more pharmaceutically acceptable excipients, wherein: wherein the particle size of the pharmaceutically acceptable inert nucleus is such that at least 90% of the inert nuclei have a particle size from 300 µm to 1700 µm measured by analytical sieving and at least the 90% of inert nuclei have a particle size variability of not more than 200 µm; particularly of not more than 150 µm; particularly of not more than 100 µm; and particularly of not more than 75 µm measured by analytical sieving. As it is mentioned above, this process is cheaper, more robust, reproducible, and easier to scale-up in comparison with the processes of the state of the art. It allows obtaining homogenous batches of both plurality of pellets in a high yield without losing a considerable or large amount of yield in sieving steps, obtaining a high final yield. All embodiments disclosed above for the particle size and particle size variability of the pharmaceutically acceptable nucleus and the first and the second plurality of the pellets, and combination thereof disclosed in the first aspect of the invention also applies for the process of the second aspect of the invention.

In an embodiment, the process for the preparation of the multiple unit oral dosage form of the first aspect of the invention comprises:
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by continuously or discontinuously spraying a liquid mixture comprising one or more enteric coating agents, one or more modified release coating agents, and optionally one or more pharmaceutically acceptable excipients; optionally by doing one or more pauses; and optionally by continuously or discontinuously drying with airflow; and
(b1) preparing the second plurality of modified release pellets of pyridoxine or a pharmaceutically acceptable salt thereof by coating the pellets of pyridoxine or a pharmaceutically acceptable salt thereof having the inner active coating layer by continuously or discontinuously spraying a liquid mixture comprising one or more enteric coating agents, one or more modified release coating agents, and optionally one or more pharmaceutically acceptable excipients; optionally by doing one or more pauses; and optionally by continuously or discontinuously drying with airflow.

In an embodiment, the steps (a1) and (b1) of the process of the invention is performed by continuously spraying a liquid mixture as defined above. In an embodiment, the steps (a1) and (b1) of the process of the invention is performed by continuously spraying a liquid mixture as defined above and comprising one or more pause periods of time. In an embodiment, the steps (a1) and (b1) of the process of the invention is performed by continuously spraying a liquid mixture as defined above and comprising one or more pause periods of time; and by continuously drying with airflow. In an embodiment, the steps (a1) and (b1) of the process of the invention is performed by continuously spraying a liquid mixture as defined above and comprising one or more pause periods of time; and by discontinuously drying with airflow.

In an embodiment, the steps (a1) and (b1) of the process of the invention is performed by discontinuously spraying a liquid mixture as defined above and comprising one or more pause periods of time. In an embodiment, the steps (a1) and (b1) of the process of the invention is performed by discontinuously spraying a liquid mixture as defined above and by continuously drying with airflow. In an embodiment, the steps (a1) and (b1) of the process of the invention is performed by discontinuously spraying a liquid mixture as defined above and by discontinuously drying with airflow. In an embodiment, the steps (a1) and (b1) of the process of the invention is performed by discontinuously spraying a liquid mixture as defined above, comprising one or more pause periods of time and by discontinuously drying with airflow.

In an embodiment, the process for the preparation of the multiple unit oral dosage form of the first aspect of the invention comprises:
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by continuously or discontinuously spraying a liquid mixture comprising one or more enteric coating agents, one or more modified release coating agents, and optionally one or more pharmaceutically acceptable excipients; and adding simultaneously or alternately a mixture in powder form comprising one or more anticaking agents, optionally one or more pore-forming agents, and optionally one or more pharmaceutically acceptable excipients; optionally by doing one or more pauses; and optionally by continuously or discontinuously drying with airflow; and
(b1) preparing the second plurality of modified release pellets of pyridoxine or a pharmaceutically acceptable salt thereof by coating the pellets of pyridoxine or a pharmaceutically acceptable salt thereof having the inner active coating layer by continuously or discontinuously spraying a liquid mixture comprising one or more enteric coating agents, one or more modified release coating agents, and optionally one or more pharmaceutically acceptable excipients; and adding simultaneously or alternately a mixture in powder form comprising the one or more anticaking agents, optionally one or more pore-forming agents, and optionally one or more pharmaceutically acceptable excipients; optionally by doing one or more pauses; optionally one or more pause periods of time; and optionally by continuously or discontinuously drying with airflow.

In an embodiment, the steps (a1) and (b1) of the process of the invention is performed by continuously spraying a liquid mixture as defined above and adding simultaneously the mixture in powder form. In an embodiment, the steps (a1) and (b1) of the process of the invention is performed by continuously spraying a liquid mixture as defined above and adding alternately a mixture in powder form. In an embodiment, the steps (a1) and (b1) of the process of the invention is performed by continuously spraying a liquid mixture as defined above, adding simultaneously the mixture in powder form; and by continuously drying with airflow. In an embodiment, the steps (a1) and (b1) of the process of the invention is performed by continuously spraying a liquid mixture as defined above, adding alternately a mixture in powder form; and by continuously drying with airflow. In an embodiment, the steps (a1) and (b1) of the process of the invention is performed by continuously spraying a liquid mixture as defined above, adding simultaneously the mixture in powder form; and by discontinuously drying with airflow. In an embodiment, the steps (a1) and (b1) of the process of the invention is performed by continuously spraying a liquid mixture as defined above, adding alternately a mixture in powder form; and by discontinuously drying with airflow.

In an embodiment, the steps (a1) and (b1) of the process of the invention is performed by discontinuously spraying the liquid mixture, adding simultaneously the mixture in powder form, and by doing one or more pauses. In an embodiment, the steps (a1) and (b1) of the process of the invention is performed by discontinuously spraying the liquid mixture, adding simultaneously the mixture in powder form, and continuously drying with airflow. In an embodiment, the steps (a1) and (b1) of the process of the invention is performed by discontinuously spraying the liquid mixture, adding simultaneously the mixture in powder form, and discontinuously drying with airflow. In an embodiment, the steps (a1) and (b1) of the process of the invention is performed by discontinuously spraying the liquid mixture, adding simultaneously the mixture in powder form, by doing one or more pauses; and discontinuously drying with airflow. In an embodiment, the steps (a1) and (b1) of the process of the invention is performed by discontinuously spraying the liquid mixture, adding alternately the mixture in powder form and by doing one or more pauses. In an embodiment, the steps (a1) and (b1) of the process of the invention is performed by discontinuously spraying the liquid mixture, adding alternately the mixture in powder form, and continuously drying with airflow. In an embodiment, the steps (a1) and (b1) of the process of the invention is performed by discontinuously spraying the liquid mixture, adding alternately the mixture in powder form, and discontinuously drying with airflow. In an embodiment, the steps (a1) and (b1) of the process of the invention is performed by discontinuously spraying the liquid mixture, adding alternately the mixture in powder form, by doing one or more pauses; and discontinuously drying with airflow. In an embodiment, the steps (a1) and (b1) of the process of the invention is performed by discontinuously spraying the liquid mixture and adding simultaneously the mixture in powder form. In an embodiment, the steps (a1) and (b1) of the process of the invention is performed by discontinuously spraying the liquid mixture and adding alternately the mixture in powder form.

For the purpose of the invention, the term "liquid mixture" refers to any mixture of one or more of the components as defined in the present invention, such as for example the coating agents, the modified release coating agents, the pharmaceutically acceptable excipients, the pharmaceutical active ingredients (pyridoxine and doxylamine) wherein the mixture have a liquid state behaviour. The term "liquid state behaviour" refers to a mixture or substance that can flow, has no fixed shape, and is not a solid or a gas. This mixture can be in form of solution or in form of a suspension (or slurry). A solution is a type of homogeneous mixture composed of two or more substances. In such a mixture, a solute is a substance dissolved in another substance, known as a solvent. A suspension is a heterogeneous mixture that contains solid particles in a liquid solvent. In fact, the solid particles do not dissolve in the liquid solvent.

The term "liquid solvent" refers to any organic and inorganic liquid solvent or a mixture thereof able to solve a compound/component/ingredient creating a liquid solution or refers to any liquid able to create a suspension or slurry of one of the compounds/components/ingredients or more. The liquid solvent is selected preferably from de group of volatile liquid solvent (boiling point below 125 °C), comprising one or more organic liquid solvent selected from the group consisting of (C₁-C₄)alcohol, (C₁-C₄)alkyl-CO-(C₁-C₄)alkyl, (C₁-C₄)alkyl-CO-O-(C₁-C₄)alkyl, or water, or mixtures thereof. The term "alcohol" refers to an "alkane" wherein at least one hydrogen atom is substituted by a hydroxyl group and which contains the number of carbon atoms specified in the description or claims. The term "alkane" refers to a saturated, branched, or linear hydrocarbon which contains the number of carbon atoms specified in the description or claims. Examples include methanol, ethanol, n-propanol, iso-propanol, butanol, iso-butanol, and sec-butanol. The term "alkyl" refers to a saturated straight, or branched hydrocarbon chain which contains the number of carbon atoms specified in the description or claims. Examples include, among others, the group methyl, ethyl, propyl, isopropyl, butyl, isobutyl, sec-butyl, and tert-butyl. In an embodiment, the process is one wherein the mixture comprises one or more organic solvents selected from the group consisting of ethanol, 2-propanol, methanol, acetone, butanone, ethyl acetate, water, and a mixture thereof; particularly selected from ethanol, acetone, water, and a mixture thereof. There is no limitation regarding the liquid solvent, except that if used for therapeutic purposes, they must be pharmaceutically acceptable.

The term "mixture in powder form" refers to any individual solid compounds/components/ingredients or combination thereof as defined in the present invention which are solids and in powder form. For the purpose of the invention powder form is considered a solid in powder form when it has a D90 equal to or below than 500 µm, preferably equal to or below than 250 µm, and more preferably equal to or below than 150 µm.

The term "spraying continuously" refers to spraying, during a certain step of the process, continuously over time until all the liquid to be sprayed is consumed. The term "spraying discontinuously" refers to intermittent and cyclical spraying over time. This means spraying during a certain period of time, stop spraying for another period of time and repeat this "cycle" as many times as necessary until all the liquid to be sprayed necessary for a specific stage of the process is consumed.

The term "adding simultaneously a mixture in powder form" refers to the addition of solids simultaneously over time with respect to the spraying of a liquid mixture during one step of the process. This means that when the powder mixture is added the liquid mixture is also sprayed at the same time. It is possible that the spraying is permanent and continuous or that, on the contrary, it is intermittent, although it always coincides with the spraying, whether it is continuous or discontinuous spraying. The term "adding alternately a mixture in powder form" refers to the addition of a mixture of solids in powder form a non-simultaneous manner, which is intermittent and discontinuous with respect to the spraying of liquid mixtures that is also intermittently and discontinuously, cyclically (in a "cycle") repeating this process until completing the addition of solids in the form of powder and / or liquid mixture spray.

The term "continuously drying" refers to the fact of drying with airflow the pellets into the coating pan during all the period of time the coating step is in progress, regardless of whether the spraying is continuous or discontinuous, or the addition of solid is simultaneous or alternate. The term "discontinuously drying" refers to the fact of drying with airflow the pellets into the coating pan only for a certain period of time within a "cycle" regardless of whether the spraying is continuous or discontinuous, or the addition of solid is simultaneous or alternate.

The term "airflow" refers to a dry air flow, at a temperature from 5 to 90 °C, measured in m³ / h.

The term "pause" refers to a certain period of time during a "cycle" repeated during a coating step, wherein there is no spray of any liquid mixture, no addition of any solid mixture in powder form and no drying with airflow. During this time, the coating pan is only spinning and mixing and homogenizing the pellets. The term "cycle" refers to a sequence that lasts a certain period of time of a coating step, this sequence is repeated continuously as many times as necessary until completing the coating step. This cycle comprises at least a certain period of spraying a liquid mixture, equal to or below than the cycle period of time; optionally a certain period of adding a mixture in powder form, equal to or below than the cycle period of time, optionally one or more pause periods of time, lower than the cycle period of time; optionally a drying period of time, equal to or below than the cycle period of time.

In an embodiment, the process for the preparation of the multiple unit oral dosage form of the second aspect of the invention comprises:
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by adding the one or more enteric coating agents, the one or more modified release coating agents, the one or more anticaking agents, optionally one or more pore-forming agent, and optionally one or more pharmaceutically acceptable excipients, wherein the particle size of the pharmaceutically acceptable inert nucleus is such that at least 90% of the inert nuclei have a particle size from 300 µm to 1700 µm measured by analytical sieving and at least the 90% of inert nuclei have a particle size variability of not more than 200 µm; particularly a particle size variability of not more than 150 µm; particularly a particle size variability of not more than 100 µm; and particularly a particle size variability of not more than 75 µm measured by analytical sieving; and the sum of the enteric coating agents and the modified release coating agents in the spraying liquid mixture is from 10% to 49% by weight in relation to the weight of the liquid mixture; and
(b1) preparing the second plurality of modified release pellets of pyridoxine or a pharmaceutically acceptable salt thereof by coating the pellets of pyridoxine or a pharmaceutically acceptable salt thereof having the inner active coating layer by adding the one or more enteric coating agents, the one or more modified release coating agents, the one or more anticaking agents, optionally the one or more pore-forming agents, and optionally one or more pharmaceutically acceptable excipients, wherein: the particle size of the pharmaceutically acceptable inert nucleus is such that at least 90% of the inert nuclei have a particle size from 300 µm to 1700 µm measured by analytical sieving and at least the 90% of inert nuclei have a particle size variability of not more than 200 µm; particularly a particle size variability of not more than 150 µm; particularly a particle size variability of not more than 100 µm; and particularly a particle size variability of not more than 75 µm measured by analytical sieving; and the sum of the enteric coating agents and the modified release coating agents in the spraying liquid mixture is from 10% to 49% by weight in relation to the weight of the liquid mixture. All embodiments disclosed above for the particle size and particle size variability of the pharmaceutically acceptable nucleus and the first and the second plurality of the pellets, and combination thereof disclosed in the first aspect of the invention also applies for the process of the second aspect of the invention.

In an embodiment, the process for the preparation of the multiple unit oral dosage form of the first aspect of the invention comprises:
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by continuously or discontinuously spraying a liquid mixture comprising from 1.0 to 7.5 % by weight of the one or more enteric coating agents, from 10.0 to 35.0 % by weight of the one or more modified release coating agents and the particle size of the pharmaceutically acceptable inert nucleus is such that at least 90% of the inert nuclei have a particle size from 300 µm to 1700 µm measured by analytical sieving and at least the 90% of inert nuclei have a particle size variability of not more than 200 µm; particularly a particle size variability of not more than 150 µm; particularly a particle size variability of not more than 100 µm; and particularly a particle size variability of not more than 75 µm measured by analytical sieving, and
(b1) preparing the second plurality of modified release pellets of pyridoxine or a pharmaceutically acceptable salt thereof by coating the pellets of pyridoxine or a pharmaceutically acceptable salt thereof having the inner active coating layer by continuously or discontinuously spraying a liquid mixture comprising from 1.0 to 7.5 % by weight of the one or more enteric coating agents, from 10.0 to 35.0 % by weight of the one or more modified release coating agents and the particle size of the pharmaceutically acceptable inert nucleus is such that at least 90% of the inert nuclei have a particle size from 300 µm to 1700 µm measured by analytical sieving and at least the 90% of inert nuclei have a particle size variability of not more than 200 µm; particularly a particle size variability of not more than 150 µm; particularly a particle size variability of not more than 100 µm and particularly a particle size variability of not more than 75 µm measured by analytical sieving. All embodiments disclosed above for the particle size and particle size variability of the pharmaceutically acceptable nucleus and the first and the second plurality of the pellets, and combination thereof disclosed in the first aspect of the invention also applies for the process of the second aspect of the invention.

In an embodiment, the process for the preparation of the multiple unit oral dosage form of the first aspect of the invention comprises:
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by continuously or discontinuously spraying a liquid mixture wherein the weight ratio between the one or more enteric coating agents and the one or more modified release coating agents in the spraying mixture is from 5:95 to 30:70, and the particle size of the pharmaceutically acceptable inert nucleus is such that at least 90% of the inert nuclei have a particle size from 300 µm to 1700 µm measured by analytical sieving and at least the 90% of inert nuclei have a particle size variability of not more than 200 µm; particularly a particle size variability of not more than 150 µm; particularly a particle size variability of not more than 100 µm; and particularly a particle size variability of not more than 75 µm measured by analytical sieving, and
(b1) preparing the second plurality of modified release pellets of pyridoxine or a pharmaceutically acceptable salt thereof by coating the pellets of pyridoxine or a pharmaceutically acceptable salt thereof having the inner active coating layer by continuously or discontinuously spraying a liquid mixture wherein the weight ratio between the one or more enteric coating agents and the one or more modified release coating agents in the spraying mixture is from 5:95 to 30:70 and the particle size of the pharmaceutically acceptable inert nucleus is such that at least 90% of the inert nuclei have a particle size from 300 µm to 1700 µm measured by analytical sieving and at least the 90% of inert nuclei have a particle size variability of not more than 200 µm; particularly a particle size variability of not more than 150 µm; particularly a particle size variability of not more than 100 µm; and particularly a particle size variability of not more than 75 µm measured by analytical sieving. All embodiments disclosed above for the particle size and particle size variability of the pharmaceutically acceptable nucleus and the first and the second plurality of the pellets, and combination thereof disclosed in the first aspect of the invention also applies for the process of the second aspect of the invention.

In an embodiment, the process for the preparation of the multiple unit oral dosage form of the first aspect of the invention comprises:
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by continuously or discontinuously spraying a liquid mixture comprising from 1.0 to 7.5 % by weight of the one or more enteric coating agents, from 10.0 to 35.0 % by weight of the one or more modified release coating agents, wherein the weight ratio between the one or more enteric coating agents and the one or more modified release coating agents in the spraying mixture is from 5:95 to 30:70 and the particle size of the pharmaceutically acceptable inert nucleus is such that at least 90% of the inert nuclei have a particle size from 300 µm to 1700 µm measured by analytical sieving and at least the 90% of inert nuclei have a particle size variability of not more than 200 µm; particularly a particle size variability of not more than 150 µm; particularly a particle size variability of not more than 100 µm; and particularly a particle size variability of not more than 75 µm measured by analytical sieving, and
(b1) preparing the second plurality of modified release pellets of pyridoxine or a pharmaceutically acceptable salt thereof by coating the pellets of pyridoxine or a pharmaceutically acceptable salt thereof having the inner active coating layer by continuously or discontinuously spraying a liquid mixture comprising from 1.0 to 7.5 % by weight of the one or more enteric coating agents, from 10.0 to 35.0 % by weight of the one or more modified release coating agents, wherein the weight ratio between the one or more enteric coating agents and the one or more modified release coating agents in the spraying mixture is from 5:95 to 30:70 and the particle size of the pharmaceutically acceptable inert nucleus is such that at least 90% of the inert nuclei have a particle size from 300 µm to 1700 µm measured by analytical sieving and at least the 90% of inert nuclei have a particle size variability of not more than 200 µm; particularly a particle size variability of not more than 150 µm; particularly a particle size variability of not more than 100 µm; and particularly a particle size variability of not more than 75 µm measured by analytical sieving. All embodiments disclosed above for the particle size and particle size variability of the pharmaceutically acceptable nucleus and the first and the second plurality of the pellets, and combination thereof disclosed in the first aspect of the invention also applies for the process of the second aspect of the invention.

In an embodiment, the process for the preparation of the multiple unit oral dosage form of the first aspect of the invention comprises:
(a1) preparing the first plurality of modified release pellets of doxylamine or a pharmaceutically acceptable salt thereof, wherein the spray average flow rate of the mixture comprising the coating is from 0.30 to 5.00 g/min per kg of pharmaceutically acceptable inert nuclei; and
(b1) preparing the second plurality of modified release pellets of pyridoxine or a pharmaceutically acceptable salt thereof, wherein the spray average flow rate of the mixture comprising the coating agents is from 0.30 to 5.00 g/min per kg of pharmaceutically acceptable inert nuclei.

In an embodiment, the process for the preparation of the multiple unit oral dosage form of the first aspect of the invention comprises:
(a1) preparing the first plurality of modified release pellets of doxylamine or a pharmaceutically acceptable salt thereof, wherein the spray local flow rate of the mixture comprising the coating is from 0.30 to 9.00 g/min per kg of pharmaceutically acceptable inert nuclei; and
(b1) preparing the second plurality of modified release pellets of pyridoxine or a pharmaceutically acceptable salt thereof, wherein the spray local flow rate of the mixture comprising the coating is from 0.30 to 9.00 g/min per kg of pharmaceutically acceptable inert nuclei.

In an embodiment, the process for the preparation of the multiple unit oral dosage form of the first aspect of the invention comprises:
(a1) preparing the first plurality of modified release pellets of doxylamine or a pharmaceutically acceptable salt thereof, wherein the average of the solid addition rate of the mixture in solid form is from 0.95 to 18 g/min per Kg of pharmaceutically acceptable inert nuclei; and
(b1) preparing the second plurality of modified release pellets of pyridoxine or a pharmaceutically acceptable salt thereof, wherein; the average of the solid addition rate of the mixture in solid form is from 0.10 to 2.25 g/min per Kg of pharmaceutically acceptable inert nuclei.

In an embodiment, the process for the preparation of the multiple unit oral dosage form of the first aspect of the invention comprises:
(a1) preparing the first plurality of modified release pellets of doxylamine or a pharmaceutically acceptable salt thereof, wherein the local solid addition rate of the mixture in solid form is from 0.95 to 40.00 g/min per Kg of pharmaceutically acceptable inert nuclei; and
(b1) preparing the second plurality of modified release pellets of pyridoxine or a pharmaceutically acceptable salt thereof, wherein the local solid addition rate of the mixture in solid form is from 0.10 to 40.00 g/min per Kg of pharmaceutically acceptable inert nuclei.

In an embodiment, the process for the preparation of the multiple unit oral dosage form of the first aspect of the invention comprises:
(a1) preparing the first plurality of modified release pellets of doxylamine or a pharmaceutically acceptable salt thereof, wherein the relation between the spray average flow rate of the liquid mixture comprising the coating agents and the average of the solid addition rate of the mixture in solid form is from 90:10 to 60:40, particularly from 90:10 to 70:30; and
(b1) preparing the second plurality of modified release pellets of pyridoxine or a pharmaceutically acceptable salt thereof, the relation between the spray average flow rate of the liquid mixture comprising the coating agents and the average of the solid addition rate of the mixture in solid form is from 90:10 to 60:40, particularly from 80:20 to 60:40.

In an embodiment, the process for the preparation of the multiple unit oral dosage form of the first aspect of the invention comprises:
(a1) preparing the first plurality of modified release pellets of doxylamine or a pharmaceutically acceptable salt thereof, wherein the spray average flow rate of the liquid mixture comprising the coating agents is from 0.30 to 5.00 g/min per kg of pharmaceutically acceptable inert nuclei and the average of the solid addition rate of the mixture in solid form is from 0.95 to 18.00 g/min per Kg of pharmaceutically acceptable inert nuclei; and
(b1) preparing the second plurality of modified release pellets of pyridoxine or a pharmaceutically acceptable salt thereof, wherein the spray average flow rate of the liquid mixture comprising the coating agents is from 0.30 to 5.00 g/min per kg of pharmaceutically acceptable inert nuclei; the average of the solid addition rate of the mixture in solid form is from 0.10 to 2.25 g/min per Kg of pharmaceutically acceptable inert nuclei.

In an embodiment, the process for the preparation of the multiple unit oral dosage form of the first aspect of the invention comprises:
(a1) preparing the first plurality of modified release pellets of doxylamine or a pharmaceutically acceptable salt thereof, wherein the spray local flow rate of the liquid mixture comprising the coating agents is from 0.30 to 9.00 g/min per kg of pharmaceutically acceptable inert nuclei, and the local solid addition rate of the mixture in solid form is from 0.95 to 40.00 g/min per Kg of inert nuclei; and
(b1) preparing the second plurality of modified release pellets of pyridoxine or a pharmaceutically acceptable salt thereof, wherein the spray local flow rate of the liquid mixture comprising the coating agents is from 0.30 to 9.00 g/min per kg of pharmaceutically acceptable inert nuclei, and the local solid addition rate of the mixture in solid form is from 0.10 to 40.00 g/min per Kg of inert nuclei.

In an embodiment, the process for the preparation of the multiple unit oral dosage form of the first aspect of the invention comprises:
(a1) preparing the first plurality of modified release pellets of doxylamine or a pharmaceutically acceptable salt thereof, wherein: the spray average flow rate of the liquid mixture comprising the coating agents is from 0.30 to 5.00 g/min per kg of pharmaceutically acceptable inert nuclei, the average of the solid addition rate of the mixture in solid form is from 0.95 to 18.00 g/min per Kg of pharmaceutically acceptable inert nuclei, and the relation between the spray average flow rate of the liquid mixture comprising the coating agents and the average of the solid addition rate of the mixture in solid form is from 90:10 to 60:40, particularly from 90:10 to 70:30;
   and
(b1) preparing the second plurality of modified release pellets of pyridoxine or a pharmaceutically acceptable salt thereof, wherein: the spray average flow rate of the mixture comprising the coating agents is from 0.30 to 5.00 g/min per kg of pharmaceutically acceptable inert nuclei; the average of the solid addition rate of the mixture in solid form is from 0.10 to 2.25 g/min per Kg of pharmaceutically acceptable inert nuclei, and the relation between the average spray flow rate of the liquid mixture comprising the coating agents and the average of the solid addition rate of the mixture in solid form is from 90: 10 to 60:40, particularly from 80:20 to 60:40.

In an embodiment, the process for the preparation of the multiple unit oral dosage form of the first aspect of the invention comprises:
(a1) preparing the first plurality of modified release pellets of doxylamine or a pharmaceutically acceptable salt thereof, wherein: the spray local flow rate of the mixture comprising the coating is from 0.30 to 9.00 g/min per kg of pharmaceutically acceptable inert nuclei, and the local solid addition rate of the mixture in solid form is from 0.95 to 40.00 g/min per Kg of inert nuclei; and
(b1) preparing the second plurality of modified release pellets of pyridoxine or a pharmaceutically acceptable salt thereof, wherein: the spray local flow rate of the mixture comprising the coating is from 0.30 to 9.00 g/min per kg of pharmaceutically acceptable inert nuclei, and the local solid addition rate of the mixture in solid form is from 0.10 to 40.00 g/min per Kg of inert nuclei.

In an embodiment, the process for the preparation of the multiple unit oral dosage form of the first aspect of the invention comprises:
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by continuously or discontinuously spraying a liquid mixture comprising from 1.0 to 7.5 % by weight of the one or more enteric coating agents, from 10.0 to 35.0 % by weight of the one or more modified release coating agents in a weight ratio between them from 5:95 to 30:70, and optionally one or more pharmaceutically acceptable excipients; and
simultaneously or alternately adding a mixture in powder form comprising the one or more anticaking agents, optionally one or more pore-forming agents, and optionally one or more pharmaceutically acceptable excipients,
wherein: the spray average flow rate of the mixture comprising the coating is from 0.30 to 5.00 g/min per kg of pharmaceutically acceptable inert nuclei; the average of the solid addition rate of the mixture in solid form is from 0.05 to 1.50 g/min per Kg of pharmaceutically acceptable inert nuclei; and the relation between the spray average flow rate of the mixture comprising the coating agents and the average of the solid addition rate of the mixture in solid form is from 90:10 to 60:40, particularly from 90:10 to 70:30; and
(b1) preparing the second plurality of modified release pellets of pyridoxine or a pharmaceutically acceptable salt thereof by coating the pellets of pyridoxine or a pharmaceutically acceptable salt thereof having the inner active coating layer by continuously or discontinuously spraying a liquid mixture comprising from 1.0 to 7.5 % by weight of the one or more enteric coating agents, from 10.0 to 35.0 % by weight of the one or more modified release coating agents in a weight ratio from 5:95 to 30:70; and optionally one or more pharmaceutically acceptable excipients; and simultaneously or alternately adding a mixture in powder form comprising one or more anticaking agents, optionally one or more pore-forming agents, and optionally one or more pharmaceutically acceptable excipients,
wherein: the spray average flow rate of the mixture comprising the coating agents is from 0.30 to 5.00 g/min per kg of pharmaceutically acceptable inert nuclei; the average of the solid addition rate of the mixture in solid form is from 0.10 to 2.25 g/min per Kg of pharmaceutically acceptable inert nuclei; and the relation between the spray average flow rate of the mixture comprising the coating agents and the average of the solid addition rate of the mixture in solid form is from 90:10 to 60:40, particularly from 80:20 to 60:40.

In an embodiment, the process for the preparation of the multiple unit oral dosage form of the first aspect of the invention comprises:
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by continuously or discontinuously spraying a liquid mixture comprising from 1.0 to 7.5 % by weight of the one or more enteric coating agents, from 10.0 to 35.0 % by weight of the one or more modified release coating agents in a weight ratio between them from 5:95 to 30:70, and optionally one or more pharmaceutically acceptable excipients; and
simultaneously or alternately adding a mixture in powder form comprising the one or more anticaking agents, optionally one or more pore-forming agents, and optionally one or more pharmaceutically acceptable excipients,
wherein: the spray local flow rate of the mixture comprising the coating is from 0.30 to 9.00 g/min per kg of pharmaceutically acceptable inert nuclei, and the local solid addition rate of the mixture in solid form is from 0.95 to 40.00 g/min per Kg of inert nuclei; and
(b1) preparing the second plurality of modified release pellets of pyridoxine or a pharmaceutically acceptable salt thereof by coating the pellets of pyridoxine or a pharmaceutically acceptable salt thereof having the inner active coating layer by continuously or discontinuously spraying a liquid mixture comprising from 1.0 to 7.5 % by weight of the one or more enteric coating agents, from 10.0 to 35.0 % by weight of the one or more modified release coating agents in a weight ratio from 5:95 to 30:70; and optionally one or more pharmaceutically acceptable excipients; and simultaneously or alternately adding a mixture in powder form comprising one or more anticaking agents, optionally one or more pore-forming agents, and optionally one or more pharmaceutically acceptable excipients, wherein: the spray local flow rate of the mixture comprising the coating is from 0.30 to 9.00 g/min per kg of pharmaceutically acceptable inert nuclei, and the local solid addition rate of the mixture in solid form is from 0.10 to 40.00 g/min per Kg of inert nuclei.

The term "spray average flow rate" refers to the average rate of spraying during a cycle, which corresponds also to the average rate of spraying of the corresponding coating step, understanding it as the result of dividing the total amount sprayed during a cycle by the period of time required to complete this cycle, which is the same result of dividing the total amount of liquid mixture to be sprayed during a coating step by the time required to complete the coating step, commonly expressed in g/min or in this document also in g/min per kg of pharmaceutically acceptable inert nuclei. The spray average flow rate can be measured by any known method of the state of the art. And, the term "spray local flow rate" refers to the real flow rate, considering one or more guns working in parallel at the same time in a gun system, that the combination (sum) of all the guns of this gun system is offering when the guns are working (spraying). The spray local flow rate can be measured by any known method of the state of the art. For the purpose of the present invention the "spray local flow rate" is measured by a previous calibration of the corresponding pump of the spraying guns, for a certain pumping value, dividing the sprayed volume by the time the pump is working, or using a massic sensor or a flowmeter during the spraying period of time. Thus, the difference between the "spray average flow rate" and the "spray local flow rate" is that the local flow rate is the real flow rate the guns system is offering when is working (spraying) and the average spraying flow rate is the average during a cycle or the coating step (g of sprayed liquid mixture per minute). Obviously, the average spraying flow rate is lower than the local spraying flow rate when the liquid mixture is sprayed discontinuously and equal when the liquid mixture is sprayed continuously.

The term "average of the solid addition rate" refers to the average rate of addition of a solid mixture in powder form during a cycle, which corresponds also to the average rate of powder addition of the corresponding coating step, understanding it as the result of dividing the total added amount during a cycle by the period of time required to complete this cycle, which is the same result of dividing the total amount of solid/powder/mixture to be added during a coating step by the time required to complete this coating step, commonly expressed in g/min or in this document also in g/min per kg of pharmaceutically acceptable inert nuclei. The average of the solid addition rate can be measured by any known method of the state of the art. And, the term "local solid addition rate" refers to the real solid addition rate the corresponding powder screw feeder is offering, or even the addition rate which corresponds to the amount added by hands in the short period of time required to add the solid by hands. The local solid addition rate can be measured by any known method of the state of the art. For the purpose of the present invention the "local solid addition rate" is measured by a previous calibration of the corresponding screw feeder or by weighting the amount of powder added by hands during the period of time required for the local addition. Thus, the difference between the "average of the solid addition rate" and the "local solid addition rate" is that the local powder addition rate is the real addition rate the screw feeder is offering or the addition by hands (with a shovel) is also offering when the powder addition is working, and the average powder addition rate is the average during a cycle or during the coating step (g of added powder per minute). Obviously, the average powder addition rate is lower than the local powder addition rate when the liquid mixture is sprayed discontinuously and the powder is added simultaneously or alternately, and when the liquid mixture is sprayed continuously but the powder is added alternately, and is equal than the local powder addition rate only when the liquid mixture is sprayed continuously, and the powder is added simultaneously.

The expression "relation between the spray average flow rate of the mixture comprising the coating agents and the average of the solid addition rate" refers to the relationship between both rates. This relation can be measured by dividing one by the other. And, the expression "relation between the spray local flow rate of the mixture comprising the coating agents and the local solid addition rate" refers to refers to the relationship between both rates. This relation can be measured by dividing one by the other.

As it is disclosed above, the multiple unit dosage form of the first aspect of the invention as defined above optionally comprises an intermediate enteric release coating layer comprising one or more enteric coating agents, one or more anticaking agents, optionally one or more pore-forming agent; and optionally one or more pharmaceutically acceptable excipients.

In an embodiment, the process of the invention comprises preparing a multiple unit dosage form of the first aspect of the invention as defined above comprises an intermediate enteric release coating layer comprising one or more enteric coating agents, one or more anticaking agents, optionally one or more pore-forming agent; and optionally one or more pharmaceutically acceptable excipients.

In an embodiment, wherein the multiple unit dosage form of the first aspect of the invention as defined above comprises an intermediate enteric release coating layer, then the process further comprises a previous step of coating separately:
(a2) the pellets of doxylamine or a pharmaceutically acceptable salt thereof having the inner active coating layer by continuously or discontinuously spraying a liquid mixture comprising the one or more enteric coating agents, and optionally one or more pharmaceutically acceptable excipients; and
(b2) the pharmaceutically acceptable inert nucleus by continuously or discontinuously spraying a liquid mixture comprising one or more coating agents, the therapeutically effective amount of pyridoxine or a pharmaceutically acceptable salt thereof, and optionally one or more pharmaceutically acceptable excipients.

In an embodiment, wherein the multiple unit dosage form of the first aspect of the invention as defined above comprises an intermediate enteric release coating layer, then the process further comprises a previous step of coating separately:
(a2) the pellets of doxylamine or a pharmaceutically acceptable salt thereof having the inner active coating layer by continuously or discontinuously spraying a liquid mixture comprising the one or more enteric coating agents, and optionally one or more pharmaceutically acceptable excipients; and simultaneously or alternately adding a mixture in solid form comprising the one or more anticaking agents, optionally the one or more pore-forming agents, and optionally one or more pharmaceutically acceptable excipients; and
(b2) the pharmaceutically acceptable inert nucleus by continuously or discontinuously spraying a liquid mixture comprising one or more coating agents, and optionally one or more pharmaceutically acceptable excipients; and simultaneously or alternately adding in powder form the therapeutically effective amount of pyridoxine or a pharmaceutically acceptable salt thereof, and optionally one or more pharmaceutically acceptable excipients.

In an embodiment, wherein the multiple unit dosage form of the first aspect of the invention as defined above comprises an intermediate enteric release coating layer, then the process further comprises a previous step of coating separately:
(a2) the pellets of doxylamine or a pharmaceutically acceptable salt thereof having the inner active coating layer by continuously or discontinuously spraying a liquid mixture comprising from 5 to 15% by weight of the one or more enteric coating agents, and optionally one or more pharmaceutically acceptable excipients; and simultaneously or alternately adding a mixture in powder form from 5.0 to 6.5 g per kg of pharmaceutically acceptable inert nuclei of the mixture in solid form comprising the one or more anticaking agents, optionally the one or more pore-forming agents, and optionally one or more pharmaceutically acceptable excipients; and
(b2) the pharmaceutically acceptable inert nucleus with a simultaneously or alternately spraying a liquid mixture comprising from 20% to 45% by weight of one or more coating agents, and optionally one or more pharmaceutically acceptable excipients; and simultaneously or alternately adding in powder form the therapeutically effective amount of pyridoxine or a pharmaceutically acceptable salt thereof, and optionally one or more pharmaceutically acceptable excipients.

In an embodiment, wherein the multiple unit dosage form of the first aspect of the invention as defined above comprises an intermediate enteric release coating layer, then the process further comprises a previous step of coating separately:
(a2) the pellets of doxylamine or a pharmaceutically acceptable salt thereof having the inner active coating layer by continuously or discontinuously spraying a liquid mixture comprising the one or more enteric coating agents, and optionally one or more pharmaceutically acceptable excipients; and simultaneously or alternately adding a mixture in solid form comprising the one or more anticaking agents, optionally the one or more pore-forming agents, and optionally one or more pharmaceutically acceptable excipients; wherein: the average of the spray flow rate of the mixture comprising the enteric coating agents is from 0.30 to 3.00 g/min per kg of pharmaceutically acceptable inert nuclei; and
(b2) the pharmaceutically acceptable inert nucleus by continuously or discontinuously spraying a liquid mixture comprising one or more coating agents, and optionally one or more pharmaceutically acceptable excipients; and simultaneously or alternately adding in powder form the therapeutically effective amount of pyridoxine or a pharmaceutically acceptable salt thereof, and optionally one or more pharmaceutically acceptable excipients; wherein: the average spray flow rate of the mixture comprising the coating agents is from 0.30 to 4.50 g/min per Kg of pharmaceutically acceptable inert nuclei.

In an embodiment, wherein the multiple unit dosage form of the first aspect of the invention as defined above comprises an intermediate enteric release coating layer, then the process further comprises a previous step of coating separately:
(a2) the pellets of doxylamine or a pharmaceutically acceptable salt thereof having the inner active coating layer by continuously or discontinuously spraying a liquid mixture comprising the one or more enteric coating agents, and optionally one or more pharmaceutically acceptable excipients; and simultaneously or alternately adding a mixture in solid form comprising the one or more anticaking agents, optionally the one or more pore-forming agents, and optionally one or more pharmaceutically acceptable excipients; wherein: the local spray flow rate of the mixture comprising the enteric coating agents is from 0.3 to 8.0 g/min per kg of pharmaceutically acceptable inert nuclei; and
(b2) the pharmaceutically acceptable inert nucleus by continuously or discontinuously spraying a liquid mixture comprising one or more coating agents, and optionally one or more pharmaceutically acceptable excipients; and simultaneously or alternately adding in powder form the therapeutically effective amount of pyridoxine or a pharmaceutically acceptable salt thereof, and optionally one or more pharmaceutically acceptable excipients; wherein: the local spray flow rate of the mixture comprising the coating agents is from 0.30 to 9.0 g/min per Kg of pharmaceutically acceptable inert nuclei.

In an embodiment, wherein the multiple unit dosage form of the first aspect of the invention as defined above comprises an intermediate enteric release coating layer, then the process further comprises a previous step of coating separately:
(a2) the pellets of doxylamine or a pharmaceutically acceptable salt thereof having the inner active coating layer by continuously or discontinuously spraying a liquid mixture comprising the one or more enteric coating agents, and optionally one or more pharmaceutically acceptable excipients; and simultaneously or alternately adding a mixture in solid form comprising the one or more anticaking agents, optionally the one or more pore-forming agents, and optionally one or more pharmaceutically acceptable excipients; wherein: the average of the solid addition rate of the mixture in solid form is from 0.025 to 0.40 g/min per Kg of pharmaceutically acceptable inert nuclei; and
(b2) the pharmaceutically acceptable inert nucleus by continuously or discontinuously spraying a liquid mixture comprising one or more coating agents, and optionally one or more pharmaceutically acceptable excipients; and simultaneously or alternately adding in powder form the therapeutically effective amount of pyridoxine or a pharmaceutically acceptable salt thereof, and optionally one or more pharmaceutically acceptable excipients; wherein: the average of the solid addition rate of the powder is from 0.50 to 9.0 g/min per Kg of pharmaceutically acceptable inert nuclei.

In an embodiment, wherein the multiple unit dosage form of the first aspect of the invention as defined above comprises an intermediate enteric release coating layer, then the process further comprises a previous step of coating separately:
(a2) the pellets of doxylamine or a pharmaceutically acceptable salt thereof having the inner active coating layer by continuously or discontinuously spraying a liquid mixture comprising the one or more enteric coating agents, and optionally one or more pharmaceutically acceptable excipients; and simultaneously or alternately adding a mixture in solid form comprising the one or more anticaking agents, optionally the one or more pore-forming agents, and optionally one or more pharmaceutically acceptable excipients; wherein: the local solid addition rate of the mixture in solid form is from 0.025 to 40.0 g/min per Kg of pharmaceutically acceptable inert nuclei; and
(b2) the pharmaceutically acceptable inert nucleus by continuously or discontinuously spraying a liquid mixture comprising one or more coating agents, and optionally one or more pharmaceutically acceptable excipients; and simultaneously or alternately adding in powder form the therapeutically effective amount of pyridoxine or a pharmaceutically acceptable salt thereof, and optionally one or more pharmaceutically acceptable excipients; wherein: the local solid addition rate of the powder is from 0.95 to 40.0 g/min per Kg of pharmaceutically acceptable inert nuclei.

In an embodiment, wherein the multiple unit dosage form of the first aspect of the invention as defined above comprises an intermediate enteric release coating layer, then the process further comprises a previous step of coating separately:
(a2) the pellets of doxylamine or a pharmaceutically acceptable salt thereof having the inner active coating layer by continuously or discontinuously spraying a liquid mixture comprising the one or more enteric coating agents, and optionally one or more pharmaceutically acceptable excipients; and simultaneously or alternately adding a mixture in solid form comprising the one or more anticaking agents, optionally the one or more pore-forming agents, and optionally one or more pharmaceutically acceptable excipients; wherein: the relation between the average spray flow rate of the mixture comprising the coating agents and the average of solid addition rate of the mixture in solid form is from 85:15 to 95:5; and
(b2) the pharmaceutically acceptable inert nucleus by continuously or discontinuously spraying a liquid mixture comprising one or more coating agents, and optionally one or more pharmaceutically acceptable excipients; and simultaneously or alternately adding in powder form the therapeutically effective amount of pyridoxine or a pharmaceutically acceptable salt thereof, and optionally one or more pharmaceutically acceptable excipients; wherein: the relation between the average spray flow rate of the mixture comprising the coating agents and the average of the solid addition rate is from 25:75 to 40:60.

In an embodiment, wherein the multiple unit dosage form of the first aspect of the invention as defined above comprises an intermediate enteric release coating layer, then the process further comprises a previous step of coating separately:
(a2) the pellets of doxylamine or a pharmaceutically acceptable salt thereof having the inner active coating layer by continuously or discontinuously spraying a liquid mixture comprising the one or more enteric coating agents, and optionally one or more pharmaceutically acceptable excipients; and simultaneously or alternately adding a mixture in solid form comprising the one or more anticaking agents, optionally the one or more pore-forming agents, and optionally one or more pharmaceutically acceptable excipients; wherein: the average of the spray flow rate of the mixture comprising the enteric coating agents is from 0.30 to 3.00 g/min per kg of pharmaceutically acceptable inert nuclei, the average of the solid addition rate of the mixture in solid form is from 0.025 to 0.40 g/min per Kg of pharmaceutically acceptable inert nuclei; and the relation between the average spray flow rate of the mixture comprising the coating agents and the average of solid addition rate of the mixture in solid form is from 85:15 to 95:5; and
(b2) the pharmaceutically acceptable inert nucleus by continuously or discontinuously spraying a liquid mixture comprising one or more coating agents, and optionally one or more pharmaceutically acceptable excipients; and simultaneously or alternately adding in powder form the therapeutically effective amount of pyridoxine or a pharmaceutically acceptable salt thereof, and optionally one or more pharmaceutically acceptable excipients; wherein: the average spray flow rate of the mixture comprising the coating agents is from 0.30 to 4.50 g/min per Kg of pharmaceutically acceptable inert nuclei, the average of the solid addition rate of the powder is from 0.50 to 9.0 g/min per Kg of pharmaceutically acceptable inert nuclei, and the relation between the average spray flow rate of the mixture comprising the coating agents and the average of the solid addition rate is from 25:75 to 40:60.

In an embodiment, wherein the multiple unit dosage form of the first aspect of the invention as defined above comprises an intermediate enteric release coating layer, then the process further comprises a previous step of coating separately:
(a2) the pellets of doxylamine or a pharmaceutically acceptable salt thereof having the inner active coating layer by continuously or discontinuously spraying a liquid mixture comprising the one or more enteric coating agents, and optionally one or more pharmaceutically acceptable excipients; and simultaneously or alternately adding a mixture in solid form comprising the one or more anticaking agents, optionally the one or more pore-forming agents, and optionally one or more pharmaceutically acceptable excipients; wherein: the local spray flow rate of the mixture comprising the enteric coating agents is from 0.3 to 8.0 g/min per kg of pharmaceutically acceptable inert nuclei and the local solid addition rate of the mixture in solid form is from 0.025 to 40.0 g/min per Kg of pharmaceutically acceptable inert nuclei; and
(b2) the pharmaceutically acceptable inert nucleus by continuously or discontinuously spraying a liquid mixture comprising one or more coating agents, and optionally one or more pharmaceutically acceptable excipients; and simultaneously or alternately adding in powder form the therapeutically effective amount of pyridoxine or a pharmaceutically acceptable salt thereof, and optionally one or more pharmaceutically acceptable excipients; wherein: the local spray flow rate of the mixture comprising the coating agents is from 0.30 to 9.0 g/min per Kg of pharmaceutically acceptable inert nuclei and the local solid addition rate of the powder is from 0.95 to 40.0 g/min per Kg of pharmaceutically acceptable inert nuclei.

In an embodiment, wherein the multiple unit dosage form of the first aspect of the invention as defined above comprises an intermediate enteric release coating layer, then the process further comprises a previous step of coating separately:
(a2) the pellets of doxylamine or a pharmaceutically acceptable salt thereof having the inner active coating layer by continuously or discontinuously spraying a liquid mixture comprising from 5 to 15% by weight of the one or more enteric coating agents, and optionally one or more pharmaceutically acceptable excipients; and simultaneously or alternately adding a mixture in powder form from 5.0 to 6.5 g per kg of pharmaceutically acceptable inert nuclei of the mixture in solid form comprising the one or more anticaking agents, optionally the one or more pore-forming agents, and optionally one or more pharmaceutically acceptable excipients; wherein: the average of the spray flow rate of the mixture comprising the enteric coating agents is from 0.30 to 3.00 g/min per kg of pharmaceutically acceptable inert nuclei; the average of the solid addition rate of the mixture in solid form is from 0.025 to 0.400 g/min per Kg of pharmaceutically acceptable inert nuclei; and the relation between the average spray flow rate of the mixture comprising the coating agents and the average of solid addition rate of the mixture in solid form is from 85:15 to 95:5; and
(b2) the pharmaceutically acceptable inert nucleus with a simultaneously or alternately spraying a liquid mixture comprising from 20% to 45% by weight of one or more coating agents, and optionally one or more pharmaceutically acceptable excipients; and simultaneously or alternately adding in powder form the therapeutically effective amount of pyridoxine or a pharmaceutically acceptable salt thereof, and optionally one or more pharmaceutically acceptable excipients: wherein the average spray flow rate of the mixture comprising the coating agents is from 0.30 to 4.50 g/min per Kg of pharmaceutically acceptable inert nuclei; the average of the solid addition rate of the powder is from 0.50 to 9.00 g/min per Kg of pharmaceutically acceptable inert nuclei; and the relation between the average spray flow rate of the mixture comprising the coating agents and the average of the solid addition rate is from 25:75 to 40:60.

In an embodiment, wherein the multiple unit dosage form of the first aspect of the invention as defined above comprises an intermediate enteric release coating layer, then the process further comprises a previous step of coating separately:
(a2) the pellets of doxylamine or a pharmaceutically acceptable salt thereof having the inner active coating layer by continuously or discontinuously spraying a liquid mixture comprising from 5 to 15% by weight of the one or more enteric coating agents, and optionally one or more pharmaceutically acceptable excipients; and simultaneously or alternately adding a mixture in powder form from 5.0 to 6.5 g per kg of pharmaceutically acceptable inert nuclei of the mixture in solid form comprising the one or more anticaking agents, optionally the one or more pore-forming agents, and optionally one or more pharmaceutically acceptable excipients; wherein: the local spray flow rate of the mixture comprising the enteric coating agents is from 0.3 to 8.0 g/min per kg of pharmaceutically acceptable inert nuclei and the local solid addition rate of the mixture in solid form is from 0.025 to 40.0 g/min per Kg of pharmaceutically acceptable inert nuclei; and
(b2) the pharmaceutically acceptable inert nucleus with a simultaneously or alternately spraying a liquid mixture comprising from 20% to 45% by weight of one or more coating agents, and optionally one or more pharmaceutically acceptable excipients; and simultaneously or alternately adding in powder form the therapeutically effective amount of pyridoxine or a pharmaceutically acceptable salt thereof, and optionally one or more pharmaceutically acceptable excipients, wherein: the local spray flow rate of the mixture comprising the coating agents is from 0.30 to 9.0 g/min per Kg of pharmaceutically acceptable inert nuclei and the local solid addition rate of the powder is from 0.95 to 40.0 g/min per Kg of pharmaceutically acceptable inert nuclei.

In an embodiment, the process of the invention comprises preparing a multiple unit dosage form of the first aspect of the invention as defined above which does not comprises an intermediate enteric release coating layer comprising one or more enteric coating agents, one or more anticaking agents, optionally one or more pore-forming agent; and optionally one or more pharmaceutically acceptable excipients. In an embodiment, wherein the multiple unit dosage form of the first aspect of the invention as defined above does not comprises the intermediate enteric release coating layer, then the process further comprises performing step (b2) as defined above. All the embodiments disclosed above for step (b2) of the process for preparing a multiple dosage form of the present invention comprising the intermediate enteric release coating layer also apply for the process for preparing the multiple unit dosage form which does not comprises the intermediate enteric release coating layer.

In an embodiment, the process for the preparation of the multiple unit oral dosage form of the first aspect of the invention as defined above further comprises a previous step (a3) of coating the pharmaceutically acceptable inert nucleus with a continuously or discontinuously spraying a liquid mixture comprising one or more coating agents, the therapeutically effective amount of doxylamine or a pharmaceutically acceptable salt thereof, and optionally one or more pharmaceutically acceptable excipients.

In an embodiment, the process for the preparation of the multiple unit oral dosage form of the first aspect of the invention as defined above further comprises a previous step (a3) of coating the pharmaceutically acceptable inert nucleus with a continuously or discontinuously spraying a liquid mixture comprising one or more coating agents, and optionally one or more pharmaceutically acceptable excipients; and simultaneously or alternately adding a mixture in powder form comprising the therapeutically effective amount of doxylamine or a pharmaceutically acceptable salt thereof, one or more anticaking, optionally one or more pore-forming agents, and optionally one or more pharmaceutically acceptable excipients.

In an embodiment, the process for the preparation of the multiple unit oral dosage form of the first aspect of the invention as defined above further comprises a previous step (a3) of coating the pharmaceutically acceptable inert nucleus with a continuously or discontinuously spraying a liquid mixture comprising from 15% to 40% by weight of one or more coating agents, and optionally one or more pharmaceutically acceptable excipients; and simultaneously or alternately adding a mixture in powder form comprising the therapeutically effective amount of doxylamine or a pharmaceutically acceptable salt thereof, from 18 to 36% by weight of the one or more anticaking, optionally one or more pore-forming agents, and optionally one or more pharmaceutically acceptable excipients.

In an embodiment, the process for the preparation of the multiple unit oral dosage form of the first aspect of the invention as defined above further comprises a previous step (a3) of coating the pharmaceutically acceptable inert nucleus with a continuously or discontinuously spraying a liquid mixture comprising one or more coating agents, and optionally one or more pharmaceutically acceptable excipients; and simultaneously or alternately adding a mixture in powder form comprising the therapeutically effective amount of doxylamine or a pharmaceutically acceptable salt thereof, one or more anticaking, optionally one or more pore-forming agents, and optionally one or more pharmaceutically acceptable excipients, wherein: the average spray flow rate of the mixture comprising the coating agents is from 0.30 to 4.50 g/min per Kg of pharmaceutically acceptable inert nuclei.

In an embodiment, the process for the preparation of the multiple unit oral dosage form of the first aspect of the invention as defined above further comprises a previous step (a3) of coating the pharmaceutically acceptable inert nucleus with a continuously or discontinuously spraying a liquid mixture comprising one or more coating agents, and optionally one or more pharmaceutically acceptable excipients; and simultaneously or alternately adding a mixture in powder form comprising the therapeutically effective amount of doxylamine or a pharmaceutically acceptable salt thereof, one or more anticaking, optionally one or more pore-forming agents, and optionally one or more pharmaceutically acceptable excipients, wherein: the local spray flow rate of the mixture comprising the coating agents is from 0.30 to 9.0 g/min per Kg of pharmaceutically acceptable inert nuclei.

In an embodiment, the process for the preparation of the multiple unit oral dosage form of the first aspect of the invention as defined above further comprises a previous step (a3) of coating the pharmaceutically acceptable inert nucleus with a continuously or discontinuously spraying a liquid mixture comprising one or more coating agents, and optionally one or more pharmaceutically acceptable excipients; and simultaneously or alternately adding a mixture in powder form comprising the therapeutically effective amount of doxylamine or a pharmaceutically acceptable salt thereof, one or more anticaking, optionally one or more pore-forming agents, and optionally one or more pharmaceutically acceptable excipients, wherein: the average of the solid addition rate of the mixture in powder form is from 0.95 to 18.0 g/min per Kg of pharmaceutically acceptable inert nuclei.

In an embodiment, the process for the preparation of the multiple unit oral dosage form of the first aspect of the invention as defined above further comprises a previous step (a3) of coating the pharmaceutically acceptable inert nucleus with a continuously or discontinuously spraying a liquid mixture comprising one or more coating agents, and optionally one or more pharmaceutically acceptable excipients; and simultaneously or alternately adding a mixture in powder form comprising the therapeutically effective amount of doxylamine or a pharmaceutically acceptable salt thereof, one or more anticaking, optionally one or more pore-forming agents, and optionally one or more pharmaceutically acceptable excipients, wherein: the local solid addition rate of the mixture in powder form is from 0.95 to 40.0 g/min per Kg of pharmaceutically acceptable inert nuclei.

In an embodiment, the process for the preparation of the multiple unit oral dosage form of the first aspect of the invention as defined above further comprises a previous step (a3) of coating the pharmaceutically acceptable inert nucleus with a continuously or discontinuously spraying a liquid mixture comprising one or more coating agents, and optionally one or more pharmaceutically acceptable excipients; and simultaneously or alternately adding a mixture in powder form comprising the therapeutically effective amount of doxylamine or a pharmaceutically acceptable salt thereof, one or more anticaking, optionally one or more pore-forming agents, and optionally one or more pharmaceutically acceptable excipients, wherein: the relation between the average spray flow rate of the mixture comprising the coating agents and the average of the solid addition rate of the mixture in powder form is from 15:85 to 30:70.

In an embodiment, the process for the preparation of the multiple unit oral dosage form of the first aspect of the invention as defined above further comprises a previous step (a3) of coating the pharmaceutically acceptable inert nucleus with a continuously or discontinuously spraying a liquid mixture comprising one or more coating agents, and optionally one or more pharmaceutically acceptable excipients; and simultaneously or alternately adding a mixture in powder form comprising the therapeutically effective amount of doxylamine or a pharmaceutically acceptable salt thereof, one or more anticaking, optionally one or more pore-forming agents, and optionally one or more pharmaceutically acceptable excipients, wherein: the average spray flow rate of the mixture comprising the coating agents is from 0.30 to 4.50 g/min per Kg of pharmaceutically acceptable inert nuclei and the average of the solid addition rate of the mixture in powder form is from 0.95 to 18.0 g/min per Kg of pharmaceutically acceptable inert nuclei; and particularly the relation between the average spray flow rate of the mixture comprising the coating agents and the average of the solid addition rate of the mixture in powder form is from 15:85 to 30:70.

In an embodiment, the process for the preparation of the multiple unit oral dosage form of the first aspect of the invention as defined above further comprises a previous step (a3) of coating the pharmaceutically acceptable inert nucleus with a continuously or discontinuously spraying a liquid mixture comprising one or more coating agents, and optionally one or more pharmaceutically acceptable excipients; and simultaneously or alternately adding a mixture in powder form comprising the therapeutically effective amount of doxylamine or a pharmaceutically acceptable salt thereof, one or more anticaking, optionally one or more pore-forming agents, and optionally one or more pharmaceutically acceptable excipients, wherein: the local spray flow rate of the mixture comprising the coating agents is from 0.30 to 9.0 g/min per Kg of pharmaceutically acceptable inert nuclei and the local solid addition rate of the mixture in powder form is from 0.95 to 40.0 g/min per Kg of pharmaceutically acceptable inert nuclei.

In an embodiment, the process for the preparation of the multiple unit oral dosage form of the first aspect of the invention as defined above further comprises a previous step (a3) of coating the pharmaceutically acceptable inert nucleus with a continuously or discontinuously spraying a liquid mixture comprising from 15% to 40% by weight of one or more coating agents, and optionally one or more pharmaceutically acceptable excipients; and simultaneously or alternately adding a mixture in powder form comprising the therapeutically effective amount of doxylamine or a pharmaceutically acceptable salt thereof, from 18 to 36% by weight of the one or more anticaking, optionally one or more pore-forming agents, and optionally one or more pharmaceutically acceptable excipients; wherein: the average spray flow rate of the mixture comprising the coating agents is from 0.30 to 4.50 g/min per Kg of pharmaceutically acceptable inert nuclei; the average of the solid addition rate of the mixture in powder form is from 0.95 to 18.00 g/min per Kg of pharmaceutically acceptable inert nuclei; and the relation between the average spray flow rate of the mixture comprising the coating agents and the average of the solid addition rate of the mixture in powder form is from 15:85 to 30:70.

In an embodiment, the process for the preparation of the multiple unit oral dosage form of the first aspect of the invention as defined above further comprises a previous step (a3) of coating the pharmaceutically acceptable inert nucleus with a continuously or discontinuously spraying a liquid mixture comprising from 15% to 40% by weight of one or more coating agents, and optionally one or more pharmaceutically acceptable excipients; and simultaneously or alternately adding a mixture in powder form comprising the therapeutically effective amount of doxylamine or a pharmaceutically acceptable salt thereof, from 18 to 36% by weight of the one or more anticaking, optionally one or more pore-forming agents, and optionally one or more pharmaceutically acceptable excipients; wherein: the local spray flow rate of the mixture comprising the coating agents is from 0.30 to 9.0 g/min per Kg of pharmaceutically acceptable inert nuclei and the local solid addition rate of the mixture in powder form is from 0.95 to 40.0 g/min per Kg of pharmaceutically acceptable inert nuclei.

In an embodiment, the process for the preparation of the multiple unit oral dosage form of the first aspect of the invention comprises preparing the first plurality of modified release pellets of doxylamine or a pharmaceutically acceptable salt thereof by coating the pellets of doxylamine or a pharmaceutically acceptable salt thereof by performing steps (a2) and (a1) as defined above. In an embodiment, the process for the preparation of the multiple unit oral dosage form of the first aspect of the invention comprises preparing the first plurality of modified release pellets of doxylamine or a pharmaceutically acceptable salt thereof by coating the pharmaceutically acceptable inert nucleus by performing steps (a3), (a2) and (a1) as defined above. In an embodiment, the process for the preparation of the multiple unit oral dosage form of the first aspect of the invention comprises preparing the first plurality of modified release pellets of doxylamine or a pharmaceutically acceptable salt thereof by coating the pellets of doxylamine or a pharmaceutically acceptable salt thereof by performing steps (a3) and (a1) as defined above.

In an embodiment, the process for the preparation of the multiple unit oral dosage form of the first aspect of the invention comprises preparing the second plurality of modified release pellets of pyridoxine or a pharmaceutically acceptable salt thereof by coating the pharmaceutically acceptable inert nucleus performing steps (b2) and (b1).

In an embodiment, the process for the preparation of the multiple unit oral dosage form of the first aspect of the invention comprises preparing separately: the first plurality of modified release pellets of doxylamine or a pharmaceutically acceptable salt thereof by coating the pellets of doxylamine or a pharmaceutically acceptable salt thereof by performing steps (a2) and (a1) as defined above; and the second plurality of modified release pellets of pyridoxine or a pharmaceutically acceptable salt thereof by coating pharmaceutically acceptable inert nucleus performing steps (b2) and (b1).

In an embodiment, the process for the preparation of the multiple unit oral dosage form of the first aspect of the invention comprises preparing separately: the first plurality of modified release pellets of doxylamine or a pharmaceutically acceptable salt thereof by coating the pharmaceutically acceptable inert nucleus performing steps (a3), (a2) and (a1) as defined above; and the second plurality of modified release pellets of pyridoxine or a pharmaceutically acceptable salt thereof by coating the pharmaceutically acceptable inert nucleus performing steps (b2) and (b1).

In an embodiment, the process for the preparation of the multiple unit oral dosage form of the first aspect of the invention comprises preparing separately: the first plurality of modified release pellets of doxylamine or a pharmaceutically acceptable salt thereof by coating the pharmaceutically acceptable inert nucleus performing steps (a3) and (a1) as defined above; and the second plurality of modified release pellets of pyridoxine or a pharmaceutically acceptable salt thereof by coating the pharmaceutically acceptable inert nucleus performing steps (b2) and (b1).

In an embodiment, each one of the coating steps of the process of the invention are performed at an air temperature from 5°C to 80°C; particularly from 20°C to 70°C; more particularly from 25°C to 65°C and much more particularly from 25°C to 55°C. In an embodiment, each one of the coating steps of the process of the invention are performed at an airflow from 0 a 20 m³/h per Kg of pharmaceutically acceptable inert nuclei; particularly from 0 to 6 m³ /h per Kg of pharmaceutically acceptable inert nuclei. The airflow is controlled using an anemometer type air inlet detection system. In an embodiment, each one of the coating steps of the process of the invention are performed at an air temperature from 5°C to 80°C and at an airflow from 0 a 20 m³/h per Kg of pharmaceutically acceptable inert nuclei.

In an embodiment, each one of the coating steps of the process of the invention which comprises a spraying mixture of one or more enteric coating agents and one or more modified release coating agents as defined above; particularly the enteric coating is methacrylic acid-methyl methacrylate copolymer; and the modified release coating agent is shellac. In an embodiment, each one of the coating steps of the process of the invention which comprises a spraying mixture of one or more enteric coating agents and one or more modified release coating agents, the enteric coating is methacrylic acid-methyl methacrylate copolymer, particularly methacrylic acid-methyl methacrylate copolymer (1:1) (Eudragit L); and the modified release coating agent is shellac. In an embodiment, each one of the coating steps of the process of the invention which comprises a spraying mixture comprising one or more enteric coating agents and one or more modified release coating agents, the enteric coating is methacrylic acid-methyl methacrylate copolymer, particularly methacrylic acid-methyl methacrylate copolymer (1:1) (Eudragit L); and the modified release coating agent is shellac in a weight ratio from 5:95 to 30:70; particularly from 8:92 to 20:80.

In an embodiment, in the coating step of step (a1) of the process of the invention which comprises a spraying mixture comprising one or more enteric coating agents and one or more modified release coating agents, the spraying mixture comprises from 4 to 7% by weight of the enteric coating agents, particularly methacrylic acid-methyl methacrylate copolymer (1:1) (Eudragit L) and from 15 to 25 % by weight of modified release coating agent, particularly (dewaxed) shellac. In an embodiment, in the coating step of step (a1) of the process of the invention which comprises a spraying mixture comprising one or more enteric coating agents and one or more modified release coating agents, the spraying mixture comprises from 4 to 7% by weight of the enteric coating agents, particularly methacrylic acid-methyl methacrylate copolymer (1:1) (Eudragit L) and from 15 to 25 % by weight of modified release coating agent, particularly (dewaxed) shellac in a weight ratio from 15:85 to 30:70. In an embodiment, in the coating step of step (b1) of the process of the invention which comprises a spraying mixture comprising one or more enteric coating agents and one or more modified release coating agents, the spraying mixture comprises from 2.0 to 7.5% by weight of the enteric coating agents and from 10 to 35% of the modified release coating agents; particularly from 20 to 35%. In an embodiment, in the coating step of step (b1) of the process of the invention which comprises a spraying mixture comprising one or more enteric coating agents and one or more modified release coating agents, the spraying mixture comprises from 2.0 to 7.5% by weight of the enteric coating agents and from 10 to 35% of the modified release coating agents; particularly from 20 to 35% in a weight ratio from 5:95 to 15:85.

In an embodiment, in the coating step of step (b1) of the process of the invention which comprises a spraying mixture comprising one or more enteric coating agents and one or more modified release coating agents, the spraying mixture comprises from 2.0 to 7.5% by weight of methacrylic acid-methyl methacrylate copolymer (1:1) (Eudragit L) as the enteric coating agents and from 10 to 35% by weight of (dewaxed) shellac as the modified release coating agents; particularly from 20 to 35%. In an embodiment, in the coating step of step (b1) of the process of the invention which comprises a spraying mixture comprising one or more enteric coating agents and one or more modified release coating agents, the spraying mixture comprises from 2.0 to 7.5% by weight of methacrylic acid-methyl methacrylate copolymer (1:1) (Eudragit L) as the enteric coating agents and from 10 to 35% by weight of (dewaxed) shellac as the modified release coating agents; particularly from 20 to 35% in a weight ratio from 5:95 to 15:85.

In an embodiment, in the coating step (a3) of the process of the invention, the coating agents are selected from the group consisting of polyvinylpyrrolidone, shellac, hydroxypropyl methylcellulose, hydroxypropyl cellulose, and microcrystalline cellulose and mixture thereof; particularly a mixture of polyvinylpyrrolidone and shellac. In an embodiment, in the coating step (a3) of the process of the invention, the coating agent is a mixture of polyvinylpyrrolidone, particularly K30, and shellac in a weight ratio from 20:80 to 30:70. In an embodiment, in the coating step (a3) of the process of the invention, the spraying mixture comprises from 30 to 40% by weight of one or more coating agents as defined above. In a particular embodiment, in the coating step (a3) the spraying mixture comprises from 30 to 40% by weight of a mixture of polyvinylpyrrolidone K30 and (dewaxed) shellac in a weight ratio from polyvinylpyrrolidone and shellac from 20:80 to 30:70. In a particular embodiment, in the coating step (a3) the spraying mixture comprises one or more organic solvents as defined above and below; particularly, the mixture comprises an amount of solvent concentration from 0 to 70% by weight. In a particular embodiment, in the coating step (a3) the spraying mixture is a solution of polyvinylpyrrolidone K-30 20% in ethanol and a solution of (dewaxed) shellac 40% w/w in ethanol in a weight ratio from 20:80 to 40:60; particularly 30:70.

In an embodiment, in the coating step (b2) of the process of the invention, the spraying mixture comprises from 20 to 45% of one or more coating agents. In an embodiment, in the coating step (b2) of the process of the invention, the coating agents are selected from the group consisting of microcrystalline cellulose, hydroxypropyl methyl cellulose (HPMC), polyvinyl pyrrolidone (PVP), shellac, polyethylene glycol (PEG) 6000, guar gum and starch; particularly (dewaxed) shellac; particularly shellac. In a particular embodiment, in the coating step (b2) the spraying mixture comprises from 30 to 45% by weight of a (dewaxed) shellac. In a particular embodiment, in the coating step (b2) the spraying mixture comprises one or more organic solvents as defined above and below; particularly ethanol.

In an embodiment, in the coating step (a2) of the process of the invention, the enteric coating agents are those defined above and below; particularly methacrylic acid-methyl methacrylate copolymer (Eudragit L). In an embodiment, in the coating step (a2) of the process of the invention, the spraying mixture comprises from 5 to 15% by weight of one or more enteric coating agents.

In an embodiment, in coating step (a2) of the process of the invention, the spraying mixture comprises from 5 to 15% by weight of one or more enteric coating agents, and the process comprises adding from 5.0 to 6.5 g per kg of pharmaceutically acceptable inert nuclei of the anticaking agent in powder form. In a particular embodiment, in the coating step (a2) the spraying mixture comprises one or more organic solvents as defined above and below; particularly acetone, or a mixture of acetone, ethanol and water. In a particular embodiment, in the coating step (a2) the spraying mixture comprises one or more solvents in a concentration from 85% to 95% by weight, more particularly in a 90% by weight of solvents.

In an embodiment, in each one of the coating steps (a1) and (b1) of the process of the invention which comprises a spraying mixture comprising coating agents, the spraying mixture further comprises one or more organic solvents as defined above in an amount from 85 to 1200 g of solvent per Kg of pharmaceutically acceptable inert nuclei; particularly from 85 to 650 g of solvent per Kg of pharmaceutically acceptable inert nuclei. In an embodiment, each one of the coating steps of the process of the invention which comprises a spraying mixture comprising coating agents, the spraying mixture further comprises one or more organic solvents as defined above in an amount from 270 to 285 g of solvent per Kg of pharmaceutically acceptable inert nuclei for (a1) and in an amount from 85 to 100 g of solvent per Kg of pharmaceutically acceptable inert nuclei for (b1).

In an embodiment, each one of the coating steps of the process of the invention is performed at airflow from 0 a 20 m³/h per Kg of pharmaceutically acceptable inert nuclei; particularly from 0 to 6 m3 /h per Kg of pharmaceutically acceptable inert nuclei.

In an embodiment, in each one of the coating steps of the process of the invention the temperature of the inert nucleus (core) of the pellets during the coating step is from 5°C to 50 °C; particularly from 10°C to 30°C. In an embodiment, in each one of the coating steps of the process of the invention the temperature of the inert nucleus (core) of the pellets during the coating step is from 19°C to 30 °C. The temperature is controlled using a calibrated PT100 sensor being directly in contact with the pellets being coated, but it could be controlled using an equivalent system.

In an embodiment, in each one of the spraying steps (a1), (a2), (a3), (b1) and (b2), the liquid mixture is sprayed at a gun atomization pressure from 0.6 to 2.2 bar and an open pattern pressure from 0.6 to 2.5 bar.

The coating steps of the process of the second aspect of the invention can be performed by any known method disclosed in the state of the art. In an embodiment, the coating steps are performed by a method selected from the group consisting of a pan-coating method and a fluid-bed coating method. In an embodiment, the coating steps are performed by a pan-coating method and the depression inside the pan-coating is from 0 to 200 Pa. In an embodiment, the coating steps are performed by a pan-coating method and the depression inside the pan-coating is from 0 to 100 Pa.

In an embodiment, the process of the present invention is one wherein one or more of the coating steps is performed in the absence of drying steps.

In an embodiment, the process of the present invention is one wherein one or more of the coating steps further comprises one or more drying steps. In an embodiment, the process of the present invention is one which further comprises an additional step of drying separately each one of the plurality of pellets obtained after the preparation of each coating layer (inter-coating drying steps). It means, for the first plurality of pellets after the preparation of the inner active coating layer, the intermediate enteric release coating layer, and the external modified release coating layer; and for the second plurality of pellets after the preparation of the inner active coating layer and/ or the external modified release coating layer.

In an embodiment, the each one of the drying steps of the process of the invention is performed at a temperature from 15 °C to 60 °C; particularly from 25°C to 45°C. In an embodiment, the each one of the drying steps of the process of the invention is performed at an airflow equal to or higher than 1 m³/ (h per kg of inert nuclei) for the appropriate period of time for having an amount of each of the solvents lower than 5000 ppm. In an embodiment, the each one of the drying steps of the process of the invention is performed at a temperature from 15 °C to 60 °C and at an airflow equal to or higher than 1 m³/ (h per kg of inert nuclei) for the appropriate period of time for having an amount of each of the solvents lower than 5000 ppm.

In a particular embodiment, the inter-coating drying steps are carried out during not less than 1 h at a temperature; particularly from 15°C to 45 °C and at an airflow higher than 1 m³/ h per kg of pharmaceutically acceptable inert nuclei. In a particular embodiment, the drying step of the external coating of both plurality of pellets is carried out during not less than 8 h and not more than 12 h; particularly at a temperature from 15 °C to 60 °C, preferably from 25°C to 50 °C; more particularly from 40°C to 45 °C at an airflow higher than 2 m³ / h per kg of pharmaceutically acceptable inert nuclei.

In a particular embodiment, the drying steps of the process of the invention is performed in any appropriate equipment; particularly in a coating pan at a speed from 0 to 10rpm.

In an embodiment, the coating steps of the process of the invention is performed in a coating pan and at a rotation speed from 0 to 50 rpm, particularly from 2 to 25 rpm; more particularly from 10 to 2 rpm. The rotation pan speed can be controlled by any method known in the state of the art. In particular, the method used in the present invention is by using a revolution counter.

In an embodiment, the process of the present invention further comprises one or more additional steps of sieving separately each one of the plurality of pellets obtained in each one of the coating steps if agglomeration of powder into granules is observed. In an embodiment, the process of the present invention further comprises one or more additional steps of sieving separately the first plurality of modified release pellets of doxylamine or a pharmaceutically acceptable salt thereof comprising: the pharmaceutically acceptable inert nucleus; the inner active coating layer; the intermediate enteric release coating layer, and the external modified release coating layer; and/or the second plurality of modified release pellets of pyridoxine or a pharmaceutically acceptable salt thereof comprising: the pharmaceutically acceptable inert nucleus; the inner active coating layer; and the external modified release coating layer.

In an embodiment, wherein when the multiple unit oral dosage form is a hard capsule, then the process of the present invention further comprises an additional step of filling a hard capsule with a first plurality of modified release pellets of doxylamine or a pharmaceutically acceptable salt thereof; and a second plurality of modified release pellets of pyridoxine or a pharmaceutically acceptable salt thereof. The process of filling the capsule can be performed using the known method disclosed in the state of the art. Examples of filling machines appropriate for the present invention include, without limitation, automatic filling machine using intermittent or continuous motion. Common capsule fillers that use intermittent or continuous motion include machines from Bosch, IMA Zanasi, Dott Bonapace and MG2. In the case, of the present invention, the filling of the hard capsules is performed using an automatic filling machine using intermittent motion. In an embodiment, wherein when the multiple unit oral dosage form is a hard capsule, then the process of the present invention further comprises an additional step of filling a hard capsule comprising filling individually and separately the first plurality of modified release pellets of doxylamine or a pharmaceutically acceptable salt thereof and a second plurality of modified release pellets of pyridoxine or a pharmaceutically acceptable salt thereof. In an embodiment, wherein when the multiple unit oral dosage form is a hard capsule, then the process of the present invention further comprises an additional step of filling a hard capsule firstly, comprising: firstly filling the body of the capsule with the first plurality of modified release pellets of doxylamine or a pharmaceutically acceptable salt thereof; and secondly, filling the body of the capsule a second plurality of modified release pellets of pyridoxine or a pharmaceutically acceptable salt thereof; or vice versa; and fitting the cap of the capsule over the body of the capsule. In an embodiment, wherein when the multiple unit oral dosage form is a hard capsule, then the process of the present invention further comprises an additional step of filling a hard capsule comprising filling a combination of the first plurality of modified release pellets of doxylamine or a pharmaceutically acceptable salt thereof and a second plurality of modified release pellets of pyridoxine or a pharmaceutically acceptable salt thereof.

The use of the first and the second plurality of pellets disclosed in the present invention allows having an accuracy of the individual amount of each plurality of pellets filled into the capsule from less than or equal to ± 5% by weight of the theoretical filling weight. For the purpose of the invention, the term "theoretical filling weight" refers to the target filling weight calculated based upon actual potency of the pellets in doxylamine succinate or pyridoxine hydrochloride so as to assure the theoretical content of X mg of doxylamine succinate (where X is for example 10.0 or 20.0 mg) and Y mg (where Y is for example 10.0 or 20.0 mg) of pyridoxine hydrochloride per capsule, which can be measured by a suitable balance.

In an embodiment, wherein when the multiple unit oral dosage form is a hard capsule, then the filling capsule step comprises adding one or more pharmaceutically acceptable excipients. Examples of appropriate pharmaceutically acceptable excipients for being used in the filling capsule step are selected from lubricants, fillers, diluents, glidants and anticaking agents or a mixture thereof.

In an embodiment, wherein when the multiple unit oral dosage form is a hard capsule, then the process further comprises an additional step of filling the capsule with the first plurality of pellets comprising the therapeutically effective amount of doxylamine or a pharmaceutically acceptable salt thereof and the second plurality of pellets comprising the therapeutically effective amount of pyridoxine or a pharmaceutically acceptable salt thereof; particularly having from 5 mg to 50 mg per capsule of doxylamine succinate and from 5 mg to 50 mg per capsule of pyridoxine hydrochloride.

All the embodiment disclosed above and below for the multiple unit oral dosage form of the first aspect of the invention also apply for the process for its preparation of the second aspect of the invention.

The multiple unit oral dosage form of the first aspect of the invention may be defined by its preparation process as defined above in the second aspect of the invention and therefore, the multiple unit oral dosage form of the first aspect of the invention obtainable by the process of the invention is considered part of the invention. For the purposes of the invention, the expressions "obtainable", "obtained" and equivalent expressions are used interchangeably, and in any case, the expression "obtainable" encompasses the expression "obtained".

All the embodiments disclosed above for the multiple unit oral dosage form of the first aspect of the invention, as well as for the process for its preparation, also apply for the multiple unit oral dosage form of the invention obtainable by the process for preparation.

Finally, it is also part of the invention the multiple unit oral dosage form of the first aspect of the invention for use in therapy. In particular, the multiple unit oral dosage form of the first aspect of the invention for use in the symptomatic treatment of nausea and vomiting. In an embodiment, the multiple unit oral dosage form of the first aspect of the invention for use in the symptomatic treatment of nausea and vomiting associated with pregnant women (NVP). In an embodiment, the multiple unit oral dosage form of the first aspect of the invention for use in the symptomatic treatment of nausea and vomiting associated with oncologic treatments, for instance chemotherapy or radiotherapy. This aspect could be also formulated as the use of multiple unit oral dosage form of the first aspect of the invention as defined above for the preparation of a medicament for the symptomatic treatment of nausea and vomiting. It also relates to a method for the prophylaxis and/or treatment of a mammal suffering, or susceptible to suffer, from nausea and vomiting, wherein the method comprises administering to said mammal the multiple unit oral dosage form of the first aspect of the invention as defined above. In an embodiment, the nausea and vomiting are associated with pregnant women (NVP) or with oncologic treatments, for instance chemotherapy or radiotherapy. All the embodiments disclosed above for the multiple unit oral dosage form of the first aspect of the invention also apply for the multiple unit oral dosage form of the first aspect of the invention limited by its use.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### General considerations

D90 measurement values of the active ingredients (doxylamine and pyridoxine) were performed using a Malvern method (Laser Mastersizer, Mie Theory; ISO 13320-1).

The measurement of the D90 value of doxylamine or a pharmaceutically acceptable salt was performed using the Malvern method (Laser Mastersizer, Mie Theory; ISO 13320-1) under the following main used parameters:
Sample, Dispersion Medium 250 mg, 5 mL Isopar G + 5 %w/w Lecithin
Ultrasound Time 20 seconds
Dispersant (volume) Isopar G (ca. 100 mL)
Washing Media (volume)
Acetone (ca. 50 mL), Toluene (ca. 50 mL)
and Isopar G (ca. 50 mL)
Particle Refractive Index 1,52
Particle Absorption 0,1
Dispersant Refractive Index 1,42
Analysis Model General Purpose - Normal Sensitivity
Particle Shape Irregular
Repeats 3 measurements per aliquot
Delay Between Measurements 10 Seconds
Sample Measuring Time 15 Seconds
Background Measuring Time 15 Seconds
Obscuration Range 15 - 30 %
Suspension Stirring Rate 2500 ± 10 rpm

The samples were measured employing the following procedure:
Procedure: The sample vial was subjected to a standard mixing cycle in a Bio Grant PTR-30 rotatory mixer. Approximately 250 mg of the sample were transferred into a 10 mL vial and 5 mL of 5% w/w Lecithin solution in Isopar G were added. The suspension was sonicated for 20 seconds using a Bandelin Sonopuls HD3100 with a sonication probe (MS72 tip at 20 % intensity). Under continuous circulation (2500 rpm), the sample suspension was added to the measuring medium (ca. 100 mL of Isopar G) until the obscuration value reached 15 - 30 %. Measurements were performed immediately after sample addition and after 3 minutes to ensure dispersion stability. Each result is the average of three consecutive measurements of 15000 swipes. The abovementioned procedure was carried out by triplicate.
- The measurement of the D90 value of pyridoxine or a pharmaceutically acceptable salt was performed using the Malvern method (Laser Mastersizer, Mie Theory; ISO 13320-1) under the following main used parameters: Sample, Dispersion Medium 70 to 100 mg, 10 mL Toluene
   Ultrasound Time 20 seconds
   Dispersant (volume) Toluene (ca. 100 mL)
   Washing Media (volume) 2x Acetone (ca. 50 mL) and Toluene (ca. 50 mL)
   Particle Refractive Index 1,52
   Particle Absorption 0,1
   Dispersant Refractive Index 1,49
   Analysis Model General Purpose - Normal Sensitivity
   Particle Shape Irregular
   Repeats 3 measurements per aliquot
   Delay Between Measurements 10 Seconds
   Sample Measuring Time 15 Seconds
   Background Measuring Time 15 Seconds
   Obscuration Range 15 - 30 %
   Suspension Stirring Rate 2500 ± 10 rpm

The samples were measured employing the following procedure:
Procedure: The sample vial was subjected to a standard mixing cycle in a Bio Grant PTR-30 rotatory mixer. Approximately 70-250 mg of the sample were transferred into a 20 mL vial and 10 mL of Toluene were added. The suspension was sonicated for 20 seconds using a Bandelin Sonopuls HD3100 with a sonication probe (MS72 tip at 20 % intensity). Under continuous circulation (2500 rpm), the sample suspension was added to the measuring medium (ca. 100 mL of Toluene) until the obscuration value reached 15 - 30 %. The sample vial was rinsed with ca. 5 mL of Toluene. Measurements were performed immediately after sample addition and after 3 minutes to ensure dispersion stability. Each result is the average of three consecutive measurements of 15000 swipes. The above-mentioned procedure was carried out by triplicate.

### 1. Capsules of pellets of doxylamine succinate and pyridoxine hydrochloride of the present invention

### 1.1. Quantitative composition per hard capsule

The amount of each one of the ingredients per capsule is as follows:

### 1.1.1 Hard gelatine capsules of Doxylamine succinate 10mq and Pyridoxine hydrochloride 10mg.

Hard gelatine capsules filled with pellets of doxylamine succinate and pyridoxine hydrochloride of the present invention obtained starting from inert nucleus having a particle size such that at least 90% of the inert nuclei have a particle size from 300 µm to 1700 µm measured by analytical sieving and at least the 90% of the pharmaceutically acceptable inert nuclei having a particle size variability of not more than 200 µm measured by analytical sieving.

| **Ingredient** | **Quantity per capsule (mg)** | **Function** |
|---|---|---|
| **Active ingredients** | | |
| Doxylamine succinate (D90 ≤ 250 µm) | 10.0 | Active ingredient |
| Pyridoxine hydrochloride (D90 ≤ 250 µm) | 10.0 | Active ingredient |

| **Excipients** | | |
|---|---|---|
| Sugar spheres of sucrose and starch ⁽¹⁾ | 79.5 | Pharmaceutically acceptable inert nucleus |
| (dewaxed) Shellac | 7.6 | Coating agent/ binder solution |
| Polyvinylpyrrolidone (Povidone K-30) | 0.2 | Coating agent/ binder solution |
| Talc (D90 equal to or below 250µm) | 9.5 | Anticaking agent |
| Methacrylic acid-methyl methacrylate copolymer (1:1) (Eudragit L) | 1.1 | Enteric coating agent |
| Silica colloidal anhydrous (Aerosil) | 0.7 | Anticaking, gliding agent |
| Hard gelatine capsule, size 3 | about 48 ⁽³⁾ | Packing of dose unit |
| Capsule fill weight | each capsule is filled with about 60mg ⁽²⁾ of pellets of doxylamine succinate having the composition as provided in example 1.2.1 and about 60mg(2) of pellets of pyridoxine hydrochloride having the composition provided in example 1.2.2 | |

| | | |
|---|---|---|
| (1) at least 90% of the inert nuclei have a particle size from 300 µm to 1700 µm and at least the 90% of the pharmaceutically acceptable inert nuclei have a particle size variability of not more than 200 µm from a given value comprised from 500 µm and 1400 µm measured by analytical sieving. (2) 60mg +/- 10%, practical target filling weight is calculated based upon actual potency of the pellets in doxylamine succinate or pyridoxine hydrochloride so as to assure the theoretical content of 10.0 mg of doxylamine succinate and 10.0 mg of pyridoxine hydrochloride per capsule (3) 48 mg +/- 10%, based upon capsule supplier specifications | | |

### 1.1.2 Hard HPMC capsules of Doxylamine succinate 10mq and Pyridoxine hydrochloride 10mq

Hard HPMC capsules filled with pellets of doxylamine succinate and pyridoxine hydrochloride of the present invention obtained starting from inert nucleus having a particle size such that at least 90% of the inert nuclei have a particle size from 300 µm to 1700 µm_measured by analytical sieving and at least the 90% of the inert nuclei having a particle size variability of not more than 200 µm measured by analytical sieving.

| **Ingredient** | **Quantity per capsule (mg)** | **Function** |
|---|---|---|
| **Active ingredients** | | |
| Doxylamine succinate (D90 ≤ 250 µm) | 10.0 | Active ingredient |
| Pyridoxine hydrochloride (D90 ≤ 250 µm) | 10.0 | Active ingredient |

| **Excipients** | | |
|---|---|---|
| Sugar spheres of sucrose and starch ⁽¹⁾ | 79.5 | Pharmaceutically acceptable inert nucleus |
| (dewaxed) Shellac | 7.6 | Coating agent/ binder solution |
| Polyvinylpyrrolidone (Povidone K-30) | 0.2 | Coating agent/ binder solution |
| Talc (D90 equal to or below 250µm) | 9.5 | Anticaking agent |
| Methacrylic acid-methyl methacrylate copolymer (1:1) (Eudragit L) | 1.1 | Enteric coating agent |
| Silica colloidal anhydrous (Aerosil) | 0.7 | Anticaking, gliding agent |
| Hard HPMe⁽⁴⁾ capsule, size 3 | about 47 ⁽³⁾ | Packing of dose unit |
| Capsule fill weight | each capsule is filled with about 60mg ⁽²⁾ of pellets of doxylamine succinate having the composition as provided in example 1.2.1 and about 60mg(2) of pellets of pyridoxine hydrochloride having the composition as provided in example 1.2.2 | |

| | | |
|---|---|---|
| (1) at least 90% of the inert nuclei have a particle size from 300 µm to 1700 µm and at least the 90% of the inert nuclei have a particle size variability of not more than 200 µm from a given value comprised from 500 µm and 1400 µm measured by analytical sieving. (2) 60mg +/- 10%, practical target filling weight is calculated based upon actual potency of the pellets in doxylamine succinate or pyridoxine hydrochloride so as to assure the theoretical content of 10.0 mg of doxylamine succinate and 10.0 mg of pyridoxine hydrochloride per capsule (3) 47 mg +/- 10%, based upon capsule supplier specifications (4) HPMC (hydroxypropyl methylcellulose or hypromellose) | | |

### 1.1.3 Hard gelatine capsules of Doxylamine succinate 10mq and Pyridoxine hydrochloride 10mq

Hard gelatine capsules filled with pellets of doxylamine succinate and pyridoxine hydrochloride of the present invention obtained starting from inert nucleus having a particle size such that at least 90% of the inert nuclei have a particle size from 600 µm to 1180 µm measured by analytical sieving and at least the 90% of the inert nuclei having a particle size variability of not more than 100 µm measured by analytical sieving.

| **Ingredient** | **Quantity per capsule (mg)** | **Function** |
|---|---|---|
| **Active ingredients** | | |
| Doxylamine succinate (D90 ≤ 250 µm) | 10.0 | Active ingredient |
| Pyridoxine hydrochloride (D90 ≤ 250 µm) | 10.0 | Active ingredient |

| **Excipients** | | |
|---|---|---|
| Sugar spheres of sucrose and starch ⁽¹⁾ | 79.5 | Pharmaceutically acceptable inert nucleus |
| (dewaxed) Shellac | 7.6 | Coating agent/ binder solution |
| Polyvinylpyrrolidone (Povidone K-30) | 0.2 | Coating agent/ binder solution |
| Talc (D90 equal to or below 250µm) | 9.5 | Anticaking agent |
| Methacrylic acid-methyl methacrylate copolymer (1:1) (Eudragit L) | 1.1 | Enteric coating agent |
| Silica colloidal anhydrous (Aerosil) | 0.7 | Anticaking, gliding agent |
| Hard gelatine capsule, size 3 | about 48 ⁽³⁾ | Packing of dose unit |
| Capsule fill weight | each capsule is filled with about 60mg ⁽²⁾ of pellets of doxylamine succinate having the composition as provided in example 1.2.1 and about 60mg(2) of pellets of pyridoxine hydrochloride having the composition as provided in example 1.2.2 | |

| | | |
|---|---|---|
| (1) at least 90% of the inert nuclei have a particle size from 600 µm to 1180 µm and at least the 90% of the inert nuclei have a particle size variability of not more than 100 µm from a given value comprised from 710 µm and 1000 µm measured by analytical sieving. (2) 60mg +/- 10%, practical target filling weight is calculated based upon actual potency of the pellets in doxylamine succinate or pyridoxine hydrochloride so as to assure the theoretical content of 10.0 mg of doxylamine succinate and 10.0 mg of pyridoxine hydrochloride per capsule (3) 48 mg +/- 10%, based upon capsule supplier specifications | | |

### 1.1.4 Hard HPMC capsules of Doxylamine succinate 10mq and Pyridoxine hydrochloride 10mq

Hard HPMC capsules filled with pellets of doxylamine succinate and pyridoxine hydrochloride of the present invention obtained starting from inert nucleus having a particle size such that at least 90% of the inert nuclei have a particle size from 600 µm to 1180 µm measured by analytical sieving and at least the 90% of the inert nuclei having a particle size variability of not more than 100 µm measured by analytical sieving.

| **Ingredient** | **Quantity per capsule (mg)** | **Function** |
|---|---|---|
| **Active ingredients** | | |
| Doxylamine succinate (D90 ≤ 250 µm) | 10.0 | Active ingredient |
| Pyridoxine hydrochloride (D90 ≤ 250 µm) | 10.0 | Active ingredient |

| **Excipients** | | |
|---|---|---|
| Sugar spheres of sucrose and starch ⁽¹⁾ | 79.5 | Pharmaceutically acceptable inert nucleus |
| (dewaxed) Shellac | 7.6 | Coating agent/ binder solution |
| Polyvinylpyrrolidone (Povidone K-30) | 0.2 | Coating agent/ binder solution |
| Talc (D90 equal to or below 250µm) | 9.5 | Anticaking agent |
| Methacrylic acid-methyl methacrylate copolymer (1:1) (Eudragit L) | 1.1 | Enteric coating agent |
| Silica colloidal anhydrous (Aerosil) | 0.7 | Anticaking, gliding agent |
| Hard HPMe⁽⁴⁾ capsule, size 3 | about 47 ⁽³⁾ | Packing of dose unit |
| Capsule fill weight | each capsule is filled with about 60mg ⁽²⁾ of pellets of doxylamine succinate having the composition as provided in example 1.2.1 and about 60mg(2) of pellets of pyridoxine hydrochloride having the composition provided in example 1.2.2 | |

| | | |
|---|---|---|
| (1) at least 90% of the inert nuclei have a particle size from 600 µm to 1180 µm and at least the 90% of the inert nuclei have a particle size variability of not more than 100 µm from a given value comprised from 710 µm and 1000 µm measured by analytical sieving. (2) 60mg +/- 10%, practical target filling weight is calculated based upon actual potency of the pellets in doxylamine succinate or pyridoxine hydrochloride so as to assure the theoretical content of 10.0 mg of doxylamine succinate and 10.0 mg of pyridoxine hydrochloride per capsule (3) 47 mg +/- 10%, based upon capsule supplier specifications (4) HPMC (hydroxypropyl methylcellulose or hypromellose) | | |

### 1.1.5 Hard gelatine capsules of Doxylamine succinate 10mq and Pyridoxine hydrochloride 10mq

Hard gelatine capsules filled with pellets of doxylamine succinate and pyridoxine hydrochloride of the present invention obtained starting from inert nucleus having a particle size such that at least 90% of the inert nuclei have a particle size from 710 µm to 1000 µm measured by analytical sieving and at least the 90% of the inert nuclei having a particle size variability of not more than 75 µm measured by analytical sieving.

| **Ingredient** | **Quantity per capsule (mg)** | **Function** |
|---|---|---|
| **Active ingredients** | | |
| Doxylamine succinate (D90 ≤ 250 µm) | 10.0 | Active ingredient |
| Pyridoxine hydrochloride (D90 ≤ 250 µm) | 10.0 | Active ingredient |

| **Excipients** | | |
|---|---|---|
| Sugar spheres of sucrose and starch ⁽¹⁾ | 79.5 | Pharmaceutically acceptable inert nucleus |
| (dewaxed) Shellac | 7.6 | Coating agent/ binder solution |
| Polyvinylpyrrolidone (Povidone K-30) | 0.2 | Coating agent/ binder solution |
| Talc (D90 equal to or below 250µm) | 9.5 | Anticaking agent |
| Methacrylic acid-methyl methacrylate copolymer (1:1) (Eudragit L) | 1.1 | Enteric coating agent |
| Silica colloidal anhydrous (Aerosil) | 0.7 | Anticaking, gliding agent |
| Hard gelatine capsule, size 3 | about 48 ⁽³⁾ | Packing of dose unit |
| Capsule fill weight | | each capsules is filled with about 60mg ⁽²⁾ of pellets of doxylamine succinate having the composition as provided in example 1.2.1 and about 60 mg(2) of pellets of pyridoxine hydrochloride having the composition as provided in example 1.2.2 |

| | | |
|---|---|---|
| (1) at least 90% of the inert nuclei have a particle size from 710 µm to 1000 µm and at least the 90% of the pharmaceutically acceptable inert nuclei have a particle size variability of not more than 75 µm from a given value comprised from 800 µm and 900 µm measured by analytical sieving. (2) 60mg +/- 10%, practical target filling weight is calculated based upon actual potency of the pellets in doxylamine succinate or pyridoxine hydrochloride so as to assure the theoretical content of 10.0 mg of doxylamine succinate and 10.0 mg of pyridoxine hydrochloride per capsule (3) 48 mg +/- 10%, based upon capsule supplier specifications. | | |

### 1.1.6 Hard HPMC capsules of Doxylamine succinate 10mq and Pyridoxine hydrochloride 10mq

Hard HPMC capsules filled with pellets of doxylamine succinate and pyridoxine hydrochloride of the present invention obtained starting from inert nucleus having a particle size such that at least 90% of the inert nuclei have a particle size from 710 µm to 1000 µm measured by analytical sieving and at least the 90% of the inert nuclei having a particle size variability of not more than 75 µm measured by analytical sieving.

| **Ingredient** | **Quantity per capsule (mg)** | **Function** |
|---|---|---|
| **Active ingredients** | | |
| Doxylamine succinate (D90 ≤ 250 µm) | 10.0 | Active ingredient |
| Pyridoxine hydrochloride (D90 ≤ 250 µm) | 10.0 | Active ingredient |

| **Excipients** | | |
|---|---|---|
| Sugar spheres of sucrose and starch ⁽¹⁾ | 79.5 | Pharmaceutically acceptable inert nucleus |
| (dewaxed) Shellac | 7.6 | Coating agent/ binder solution |
| Polyvinylpyrrolidone (Povidone K-30) | 0.2 | Coating agent/ binder solution |
| Talc (D90 equal to or below 250µm) | 9.5 | Anticaking agent |
| Methacrylic acid-methyl methacrylate copolymer (1:1) (Eudragit L) | 1.1 | Enteric coating agent |
| Silica colloidal anhydrous (Aerosil) | 0.7 | Anticaking, gliding agent |
| Hard HPMe⁽⁴⁾ capsule, size 3 | about 47 ⁽³⁾ | Packing of dose unit |
| Capsule fill weight | each capsule is filled with about 60mg ⁽²⁾ of pellets of doxylamine succinate having the composition as provided in example 1.2.1 and about 60 mg(2) of pellets of pyridoxine | |
| | | hydrochloride having the composition provided in example 1.2.2 |

| | | |
|---|---|---|
| (1) at least 90% of the inert nuclei have a particle size from 710 µm to 1000 µm and at least the 90% of the inert nuclei have a particle size variability of not more than 75 µm from a given value comprised from 800 µm and 900 µm measured by analytical sieving (2) 60mg +/- 10%, practical target filling weight is calculated based upon actual potency of the pellets in doxylamine succinate or pyridoxine hydrochloride so as to assure the theoretical content of 10.0 mg of doxylamine succinate and 10.0 mg of pyridoxine hydrochloride per capsule (3) 47 mg +/- 10%, based upon capsule supplier specifications (4) HPMC (hydroxypropyl methylcellulose or hypromellose) | | |

### 1.1.7 Hard gelatine capsules of Doxylamine succinate 20mq and Pyridoxine hydrochloride 20mq

Hard gelatine capsules filled with pellets of doxylamine succinate and pyridoxine hydrochloride of the present invention obtained starting from inert nucleus having a particle size such that at least 90% of the inert nuclei have a particle size from 600 µm to 1180 µm measured by analytical sieving and at least the 90% of the inert nuclei having a particle size variability of not more than 100 µm measured by analytical sieving.

| **Ingredient** | **Quantity per capsule (mg)** | **Function** |
|---|---|---|
| **Active ingredients** | | |
| Doxylamine succinate (D90 ≤ 250 µm) | 20.0 | Active ingredient |
| Pyridoxine hydrochloride (D90 ≤ 250 µm) | 20.0 | Active ingredient |

| **Excipients** | | |
|---|---|---|
| Sugar spheres of sucrose and starch ⁽¹⁾ | 159.0 | Pharmaceutically acceptable inert nucleus |
| (dewaxed) Shellac | 15.2 | Coating agent/ binder solution |
| Polyvinylpyrrolidone (Povidone K-30) | 0.4 | Coating agent/ binder solution |
| Talc (D90 equal to or below 250µm) | 19.0 | Anticaking agent |
| Methacrylic acid-methyl methacrylate copolymer (1:1) (Eudragit L) | 2.2 | Enteric coating agent |
| Silica colloidal anhydrous (Aerosil) | 1.4 | Anticaking, gliding agent |
| Hard gelatine capsule, size 2 | about 61 ⁽³⁾ | Packing of dose unit |
| Capsule fill weight | each capsule is filled with about 120mg ⁽²⁾ of pellets of doxylamine succinate having the composition as provided in example 1.2.1 and about 120 mg(2) of pellets of pyridoxine hydrochloride having the composition provided in example 1.2.2 | |

| | | |
|---|---|---|
| (1) at least 90% of the inert nuclei have a particle size from 600 µm to 1180 µm and at least the 90% of the inert nuclei have a particle size variability of not more than 100 µm from a given value comprised from 710 µm and 1000 µm measured by analytical sieving. (2) 120mg +/- 10%, practical target filling weight is calculated based upon actual potency of the pellets in doxylamine succinate or pyridoxine hydrochloride so as to assure the theoretical content of 20.0 mg of doxylamine succinate and 20.0 mg of pyridoxine hydrochloride per capsule (3) 61 mg +/- 10%, based upon capsule supplier specifications | | |

### 1.1.8 Hard HPMC capsules of Doxylamine succinate 20mq and Pyridoxine hydrochloride 20mg

Hard HPMC capsules filled with pellets of doxylamine succinate and pyridoxine hydrochloride of the present invention obtained starting from inert nucleus having a particle size such that at least 90% of the inert nuclei have a particle size from 600 µm to 1180 µm measured by analytical sieving and at least the 90% of the inert nuclei having a particle size variability of not more than 100 µm measured by analytical sieving.

| **Ingredient** | **Quantity per capsule (mg)** | **Function** |
|---|---|---|
| **Active ingredients** | | |
| Doxylamine succinate (D90 ≤ 250 µm) | 20.0 | Active ingredient |
| Pyridoxine hydrochloride (D90 ≤ 250 µm) | 20.0 | Active ingredient |

| **Excipients** | | |
|---|---|---|
| Sugar spheres of sucrose and starch ⁽¹⁾ | 159.0 | Pharmaceutically acceptable inert nucleus |
| (dewaxed) Shellac | 15.2 | Coating agent/ binder solution |
| Polyvinylpyrrolidone (Povidone K-30) | 0.4 | Coating agent/ binder solution |
| Talc (D90 equal to or below 250µm) | 19.0 | Anticaking agent |
| Methacrylic acid-methyl methacrylate copolymer (1:1) (Eudragit L) | 2.2 | Enteric coating agent |
| Silica colloidal anhydrous (Aerosil) | 1.4 | Anticaking, gliding agent |
| Hard HPMe⁽⁴⁾ capsule, size 2 | about 61 ⁽³⁾ | Packing of dose unit |
| Capsule fill weight | each capsule is filled with about 120mg ⁽²⁾ of pellets of doxylamine succinate having the composition as provided in example 1.2.1 and about 120 mg⁽²⁾ of pellets of pyridoxine hydrochloride having the composition provided in example 1.2.2 | |

| | | |
|---|---|---|
| (1) at least 90% of the inert nuclei have a particle size from 600 µm to 1180 µm and at least the 90% of the inert nuclei have a particle size variability of not more than 100 µm from a given value comprised from 710 µm and 1000 µm measured by analytical sieving. (2) 120mg +/- 10%, practical target filling weight is calculated based upon actual potency of the pellets in doxylamine succinate or pyridoxine hydrochloride so as to assure the theoretical content of 20.0 mg of doxylamine succinate and 20.0 mg of pyridoxine hydrochloride per capsule (3) 61 mg +/- 10%, based upon capsule supplier specifications (4) HPMC (hydroxypropyl methylcellulose or hypromellose) | | |

### 1.1.9 Hard gelatine capsules of Doxylamine succinate 20mq and Pyridoxine hydrochloride 20mq

Hard gelatine capsules filled with pellets of doxylamine succinate and pyridoxine hydrochloride of the present invention obtained starting from inert nucleus having a particle size such that at least 90% of the inert nuclei have a particle size from 600 µm to 1180 µm measured by analytical sieving and at least the 90% of the inert nuclei having a particle size variability of not more than 100 µm measured by analytical sieving.

| **Ingredient** | **Quantity per capsule (mg)** | **Function** |
|---|---|---|
| **Active ingredients** | | |
| Doxylamine succinate (D90 ≤ 250 µm) | 20.0 | Active ingredient |
| Pyridoxine hydrochloride (D90 ≤ 250 µm) | 20.0 | Active ingredient |

| **Excipients** | | |
|---|---|---|
| Sugar spheres of sucrose and starch(') | 159.0 | Pharmaceutically acceptable inert nucleus |
| (dewaxed) Shellac | 15.2 | Coating agent/ binder solution |
| Polyvinylpyrrolidone (Povidone K-30) | 0.4 | Coating agent/ binder solution |
| Talc (D90 equal to or below 250µm) | 19.0 | Anticaking agent |
| Methacrylic acid-methyl methacrylate copolymer (1:1) (Eudragit L) | 2.2 | Enteric coating agent |
| Silica colloidal anhydrous (Aerosil) | 1.4 | Anticaking, gliding agent |
| Hard gelatine capsule, size 1 | about 76 ⁽³⁾ | Packing of dose unit |
| Capsule fill weight | each capsule is filled with about 120mg ⁽²⁾ of pellets of doxylamine succinate having the composition as provided in example 1.2.1 and about 120mg(2) of pellets of pyridoxine hydrochloride having the composition as provided in example 1.2.2 | |

| | | |
|---|---|---|
| (1) at least 90% of the inert nuclei have a particle size from 600 µm to 1180 µm and at least the 90% of the inert nuclei having a particle size variability of not more than 100 µm from a given value comprised from 710 µm and 1000 µm measured by analytical sieving. (2) 120mg +/- 10%, practical target filling weight is calculated based upon actual potency of the pellets in doxylamine succinate or pyridoxine hydrochloride so as to assure the theoretical content of 20.0 mg of doxylamine succinate and 20.0 mg of pyridoxine hydrochloride per capsule (3) 76 mg +/- 10%, based upon capsule supplier specifications | | |

### 1.1.10 Hard HPMC capsules of Doxylamine succinate 20mq and Pyridoxine hydrochloride 20mg

Hard HPMC capsules filled with pellets of doxylamine succinate and pyridoxine hydrochloride of the present invention obtained starting from inert nucleus having a particle size such that at least 90% of the inert nuclei have a particle size from 600 µm to 1180 µm measured by analytical sieving and at least the 90% of the inert nuclei having a particle size variability of not more than 100 µm measured by analytical sieving.

| **Ingredient** | **Quantity per capsule (mg)** | **Function** |
|---|---|---|
| **Active ingredients** | | |
| Doxylamine succinate (D90 ≤ 250 µm) | 20.0 | Active ingredient |
| Pyridoxine hydrochloride (D90 ≤ 250 µm) | 20.0 | Active ingredient |

| **Excipients** | | |
|---|---|---|
| Sugar spheres of sucrose and starch ⁽¹⁾ | 159.0 | Pharmaceutically acceptable inert nucleus |
| (dewaxed) Shellac | 15.2 | Coating agent/ binder solution |
| Polyvinylpyrrolidone (Povidone K-30) | 0.4 | Coating agent/ binder solution |
| Talc (D90 equal to or below 250µm) | 19.0 | Anticaking agent |
| Methacrylic acid-methyl methacrylate copolymer (1:1) (Eudragit L) | 2.2 | Enteric coating agent |
| Silica colloidal anhydrous (Aerosil) | 1.4 | Anticaking, gliding agent |
| Hard HPMe⁽⁴⁾ capsule, size 1 | about 76 ⁽³⁾ | Packing of dose unit |
| Capsule fill weight | each capsule is filled with about 120mg ⁽²⁾ of pellets of doxylamine succinate having the composition as provided in example 1.2.1 and about 120 mg(2) of pellets of pyridoxine hydrochloride having the composition provided in example 1.2.2 | |

| | | |
|---|---|---|
| (1) at least 90% of the inert nuclei have a particle size from 600 µm to 1180 µm and at least the 90% of the inert nuclei having a particle size variability of not more than 100 µm from a given value comprised from 710 µm and 1000 µm measured by analytical sieving. (2) 120mg +/- 10%, practical target filling weight is calculated based upon actual potency of the pellets in doxylamine succinate or pyridoxine hydrochloride so as to assure the theoretical content of 20.0 mg of doxylamine succinate and 20.0 mg of pyridoxine hydrochloride per capsule (3) 76 mg +/- 10%, based upon capsule supplier specifications (4) HPMC (hydroxypropyl methylcellulose or hypromellose) | | |

Dissolution Profile: The capsules of pellets of doxylamine succinate and pyridoxine hydrochloride of the present invention of examples 1.1.1 to 1.1.10 exhibit a dissolution profile according to the target dissolution profile measured using a USP type 2 apparatus (basket), placing the composition in 900mL of the corresponding media / buffered 37°C ±0.5 °C and 100 rpm (revolution per minute) as it is shown below.

### 1.2. Quantitative composition per plurality of pellets

1.2.1 Composition of the pellets of doxylamine succinate of the present invention obtained starting from inert nucleus having a particle size such that at least 90% of the inert nuclei have a particle size from 600 µm to 1180 µm and at least the 90% of the inert nuclei having a particle size variability of not more than 100 µm from a given value comprised from 710 µm and 1000 µm and with doxylamine succinate active ingredient having a particle size characterized for having a D90 equal to or below than 250 µm and with talc anticaking agent having a particle size characterized for having a D90 equal to or below than 250 µm.

The amount of each one of the ingredients per a batch of about 25 Kg or about 180 Kg of the pellets of doxylamine succinate is as follows:

| **Layer of the pellet** | **Ingredients** | **function** | **Amount (kg) per a batch of about 25Kg** | **Amount (kg) per a batch of about 180 Kg** |
|---|---|---|---|---|
| Core | Sugar spheres of sucrose and starch | Pharmaceutically acceptable inert nucleus | 15.625 | 112.564 |
| Inner active coating layer | Doxylamine succinate | Active ingredient | 4.192 | 30.200 |
| | Talc | Anticaking agent | 1.397 | 10.067 |
| | Silica colloidal anhydrous (Aerosil 200 pharma) | Anti-caking agent, gliding agent | 0.279 | 2.013 |
| | Polyvinyl pyrrolidone 20% solution in ethanol (Povidone K-30) | Coating agent solution/ binder solution | 0.4188 | 3.020 |
| | (dewaxed) Shellac 40% solution in ethanol | Coating agent solution/ binder solution | 1.169 | 8.419 |
| Intermediate enteric release layer | Talc | Pore-forming agent | 0.0890 | 0.641 |
| | Methacrylic acid and methyl methacrylate copolymer (1:1) (Eudragit L) 10% in acetone (*) | Enteric release coating agent mixture | 0.890 | 6.406 |
| External modified release layer | Methacrylic acid and methyl methacrylate copolymer (1:1) (Eudragit L) 10% in acetone (*) | Enteric release coating agent mixture | 2.950 | 21.155 |
| | (dewaxed) Shellac 40% solution in ethanol | Modified release coating agent solution | 2.937 | 21.156 |
| | Talc | Pore-forming agent | 1.308 | 9.425 |
| **Total dry weight** | | | **25.000** | **180.14** |

| | | | | |
|---|---|---|---|---|
| (*) if necessary purified water or ethanol can be added as processing aid to facilitate the obtainment of the enteric release coating agent solution. | | | | |

The obtained pellets of doxylamine succinate have a particle size such that at least 90% of pellets have a particle size from 710 µm to 1400 µm measured by analytical sieving and at least the 90% of the pellets having a particle size variability of not more than 100 µm from a given value comprised from 850 µm and 1250 µm measured by analytical sieving. The homogeneity of the particle size of pellets and the inter-pellet homogeneity of the content of the active ingredient is such that the pellets are easier to handle allowing a high uniformity in dosification, assuring the desired content of active ingredient and the desired dissolution profile as described in examples above.

1.2.2 Composition of the pellets of pyridoxine hydrochloride of the present invention obtained starting from inert nucleus having a particle size such that at least 90% of the inert nuclei have a particle size from 600 µm to 1180 µm and at least the 90% of the pharmaceutically acceptable inert nuclei having a particle size variability of not more than 100 µm from a given value comprised from 710 µm and 1000 µm and with pyridoxine hydrochloride active ingredient having a particle size characterized for having a D90 equal to or below than 250 µm and with talc as anticaking agent having a particle size characterized for having a D90 equal to or below than 250 µm.

The amount of each one of the ingredients per a batch of about 25 Kg or about 180 Kg of the pellets of pyridoxine hydrochloride is as follows:

| **Layer of the pellets** | **Ingredients** | **function** | **Amount (Kg) per a batch of about 25 Kg** | **Amount (Kg) per a batch of about 180 (Kg)** |
|---|---|---|---|---|
| Core | Sugar spheres of sucrose and starch | Pharmaceutically acceptable inert | 17.962 | 129.285 |
| | | nucleus | | |
| Inner active coating layer | pyridoxine hydrochloride | Active ingredient | 4.243 | 30.600 |
| | (dewaxed) Shellac 40% solution in ethanol | Coating agent solution/ binder solution | 2.122 | 15.300 |
| External modified release layer | Methacrylic acid and methyl methacrylate copolymer (1:1) (Eudragit L) 10% in acetone (*) | Enteric release coating agent mixture | 0.679 | 4.896 |
| | (dewaxed) Shellac 40% solution in ethanol | Modified release coating agent solution | 1.758 | 12.677 |
| | Talc | Anticaking agent | 1.212 | 8.743 |
| **Total dry weight:** | | | **25.017** | **180.31** |

| | | | | |
|---|---|---|---|---|
| (*) if necessary purified water or ethanol can be added as processing aid to facilitate the obtainment of the enteric release coating agent solution. | | | | |

The obtained pellets of pyridoxine hydrochloride have a particle size such that at least 90% of the pellets have a particle size from 710 µm to 1400 µm measured by analytical sieving and at least the 90% of the pellets having a particle size variability of not more than 100 µm from a given value comprised from 850 µm and 1250 µm measured by analytical sieving. The homogeneity of the particle size of pellets and the inter-pellet homogeneity of the content of the active ingredient is such that the pellets are easier to handle allowing a high uniformity in dosification, assuring the desired content of active ingredient and the desired dissolution profile as described in examples above.

### 1.3. Preparation process by continuously spraying a liquid slurry/suspension

### 1.3.1. Preparation of a 25 kg scale batch of a plurality of doxylamine succinate pellets

### A. Preparation of phases

Phase 1-Liquid coating slurry/suspension mixture for the preparation of the inner active coating layer: A mixture of 84 g of povidone K30, 467 g of (dewaxed) shellac and 4.28 kg of ethanol was prepared. Then, over this mixture, 4.192 kg of doxylamine succinate (having a D90 <250 µm), 0.279 kg aerosol 200 Ph (having a D90 <250 µm) and 1.397 kg of talc (with a D90 <250 µm) were added. The resulting 10.7 kg of mixture were stirred continuously.

Phase 2-Liquid coating slurry/suspension mixture for the preparation of the intermediate coating layer: A mixture of 89 g of Eudradil L 100 and 1064 g of acetone and 133 g of purified water and 45 g of talc (with a D90 <250 µm) were prepared. The resulting 1.331 kg of mixture were stirred continuously.

Phase 3-Liquid coating slurry/suspension mixture for the preparation of the external coating layer: 735 g of talc (with a D90 <250 µm) were added to a mixture of 294 g of Eudradil L100, 1175 g of shellac, 10.71 kg of acetone, 7.93 kg of ethanol and 1.19 kg of purified water. The resulting 22.04 kg of mixture were stirred continuously.

### B. Preparation process

### -inner active coating layer

15.625 kg of inert nuclei (sugar spheres of sucrose and starch) having a particle size such that at least 90% of the inert nuclei have a particle size from 600 µm to 1180 µm and at least the 90% of the pharmaceutically acceptable inert nuclei having a particle size variability of not more than 100 µm from a given value comprised from 710 µm and 1000 µm were transferred into a coating pan. Then liquid coating slurry/suspension mixture phase 1 was sprayed continuously over the pellets, at a coating pan rotation rate of 15 rpm, a spray flow rate of 65 g/min, a depression inside the pan-coating <100 Pa, a gun pressure of about 1.0 bar and an open pattern pressure of about 1.2 bar. While the phase 1 was sprayed, simultaneously the pellets were continuously dried by using hot air (airflow about 100 m³/h at 30-35 °C) and keeping the core temperature of the pellets between 22°C and 26°C. The coated pellets thus obtained were dried during 10 minutes at 35-50 °C by keeping them rotate in the coating pan at a rate comprised from 0 and 10 rpm and with an airflow of about 160 m³/h to obtain the dried coated pellets with the inner active coating layer having the active ingredient. If necessary, the dried pellets can be sieved and unwanted particle size, powders and aggregates can be discarded.

### -intermediate coating layer

On the coated active pellets obtained in previous step, at a coating pan rotation rate of 15 rpm, the phase 2 liquid mixture was continuously sprayed at a spray flow rate of 20 g/min, a depression inside the pan-coating <100 Pa, a gun pressure of about 1.0 bar, and an open pattern pressure of about 1.2 bar. While the phase 2 was sprayed, simultaneously the pellets were continuously dried by using hot air (airflow <100 m³/h at 30-35 °C) and keeping the core temperature of the pellets between 24°C and 28°C. The obtained coated pellets were dried during 30 min at 35-50 °C by keeping them rotate in the coating pan at a rate comprised from 0 and 10 rpm and with an airflow of about 160 m³/h to obtain the dried coated pellets with the inner active coating layer and the intermediate coating layer.

### -external modified release coating layer

Then, the phase 3 coating mixture was continuously sprayed over the bi-layered pellets obtained in the previous step, at a coating pan rotation rate of 15 rpm, a spray flow rate of 75 g/min, a depression inside the pan-coating <100 Pa, a gun pressure of about 1.0 bar, and an open pattern pressure of about 1.2 bar. While the phase 3 was sprayed, simultaneously, the pellets were continuously dried by using hot air (airflow >140 m³/h at 45-50 °C) and keeping the core temperature of the pellets between 25°C and 29°C. The obtained coated pellets were dried during 30 min at about 50 °C by keeping them rotate in the coating pan at a rate comprised from 0 and 10 rpm and with an airflow of about 160 m³/h to obtain the dried coated pellets with the inner active coating layer, the intermediate coating layer, and the external coating layer.

The pellet thus obtained has the target dissolution profile. If necessary, the dried pellets can be sieved and unwanted particle size, powders and aggregates were discarded.

The modified release pellets of doxylamine succinate thus obtained were stored in 25 kg closed double food pharmaceutical grade polyethylene bags inside closed High Density Polyethylene drums.

### 1.3.2. Preparation of a 25 kq scale batch of a plurality of pyridoxine hydrochloride pellets

### A. Preparation of phases

Phase 4-Liquid coating slurry/suspension mixture for the preparation of the inner active coating layer: A mixture of 0.850 kg of (dewaxed) shellac and 3.48 kg of ethanol was prepared. Then, over this mixture, 4.24 kg of pyridoxine hydrochloride (with a D90 <250 µm) were added. The resulting 8.43 kg of mixture were stirred continuously.

Phase 5-Liquid coating slurry/suspension mixture for the preparation of the external coating layer: 385 g of talc (with a D90 <250 µm) were added to a mixture of 68 g of Eudradil L100, 702 g of shellac, 610 g of acetone, 9.72 kg of ethanol and 68 g of purified water. The resulting 11.56 kg of mixture were stirred continuously.

### B. Preparation process

### -inner active coating layer

17.962 kg of inert nuclei (sugar spheres of sucrose and starch) having a particle size such that at least 90% of the inert nuclei have a particle size from 600 µm to 1180 µm and at least the 90% of the pharmaceutically acceptable inert nuclei having a particle size variability of not more than100 µm from a given value comprised from 710 µm and 1000 µm were transferred into a coating pan. Then liquid coating slurry/suspension mixture phase 4 was sprayed continuously over the pellets, at a coating pan rotation rate of 15 rpm, a spray flow rate of 60 g/min, a depression inside the pan-coating <100 Pa, a gun pressure of about 1.0 bar and an open pattern pressure of about 1.2 bar. While the phase 4 was sprayed, simultaneously the pellets were continuously dried by using hot air (airflow about 100 m³/h at 38-42 °C) and keeping the core temperature of the pellets between 21°C and 24°C. The obtained coated pellets were dried during 20 min with hot air (airflow of about 160 m³/h at 40-50 °C) by keeping them rotate in the coating pan at a rotation rate comprised from 0 and 10 rpm, to obtain the dried coated pellets with the inner active coating layer having the active ingredient. If necessary, the dried pellets can be sieved and unwanted particle size, powders and aggregates were discarded.

### -external modified release coating layer

Then, the phase 5 coating mixture was continuously sprayed over coated active pellets obtained in previous step, at a coating pan rotation rate of 15 rpm, a spray flow rate of 63 g/min, a depression inside the pan-coating <100 Pa, a gun pressure of about 1.0 bar, and an open pattern pressure of about 1.2 bar. While the phase 5 was sprayed, simultaneously, the pellets were continuously dried by using hot air (airflow >130 m³/h at 50-65 °C) and keeping the core temperature of the pellets between 26°C and 30°C. The obtained coated pellets were dried during 30 min with hot air (airflow >120 m³/h at 45-55 °C) by keeping them rotate in the coating pan at a rotation rate comprised from 0 and 10 rpm, to obtain the dried coated pellets with the inner active coating layer and the external coating layer, in a yield ≥93%.

The pellet thus obtained has the target dissolution profile. If necessary, the dried pellets can be sieved and unwanted particle size, powders and aggregates were discarded.

The modified release pellets of doxylamine succinate thus obtained were stored in 25 kg closed double food pharmaceutical grade polyethylene bags inside closed High Density Polyethylene drums.

### 1.4. Preparation process by discontinuously spraying solution and alternately adding solid in powder form

### 1.4.1. Preparation of a 25 kg scale batch of a plurality of doxylamine succinate pellets

### A. Preparation of phases

Phase 1-powdered mixture for the preparation of the inner active coating layer: In a coating pan were mixed 4.192 kg of doxylamine succinate (D90 < 250 µm), 0.279 kg of Aerosil 200 pharma (with a D90 < 250 µm) and 1.397 kg of talc (with a D90 < 250 µm).

Phase 2-binding solution for the preparation of the inner active coating layer: 0.419 kg of povidone K30 20% w/w in ethanol and 1.169 kg of (dewaxed) shellac 40 % w/w in ethanol were mixed.

Phase 3-coating solution for the preparation of the external coating: 89 g of Eudragit L 100 were added to 711 g of acetone and the mixture was stirred, then, 89 g of water were added obtaining a clear solution.

Phase 4-coating solution for the preparation of the external coating: 2.950 g of Eudragit L 10% w/w in acetone, 300 g of purified water and 2.937 kg of (dewaxed) shellac 40% w/w in ethanol were mixed.

### B. Preparation process

### -inner active coating layer

The abovementioned amount of inert nuclei (sugar spheres of sucrose and starch) having a particle size such that at least 90% of the inert nuclei have a particle size from 600 µm to 1180 µm and at least the 90% of the pharmaceutically acceptable inert nuclei having a particle size variability of not more than 100 µm from a given value comprised from 710 µm and 1000 µm were transferred into a coating pan. Then the rotation started and was maintained a coating pan rotation rate of 20 rpm and a depression inside the pan-coating <100 Pa during all this coating step while the binding solution phase 2 was discontinuously sprayed repeating cycles with the next sequence:
- 24 s spraying the binding solution Phase-2 at a spray flow rate of 78 g/min at a gun pressure of about 0.8 bar and an open pattern pressure of about 1.0 bar.
- 5 s of pause 1
- 66-s of addition of the powdered mixture Phase 1 at a powder addition rate of 107 g/min
- 30 s of pause
- 0 s of drying time (no drying).

The core temperature of the pellets was kept between 21°C and 25°C. The coated pellets thus obtained were dried during 2 h at room temperature by keeping them rotate in the coating pan at a rate comprised from 0 and 5 rpm and with an airflow >50 m³/h to obtain the dried coated pellets with the inner active coating layer having the active ingredient. If necessary, the dried pellets can be sieved and unwanted particle size, powders and aggregates were discarded.

### -intermediate enteric release coating layer

The coated active pellets obtained in previous step were transferred into a coating pan. Then the rotation started and was maintained a coating pan rotation rate of 20 rpm and a depression inside the pan-coating <100 Pa during this coating step while the coating solution phase 3 was discontinuously sprayed repeating cycles with the next sequence:
- 60 s spraying the coating solution Phase-3 at a spray flow rate of 46 g/min and a gun pressure of about 1.0 bar and an open pattern pressure of about 1.0 bar.
- 15-s of addition of talc (with a D90 <250 µm) at a powder addition rate of 16.2 g/min
- 15 s of pause
- 45 s of drying time (airflow 50 m3/h at 40-45 °C)

The core temperature of the pellets was kept between 19°C and 24°C. The coated pellets thus obtained were dried during 1 h at room temperature by keeping them rotate in the coating pan at a rate comprised from 0 and 5 rpm and with an airflow >50 m³/h to obtain the dried coated pellets with the inner active coating layer having the active ingredient. If necessary, the dried pellets can be sieved and unwanted particle size, powders and aggregates were discarded.

### -external modified release coating layer

The coated active pellets obtained in previous step were transferred into a coating pan. Then the rotation started and was maintained a coating pan rotation rate of 20 rpm and a depression inside the pan-coating <100 Pa during this coating step while the coating solution phase 4 was discontinuously sprayed repeating cycles with the next sequence:
- 20 s spraying the binding solution Phase-4 at a spraying flow rate of 39 g/min at a gun pressure of about 1.4 bar and an open pattern pressure of about 1.6 bar.
- 10-s of addition of talc (with a D90 <250 µm) at a powder addition rate of 17 g/min
- 20 s of pause
- 0 s of drying time (no drying).

The core temperature of the pellets was kept between 24°C and 29°C. The coated pellets thus obtained were dried during 12 h at 40-45 °C by keeping them rotate in the coating pan at a rate comprised from 0 and 5 rpm and with an airflow >80 m³/h to obtain the dried coated pellets with the inner active coating layer having the active ingredient, in a yield equal to higher than 93% calculated by dividing the obtained amount into the theoretical amount x 100.

The pellet thus obtained has the target dissolution profile. If necessary, the dried pellets can be sieved and unwanted particle size, powders and aggregates were discarded.

The modified release pellets of doxylamine succinate thus obtained were stored in 25 kg closed double food pharmaceutical grade polyethylene bags inside closed High Density Polyethylene drums.

### 1.4.2. Preparation of a 25 kg scale batch of a plurality of pyridoxine hydrochloride pellets

### A. Preparation of phases

Phase 5 -coating solution for the preparation of the external coating: 0.679 kg of Eudragit L 10% w/w in acetone, 68 g of water and 1.758 kg of (dewaxed) shellac 40 % w/w in ethanol were mixed.

### B. Preparation process

### -inner active coating layer

The above-mentioned amount of inert nuclei (sugar spheres of sucrose and starch) having a particle size such that at least 90% of the inert nuclei have a particle size from 600 µm to 1180 µm and at least the 90% of the pharmaceutically acceptable inert nuclei having a particle size variability of not more than 100 µm from a given value comprised from 710 µm and 1000 µm were transferred into a coating pan. Then the rotation started and was maintained a coating pan rotation rate of 20 rpm and a depression inside the pan-coating <100 Pa during all this coating step while (dewaxed) shellac 40% w/w in ethanol was discontinuously sprayed repeating cycles with the next sequence:
- 18 s spraying the binding solution Phase at 56 g/min at a gun pressure of about 0.8 bar and an open pattern pressure of about 1.0 bar.
- 5 s of pause 1
- 9-s of addition of pyridoxine hydrochloride (with a D90 <250 µm) at a powder addition rate of 233 g/min
- 30 s of pause 2
- 0 s of drying time (no drying).

The core temperature of the pellets was kept between 19°C and 24°C. The coated pellets thus obtained were dried during 2 h at room temperature by keeping them rotate in the coating pan at a rate comprised from 0 and 5 rpm and with an airflow >50 m³/h to obtain the dried coated pellets with the inner active coating layer having the active ingredient. If necessary, the dried pellets can be sieved and unwanted particle size, powders and aggregates were discarded.

### -external modified release coating layer

The coated active pellets obtained in previous step were transferred into a coating pan. Then the rotation started and was maintained a coating pan rotation rate of 20 rpm and a depression inside the pan-coating <100 Pa during this coating step while the coating solution phase 4 was discontinuously sprayed repeating cycles with the next sequence:
- 18 s of spraying the binding solution Phase-4 at a spraying flow rate of 40 g/min at a gun pressure of about 1.4 bar and an open pattern pressure of about 1.6 bar.
- 9-s of addition talc (with a D90 <250 µm) at a powder addition rate of 40 g/min
- 20 s of pause
- 0 s of drying time (no drying).

The core temperature of the pellets was kept between 23°C and 28°C. The coated pellets thus obtained were dried during 12 h at 40-45 °C by keeping them rotate in the coating pan at a rate comprised from 0 and 5 rpm and with an airflow >50 m³/h to obtain the dried coated pellets with the inner active coating layer having the active ingredient, in a yield ≥93%.

The pellet thus obtained has the target dissolution profile. If necessary, the dried pellets can be sieved and unwanted particle size, powders and aggregates were discarded.

The modified release pellets of doxylamine succinate thus obtained were stored in 25 kg closed double food pharmaceutical grade polyethylene bags inside closed High Density Polyethylene drums.

### -external modified release coating layer

On the coated active pellets obtained in previous step, phase 4 was sprayed at flow rate of 100 g/min. While solution phase 4 was sprayed, talc was applied in solid form at a solid addition rate of 50 g/min and a rotation rate of 16 rpm, keeping the core temperature of the pellets between 17°C and 22°C and the airflow lower than 100 m³/h. The obtained coated pellets were dried during not less than 8 h and up to 12 h at 40-45 °C by keeping them rotate in the coating pan at a rate comprised from 0 and 10 rpm and with an airflow >130 m³/h (yield equal to higher than 93%).

The pellet thus obtained has the target dissolution profile. If necessary, the dried pellets can be sieved and unwanted particle size, powders and aggregates were discarded.

The modified release pellets of pyridoxine hydrochloride thus obtained were stored in 50 kg closed double food pharmaceutical grade polyethylene bags inside closed High Density Polyethylene drums.

With the procedures mentioned above for the preparation of the first and the second plurality of the modified release pellets of the present invention the obtained pellets of doxylamine succinate have a particle size such that at least 90% of the pellets have a particle size from 710 µm to 1400 µm measured by analytical sieving and at least the 90% of the pellets having a particle size variability of not more than 100 µm from a given value comprised from 850 µm and 1250 µm measured by analytical sieving and the obtained pellets of pyridoxine hydrochloride have a particle size such that at least 90% of the pellets have a particle size from 710 µm to 1400 µm measured by analytical sieving and at least the 90% of the pellets having a particle size variability of not more than 100 µm from a given value comprised from 850 µm and 1250 µm measured by analytical sieving.

The same procedures mentioned above for the preparation of the first and the second plurality of the modified release pellets of the present invention can be executed using inert nuclei (sugar spheres of sucrose and starch) but having a particle size such that at least 90% of the inert nuclei have a particle size from 710 µm to 1000 µm measured by analytical sieving and at least the 90% of the pharmaceutically acceptable inert nuclei have a particle size variability of not more than 75 µm for a given value comprised from 800 µm and 900 µm measured by analytical sieving. In this case the obtained pellets of doxylamine succinate have a particle size such that at least 90% of pellets have a particle size from 800 µm to 1400 µm measured by analytical sieving and at least the 90% of the pellets have a particle size variability of not more than 75 µm from a given value comprised from 900 µm and 1180 µm measured by analytical sieving; and the obtained pellets of pyridoxine hydrochloride have a particle size such that at least 90% of pellets have a particle size from 800 µm to 1400 µm measured by analytical sieving and at least the 90% of the pellets have a particle size variability of not more than 75 µm from a given value comprised from 900 µm and 1180 µm measured by analytical sieving.

### Capsule filling

Each Hard capsule was filled with about 60 mg of the modified release pellets of doxylamine succinate and about 60 mg of the modified release pellets of pyridoxine hydrochloride of the present invention as defined above using a Bosch Zanassi E48 automatic capsule-filling machine; to obtain capsules containing about 10 mg of doxylamine succinate and about 10 mg of pyridoxine hydrochloride.

The same procedures mentioned above for the preparation of capsules having about 10 mg of doxylamine succinate and about 10 mg of pyridoxine hydrochloride, can be performed by filling the capsules with about 120 mg of the modified release pellets of doxylamine succinate and about 120 mg of the modified release pellets of pyridoxine hydrochloride of the present invention as defined above using the Bosch Zanassi E48 automatic capsule-filling machine; to obtain capsules containing about 20 mg of doxylamine succinate and about 20 mg of pyridoxine hydrochloride.

### 2. Dissolution Test

### Dissolution profile

The target dissolution profile requires that both the doxylamine succinate and the pyridoxine hydrochloride were slightly dissolved under the stomach conditions and that the major therapeutic concentration was achieved in the intestinal tract due to its rapid dissolution rate. In particular, the process for the preparation of the present invention allows obtaining capsules, filled with such a modified release pellets of doxylamine succinate and modified release pellets of pyridoxine hydrochloride, which exhibit a dissolution profile according to which:
from 5% to 35% by weight of doxylamine content is dissolved at 1^{st}h in 0.1 N HCl medium (pH = 1);
then, the medium is replaced by a pH = 4.5 medium (0.05 M acetate buffer) and at 4^{th} h from an accumulated more than 35% to 75% by weight of doxylamine initial content is dissolved;
then, the medium is replaced by a pH = 6.8 medium (0.05 M phosphate buffer) and at 7^{th} h at least an accumulated more than 75% of doxylamine initial content is dissolved
from 5% to 35% by weight of pyridoxine content is dissolved at 1^{st}h in 0.1 N HCl medium (pH = 1);
then, the medium is replaced by a pH = 4.5 medium (0.05 M acetate buffer) and at 4^{th} h from an accumulated more than 35% to 75% by weight of pyridoxine initial content is dissolved;
then, the medium is replaced by a pH = 6.8 medium (0.05 M phosphate buffer) and at 7^{th} h at least an accumulated more than 75% of pyridoxine initial content is dissolved.
wherein the dissolution profile is measured using a USP type II apparatus (basket), placing the composition in 900mL of the corresponding media / buffered 37°C ±0.5 °C and 100 rpm.

### Conditions of the dissolution bath

- Paddle speed: 100 rpm
- Temperature of dissolution medium: 37 °C ± 0.5 °C
- Dissolution media: hydrochloric acid 0.1N
- Vessel volume: 900 mL
- Time: 1 hour
- Dissolution media: pH 4.5; 0.05 M acetate buffer
- Vessel volume: 900 mL
- Time: From the 1^{st} h to the 4^{th} hour
- Dissolution media: pH 6.8; 0.05 M phosphate buffer
- Vessel volume: 900 mL
- Time: From the 4^{th} h to the 7^{th} hour

### Conditions of the chromatographic analysis

- Sample preparation: Take an aliquot of approximately 10 ml and filter it through 0.70 µm membrane filter, then filter it through another 0.22 µm membrane filter.
- Flux: 1 mL/min
- Column: Kromasil 100-5 C18, 150 × 4.0 mm
- Phases: methanol in water
- Injection volume: 100 µL
- Excitation wavelength: 220 nm
- Chromatographic time: 25 min.
- Aqueous phase: Ammonium acetate buffer 0.06 M pH 5.0 + 0.1% sodium hexane sulfonate (PICB6)):
- Gradient:

| Time (min) | Methanol (%) | Aqueous phase (%) |
|---|---|---|
| 0 | 20 | 80 |
| 4 | 32.5 | 80 |
| 8 | 100 | 50 |
| 13 | 100 | 50 |
| 17 | 20 | 80 |
| 25 | 20 | 80 |

### Results

The capsules of the present invention as defined above and below which comprises pellets of doxylamine succinate and pyridoxine hydrochloride exhibit the target dissolution profile. Thus, the dissolution of both doxylamine succinate and pyridoxine hydrochloride when it is submitted to stomach conditions is, at least a 5% of the total amount in 1 hour and at least a 35% of doxylamine succinate and pyridoxine hydrochloride after 4 hours is dissolved when it is submitted to duodenal conditions (pH = 4.5) and at least an 75% of doxylamine succinate and pyridoxine hydrochloride after 7 hours is dissolved when it is submitted to colon conditions.

### Packaging and Stability Test

### Blisters:

- The capsules of pellets of doxylamine succinate and pyridoxine hydrochloride of the present invention of examples 1.1.1 to 1.1.10 when primary packaged in blister made of PVC/PVdC (on one side of the blister) and aluminium (on the other side of the blister) are stable allowing storage at or below 25°C and 60% Relative Humidity.
- The capsules of pellets of doxylamine succinate and pyridoxine hydrochloride of the present invention of examples 1.1.2; 1.1.4; 1.1.6; 1.1.8 and 1.1.10 when primary packaged in blister made in AquaBa^{®} or Aluminium (one side of the blister) and Aluminium (the other side of the blister) are stable allowing storage at or below 30°C and 75% Relative Humidity.

### Bottles:

- The capsules of pellets of doxylamine succinate and pyridoxine hydrochloride of the present invention of examples 1.1.1 to 1.1.10 when primary packaged in plastic bottles plus desiccant are stable allowing storage at or below 30°C and 75% Relative Humidity.

### Citation List

1. WO2013123569
2. WO2016029290
3. European Pharmacopoeia chapter 2.9.38
4. European Pharmacopoeia Doxylamine hydrogen succinate monograph
5. European Pharmacopoeia Pyridoxine hydrochloride monograph

## Claims

1. A modified release multiple unit oral dosage form comprising:
a first plurality of modified release pellets of doxylamine or a pharmaceutically acceptable salt thereof comprising:
- a pharmaceutically acceptable inert nucleus;
- an inner active coating layer comprising a therapeutically effective amount of doxylamine or a pharmaceutically acceptable salt thereof, one or more coating agents, one or more anticaking agents, optionally one or more pore-forming agent; and optionally one or more pharmaceutically acceptable excipients;
- optionally an intermediate enteric release coating layer comprising one or more enteric coating agents, one or more anticaking agents, optionally one or more pore-forming agent; and optionally one or more pharmaceutically acceptable excipients, and
- an external modified release coating layer comprising one or more enteric coating agents, one or more modified release coating agents, one or more anticaking agents, optionally one or more pore-forming agents, and optionally one or more pharmaceutically acceptable excipients; and
a second plurality of modified release pellets of pyridoxine or a pharmaceutically acceptable salt thereof comprising:
- a pharmaceutically acceptable inert nucleus;
- an inner active coating layer comprising a therapeutically effective amount of pyridoxine or a pharmaceutically acceptable salt thereof, one or more coating agents, and optionally one or more pharmaceutically acceptable excipients; and
- an external modified release coating layer comprising one or more enteric coating agents, one or more modified release coating agents, optionally one or more pore-forming agents, and optionally one or more pharmaceutically acceptable excipients;
wherein:
the particle size of the pharmaceutically acceptable inert nucleus of the first and the second plurality of pellets is such that at least 90% of the inert nuclei have a particle size from 300 µm to 1700 µm measured by analytical sieving and at least the 90% of inert nuclei have a particle size variability of not more than 200 µm from a given value comprised from 500 µm and 1400 µm measured by analytical sieving;
wherein the particle size variability means that from a given value, at least the 90% of the pharmaceutically acceptable inert nuclei of the first and the second plurality of pellets have a particle size comprised from ± 200 µm from the given value.

2. The modified release multiple unit oral dosage form according to claim 1, wherein the first plurality of modified release pellets of doxylamine or a pharmaceutically acceptable salt thereof comprises the intermediate enteric release coating layer comprising one or more enteric coating agents, one or more anticaking agents, optionally one or more pore-forming agent; and optionally one or more pharmaceutically acceptable excipients.

3. The modified release multiple unit oral dosage form according to any of the claims 1 or 2, wherein:
the dosage form comprises pharmaceutically acceptable inert nucleus of the first and the second plurality of pellets having a particle size such that at least 90% of the inert nuclei have a particle size from 300 µm to 1400 µm measured by analytical sieving, and at least the 90% of the pharmaceutically acceptable inert nuclei have a particle size variability of not more than 150 µm from a given value comprised from 450 µm and 1250 µm measured by analytical sieving; wherein the particle size variability means that from a given value, at least the 90% of the pharmaceutically acceptable inert nuclei of the first and the second plurality of pellets have a particle size comprised from ± 150 µm from the given value;
particularly, the dosage form comprises pharmaceutically acceptable inert nucleus of the first and the second plurality of pellets having a particle size such that at least 90% of the inert nuclei have a particle size from 600 µm to 1180 µm measured by analytical sieving and at least the 90% of the pharmaceutically acceptable inert nuclei have a particle size variability of not more than 100 µm from a given value comprised from 710 µm and 1000 µm measured by analytical sieving; wherein the particle size variability means that from a given value, at least the 90% of the pharmaceutically acceptable inert nuclei of the first and the second plurality of pellets have a particle size comprised from ± 100 µm from the given value; and
particularly, the dosage form comprises pharmaceutically acceptable inert nucleus of the first and the second plurality of pellets having a particle size such that at least 90% of the inert nuclei have a particle size from 710 µm to 1000 µm measured by analytical sieving and at least the 90% of the pharmaceutically acceptable inert nuclei have a particle size variability of not more than 75 µm from a given value comprised from 800 µm and 900 µm measured by analytical sieving; wherein the particle size variability means that from a given value, at least the 90% of the pharmaceutically acceptable inert nuclei of the first and the second plurality of pellets have a particle size comprised from ± 75 µm from the given value.

4. The modified release multiple unit oral dosage form according to any of the claims 1-3, wherein:
the particle size of the pellets of the first plurality of modified release pellets of doxylamine or a pharmaceutically acceptable salt thereof is such that at least 90% of the pellets have a particle size from 400 µm to 2000 µm measured by analytical sieving and at least the 90% of the pellets have a particle size variability of not more than 200 µm from a given value comprised from 600 µm and 1800 µm measured by analytical sieving; wherein the particle size variability means that from a given value, the at least 90% of the first plurality of modified release pellets have a particle size comprised from ± 200 µm from the given value;
particularly, the particle size of the pellets of the first plurality of modified release pellets of doxylamine or a pharmaceutically acceptable salt thereof is such that at least 90% of the pellets have a particle size from 600 µm to 1600 µm measured by analytical sieving and at least the 90% of the pellets have a particle size variability of not more than 150 µm from a given value comprised from 800 µm and 1400 µm measured by analytical sieving; wherein the particle size variability means that from a given value, the at least 90% of the first plurality of modified release pellets have a particle size comprised from ± 150 µm from the given value;
particularly, the particle size of the pellets of the first plurality of modified release pellets of doxylamine or a pharmaceutically acceptable salt thereof is such that at least 90% of the pellets have a particle size from 710 µm to 1400 µm measured by analytical sieving and at least the 90% of the pellets have a particle size variability of not more than 100 µm from a given value comprised from 850 µm and 1250 µm measured by analytical sieving; wherein the particle size variability means that from a given value, the at least 90% of the first plurality of modified release pellets have a particle size comprised from ± 100 µm from the given value; and
particularly, the particle size of the pellets of the first plurality of modified release pellets of doxylamine or a pharmaceutically acceptable salt thereof is such that at least 90% of the pellets have a particle size from 800 µm to 1400 µm measured by analytical sieving and at least the 90% of the pellets have a particle size variability of not more than 75 µm from a given value comprised from 900 µm and 1180 µm measured by analytical sieving; wherein the particle size variability means that from a given value, the at least 90% of the first plurality of modified release pellets have a particle size comprised from ± 75 µm from the given value; and
the particle size of the pellets of the second plurality of modified release pellets of pyridoxine or a pharmaceutically acceptable salt thereof is such that at least 90% of the pellets have a particle size from 400 µm to 2000 µm measured by analytical sieving and at least the 90% of the pellets have a particle size variability of not more than 200 µm from a given value comprised from 600 µm and 1800 µm measured by analytical sieving; wherein the particle size variability means that from a given value, the at least 90% of the second plurality of modified release pellets have a particle size comprised from ± 200 µm from the given value;
particularly, the particle size of the pellets of the second plurality of modified release pellets of pyridoxine or a pharmaceutically acceptable salt thereof is such that at least 90% of the pellets have a particle size from 600 µm to 1600 µm measured by analytical sieving and at least the 90% of the pellets have a particle size variability of not more than 150 µm from a given value comprised from 800 µm and 1400 µm measured by analytical sieving; wherein the particle size variability means that from a given value, the at least 90% of the second plurality of modified release pellets have a particle size comprised from ± 150 µm from the given value;
particularly, the particle size of the pellets of the second plurality of modified release pellets of pyridoxine or a pharmaceutically acceptable salt thereof is such that at least 90% of the pellets have a particle size from 710 µm to 1400 µm measured by analytical sieving and at least the 90% of the pellets have a particle size variability of not more than 100 µm from a given value comprised from 850 µm and 1250 µm measured by analytical sieving; wherein the particle size variability means that from a given value, the at least 90% of the second plurality of modified release pellets have a particle size comprised from ± 100 µm from the given value; and
particularly, the particle size of the pellets of the second plurality of modified release pellets of pyridoxine or a pharmaceutically acceptable salt thereof is such that at least 90% of the pellets have a particle size from 800 µm to 1400 µm measured by analytical sieving and at least the 90% of the pellets have a particle size variability of not more than 75 µm from a given value comprised from 900 µm and 1180 µm measured by analytical sieving; wherein the particle size variability means that from a given value, the at least 90% of the second plurality of modified release pellets have a particle size comprised from ± 75 µm from the given value.

5. The modified release multiple unit oral dosage form according to any of the claims 1-4, wherein:
the particle size of doxylamine or a pharmaceutically acceptable salt thereof is characterized for having a D90 equal to or below than 250 µm; and the particle size of pyridoxine or a pharmaceutically acceptable salt thereof is characterized for having a D90 equal to or below than 250 µm;
or alternatively,
the particle size of doxylamine or a pharmaceutically acceptable salt is characterized for having a D90 equal to or below than 250 µm; and the particle size of pyridoxine or a pharmaceutically acceptable salt thereof is characterized for having a D90 equal to or below than 250 µm; the particle size of the one or more anticaking agent is characterized for having a D90 equal to or below than 250 µm
wherein:
the D90 of the particle size of doxylamine or a pharmaceutically acceptable salt thereof is expressed by volume; the D90 of the particle size of pyridoxine or a pharmaceutically acceptable salt thereof is expressed by volume; and the D90 of the particle size of the anticaking agents is expressed by weight.

6. The modified release multiple unit oral dosage form according to any of the claims 1-5, which comprises a pharmaceutically acceptable salt of doxylamine and a pharmaceutically acceptable salt of pyridoxine; preferably, comprises doxylamine succinate and pyridoxine hydrochloride.

7. The modified release multiple unit oral dosage form according to any of the claims 1-6, which comprises:
from 5 mg to 50 mg per oral dosage form of doxylamine or a pharmaceutically acceptable salt thereof; and
from 5 mg to 50 mg per oral dosage form of pyridoxine or a pharmaceutically acceptable salt thereof;
particularly,
from 10 mg to 20 mg per oral dosage form of doxylamine or a pharmaceutically acceptable salt thereof; and
from 10 mg to 20 mg per oral dosage form of pyridoxine or a pharmaceutically acceptable salt thereof.

8. The modified release multiple unit oral dosage form according to any of the claims 1-7,
wherein the modified release multiple unit oral dosage form exhibits a dissolution profile according to which:
from 5% to 35% by weight of doxylamine content is dissolved at 1^{st}h in 0.1 N HCl medium (pH = 1);
then, the medium is replaced by a pH = 4.5 medium (0.05 M acetate buffer) and at 4^{th} h from an accumulated more than 35% to 75% by weight of doxylamine initial content is dissolved;
then, the medium is replaced by a pH = 6.8 medium (0.05 M phosphate buffer) and at 7^{th} h at least an accumulated more than 75% by weight of doxylamine initial content is dissolved; and
from 5% to 35% by weight of pyridoxine content is dissolved at 1^{st}h in 0.1 N HCl medium (pH = 1);
then, the medium is replaced by a pH = 4.5 medium (0.05 M acetate buffer) and at 4^{th} h from an accumulated more than 35% to 75% by weight of pyridoxine initial content is dissolved;
then, the medium is replaced by a pH = 6.8 medium (0.05 M phosphate buffer) and at 7^{th} h at least an accumulated more than 75% by weight of pyridoxine initial content is dissolved;
wherein the dissolution profile is measured using a USP type 2 apparatus (basket), placing the composition in 900mL of the corresponding media / buffered 37°C ± 0.5 °C and 100 rpm.

9. The modified release multiple unit oral dosage form according to any of the claims 1-8, which is a capsule; particularly a hard capsule; more particularly hard capsule is selected from the group consisting of hard gelatine capsule and a hard hydroxypropyl methylcellulose capsule.

10. A process for the preparation of the modified release multiple unit oral dosage form as defined in any of the claims 1-9 comprising:
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by adding the one or more enteric coating agents, the one or more modified release coating agents, the one or more anticaking agents,
optionally one or more pore-forming agent, and optionally one or more pharmaceutically acceptable excipients, wherein the particle size of the pharmaceutically acceptable inert nucleus is such that at least 90% of the inert nuclei have a particle size from 300 µm to 1700 µm measured by analytical sieving and at least the 90% of inert nuclei have a particle size variability of not more than 200 µm measured by analytical sieving; particularly of not more than 150 µm; particularly of not more than 100 µm; and particularly of not more than 75 µm; and
(b1) preparing the second plurality of modified release pellets of pyridoxine or a pharmaceutically acceptable salt thereof by coating the pellets of pyridoxine or a pharmaceutically acceptable salt thereof having the inner active coating layer by adding the one or more enteric coating agents, the one or more modified release coating agents, optionally the one or more pore-forming agents, and optionally one or more pharmaceutically acceptable excipients, wherein the particle size of the pharmaceutically acceptable inert nucleus is such that at least 90% of the inert nuclei have a particle size from 300 µm to 1700 µm measured by analytical sieving and at least the 90% of inert nuclei have a particle size variability of not more than 200 µm measured by analytical sieving; particularly of not more than 150 µm; particularly of not more than 100 µm; and particularly of not more than 75 µm.

11. The process according to claim 10, wherein the process comprises:
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by continuously or discontinuously spraying a liquid mixture comprising the one or more enteric coating agents, the one or more modified release coating agents, and optionally one or more pharmaceutically acceptable excipients; and
(b1) preparing the second plurality of modified release pellets of pyridoxine or a pharmaceutically acceptable salt thereof by coating the pellets of pyridoxine or a pharmaceutically acceptable salt thereof having the inner active coating layer by continuously or discontinuously spraying a liquid mixture comprising the one or more enteric coating agents, the one or more modified release coating agents and optionally one or more pharmaceutically acceptable excipients;
or alternatively,
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by continuously or discontinuously spraying a liquid mixture comprising the one or more enteric coating agents, the one or more modified release coating agents, and optionally one or more pharmaceutically acceptable excipients; and adding simultaneously or alternately a mixture in powder form comprising the one or more anticaking agents,
optionally one or more pore-forming agents, and optionally one or more pharmaceutically acceptable excipients; and
(b1) preparing the second plurality of modified release pellets of pyridoxine or a pharmaceutically acceptable salt thereof by coating the pellets of pyridoxine or a pharmaceutically acceptable salt thereof having the inner active coating layer by continuously or discontinuously spraying a liquid mixture comprising the one or more enteric coating agents, the one or more modified release coating agents, and optionally one or more pharmaceutically acceptable excipients; and adding simultaneously or alternately a mixture in powder form comprising the one or more anticaking agents, optionally one or more pore-forming agents, and optionally one or more pharmaceutically acceptable excipients.

12. The process according to claim 11 comprising:
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by adding the one or more enteric coating agents, the one or more modified release coating agents, the one or more anticaking agents,
optionally one or more pore-forming agent, and optionally one or more pharmaceutically acceptable excipients, wherein: the sum of the enteric coating agents and the modified release coating agents in the spraying liquid mixture is from 10% to 49% by weight in relation to the weight of the liquid mixture, and
(b1) preparing the second plurality of modified release pellets of pyridoxine or a pharmaceutically acceptable salt thereof by coating the pellets of pyridoxine or a pharmaceutically acceptable salt thereof having the inner active coating layer by adding the one or more enteric coating agents, the one or more modified release coating agents, the one or more anticaking agents, optionally the one or more pore-forming agents, and
optionally one or more pharmaceutically acceptable excipients, wherein: the sum of the enteric coating agents and the modified release coating agents in the spraying liquid mixture is from 10% to 49% by weight in relation to the weight of the liquid mixture.

13. The process according to any of the claims 11 or 12, wherein the process comprises:
(a1) preparing the first plurality of modified release pellets of doxylamine or a pharmaceutically acceptable salt thereof by coating the pellets of doxylamine or a pharmaceutically acceptable salt thereof having the inner active coating layer and optionally the intermediate enteric coating layer by continuously or discontinuously spraying a liquid mixture comprising from 1.0 to 7.5 % by weight of the one or more enteric coating agents, from 10.0 to 35.0 % by weight of the one or more modified release coating agents in a weight ratio between them from 5:95 to 30:70, and optionally one or more pharmaceutically acceptable excipients; and
simultaneously or alternately adding a mixture in powder form comprising the one or more anticaking agents, optionally one or more pore-forming agents, and optionally one or more pharmaceutically acceptable excipients,
wherein: the spray average flow rate of the mixture comprising the coating is from 0.30 to 5.00 g/min per kg of pharmaceutically acceptable inert nuclei; the average of the solid addition rate of the mixture in solid form is from 0.05 to 1.50 g/min per Kg of pharmaceutically acceptable inert nuclei; and the relation between the spray average flow rate of the mixture comprising the coating agents and the average of the solid addition rate of the mixture in solid form is from 90:10 to 60:40, particularly from 90:10 to 70:30; and
(b1) preparing the second plurality of modified release pellets of pyridoxine or a pharmaceutically acceptable salt thereof by coating the pellets of pyridoxine or a pharmaceutically acceptable salt thereof having the inner active coating layer by continuously or discontinuously spraying a liquid mixture comprising from 1.0 to 7.5 % by weight of the one or more enteric coating agents, from 10.0 to 35.0 % by weight of the one or more modified release coating agents in a weight ratio from 5:95 to 30:70; and optionally one or more pharmaceutically acceptable excipients; and simultaneously or alternately adding a mixture in powder form comprising one or more anticaking agents, optionally one or more pore-forming agents, and optionally one or more pharmaceutically acceptable excipients,
wherein: the spray average flow rate of the mixture comprising the coating agents is from 0.30 to 5.00 g/min per kg of pharmaceutically acceptable inert nuclei; the average of the solid addition rate of the mixture in solid form is from 0.10 to 2.25 g/min per Kg of pharmaceutically acceptable inert nuclei; and the relation between the spray average flow rate of the mixture comprising the coating agents and the average of the solid addition rate of the mixture in solid form is from 90:10 to 60:40, particularly from 80:20 to 60:40.

14. The process according to any of the claims 10-13, wherein the process further comprises a previous step of coating separately:
optionally (a2) the pellets of doxylamine or a pharmaceutically acceptable salt thereof having the inner active coating layer by continuously or discontinuously spraying a liquid mixture comprising the one or more enteric coating agents, and optionally one or more pharmaceutically acceptable excipients; and
(b2) the pharmaceutically acceptable inert nucleus by continuously or discontinuously spraying a liquid mixture comprising one or more coating agents, the therapeutically effective amount of pyridoxine or a pharmaceutically acceptable salt thereof, and optionally one or more pharmaceutically acceptable excipients;
or alternatively,
optionally (a2) the pellets of doxylamine or a pharmaceutically acceptable salt thereof having the inner active coating layer by continuously or discontinuously spraying a liquid mixture comprising the one or more enteric coating agents, and optionally one or more pharmaceutically acceptable excipients; and simultaneously or alternately adding a mixture in solid form comprising the one or more anticaking agents, optionally the one or more pore-forming agents, and optionally one or more pharmaceutically acceptable excipients; and
(b2) the pharmaceutically acceptable inert nucleus by continuously or discontinuously spraying a liquid mixture comprising one or more coating agents, and optionally one or more pharmaceutically acceptable excipients; and simultaneously or alternately adding in powder form the therapeutically effective amount of pyridoxine or a pharmaceutically acceptable salt thereof, and optionally one or more pharmaceutically acceptable excipients.

15. The process according to claim 14, wherein the process comprises:
optionally (a2) the pellets of doxylamine or a pharmaceutically acceptable salt thereof having the inner active coating layer by continuously or discontinuously spraying a liquid mixture comprising from 5 to 15% by weight of the one or more enteric coating agents, and optionally one or more pharmaceutically acceptable excipients;
and simultaneously or alternately adding a mixture in powder form from 5.0 to 6.5 g per kg of pharmaceutically acceptable inert nuclei of the mixture in solid form comprising the one or more anticaking agents, optionally the one or more pore-forming agents, and optionally one or more pharmaceutically acceptable excipients;
wherein: the average of the spray flow rate of the mixture comprising the enteric coating agents is from 0.30 to 3.00 g/min per kg of pharmaceutically acceptable inert nuclei; the average of the solid addition rate of the mixture in solid form is from 0.025 to 0.400 g/min per Kg of pharmaceutically acceptable inert nuclei; and the relation between the average spray flow rate of the mixture comprising the coating agents and the average of solid addition rate of the mixture in solid form is from 85:15 to 95:5; and
(b2) the pharmaceutically acceptable inert nucleus with a simultaneously or alternately spraying a liquid mixture comprising from 20% to 45% by weight of one or more coating agents, and optionally one or more pharmaceutically acceptable excipients; and simultaneously or alternately adding in powder form the therapeutically effective amount of pyridoxine or a pharmaceutically acceptable salt thereof, and optionally one or more pharmaceutically acceptable excipients: wherein the average spray flow rate of the mixture comprising the coating agents is from 0.30 to 4.50 g/min per Kg of pharmaceutically acceptable inert nuclei;
the average of the solid addition rate of the powder is from 0.50 to 9.00 g/min per Kg of pharmaceutically acceptable inert nuclei; and the relation between the average spray flow rate of the mixture comprising the coating agents and the average of the solid addition rate is from 25:75 to 40:60.

16. The process according to any of the claims 14 or 15, wherein the process further comprises an additional step which comprises:
(a3) coating the pharmaceutically acceptable inert nucleus with a continuously or discontinuously spraying a liquid mixture comprising one or more coating agents, the therapeutically effective amount of doxylamine or a pharmaceutically acceptable salt thereof, and optionally one or more pharmaceutically acceptable excipients;
or alternatively,
(a3) coating the pharmaceutically acceptable inert nucleus with a continuously or discontinuously spraying a liquid mixture comprising one or more coating agents, and optionally one or more pharmaceutically acceptable excipients; and simultaneously or alternately adding a mixture in powder form comprising the therapeutically effective amount of doxylamine or a pharmaceutically acceptable salt thereof, one or more anticaking, optionally one or more pore-forming agents, and optionally one or more pharmaceutically acceptable excipients.

17. The process according to claim 16, wherein the process comprises:
(a3) coating the pharmaceutically acceptable inert nucleus with a continuously or discontinuously spraying a liquid mixture comprising from 15% to 40% by weight of one or more coating agents, and optionally one or more pharmaceutically acceptable excipients; and simultaneously or alternately adding a mixture in powder form comprising the therapeutically effective amount of doxylamine or a pharmaceutically acceptable salt thereof, from 18 to 36% by weight of the one or more anticaking, optionally one or more pore-forming agents, and optionally one or more pharmaceutically acceptable excipients;
wherein: the average spray flow rate of the mixture comprising the coating agents is from 0.30 to 4.50 g/min per Kg of pharmaceutically acceptable inert nuclei; the average of the solid addition rate of the mixture in powder form is from 0.95 to 18.00 g/min per Kg of pharmaceutically acceptable inert nuclei; and the relation between the average spray flow rate of the mixture comprising the coating agents and the average of the solid addition rate of the mixture in powder form is from 15:85 to 30:70.

18. The process according to any of the claims 10-17, wherein in each one of the spraying steps (a1), (a2), (a3), (b1) and (b2), the liquid mixture is sprayed at a gun atomization pressure from 0.6 to 2.2 bar and an open pattern pressure from 0.6 to 2.5 bar.

## Patentansprüche

1. Orale Mehrfacheinheitsdosierungsform modifizierter Freisetzung, umfassend:
eine erste Vielzahl von Doxylamin-Pellets modifizierter Freisetzung oder einem pharmazeutisch annehmbaren Salz davon, umfassend:
- einen pharmazeutisch annehmbaren inerten Kern;
- eine innere aktive Überzugsschicht, umfassend eine therapeutisch wirksame Menge an Doxylamin oder einem pharmazeutisch annehmbaren Salz davon, ein oder mehrere Überzugsmittel, ein oder mehrere Antibackmittel, gegebenenfalls einen oder mehrere Porenbildner; und gegebenenfalls einen oder mehrere pharmazeutisch annehmbare Hilfsstoffe;
- gegebenenfalls eine Zwischenschicht magensaftresistenter Freisetzung, umfassend ein oder mehrere magensaftresistente Überzugsmittel, ein oder mehrere Antibackmittel, gegebenenfalls einen oder mehrere Porenbildner und gegebenenfalls einen oder mehrere pharmazeutisch annehmbare Hilfsstoffe, und
- eine äußere Überzugsschicht modifizierter Freisetzung, umfassend ein oder mehrere magensaftresistente Überzugsmittel, ein oder mehrere Überzugsmittel modifizierter Freisetzung, ein oder mehrere Antibackmittel, gegebenenfalls ein oder mehrere Porenbildner und gegebenenfalls einen oder mehrere pharmazeutisch annehmbare Hilfsstoffe; und
eine zweite Vielzahl von Pyridoxin-Pellets modifizierter Freisetzung oder einem pharmazeutisch annehmbaren Salz davon, umfassend:
- einen pharmazeutisch annehmbaren inerten Kern;
- eine innere aktive Überzugsschicht, umfassend eine therapeutisch wirksame Menge an Pyridoxin oder einem pharmazeutisch annehmbaren Salz davon, ein oder mehrere Überzugsmittel und gegebenenfalls einen oder mehrere pharmazeutisch annehmbare Hilfsstoffe; und
- eine äußere Überzugsschicht modifizierter Freisetzung, umfassend ein oder mehrere magensaftresistente Überzugsmittel, ein oder mehrere Überzugsmittel modifizierter Freisetzung, gegebenenfalls einen oder mehrere Porenbildner und gegebenenfalls einen oder mehrere pharmazeutisch annehmbare Hilfsstoffe;
wobei:
die Teilchengröße des pharmazeutisch annehmbaren inerten Keims der ersten und der zweiten Vielzahl von Pellets derart ist, dass mindestens 90 % der inerten Keime eine Teilchengröße von 300 µm bis 1700 µm, gemessen durch analytisches Sieben, aufweisen und mindestens die 90 % der inerten Keime eine Teilchengrößenvariabilität von nicht mehr als 200 µm von einem gegebenen Wert aus 500 µm und 1400 um, gemessen durch analytisches Sieben, aufweisen;
wobei die Teilchengrößenvariabilität bedeutet, dass von einem gegebenen Wert mindestens die 90 % der pharmazeutisch annehmbaren inerten Kerne der ersten und der zweiten Vielzahl von Pellets eine Teilchengröße aufweisen, die von 1200 µm von dem gegebenen Wert umfasst ist.

2. Orale Mehrfacheinheit-Darreichungsform modifizierter Freisetzung nach Anspruch 1, wobei die erste Vielzahl von Doxylamin-Pellets modifizierter Freisetzung oder einem pharmazeutisch annehmbaren Salz davon die Zwischenschicht magensaftresistenter Freisetzung umfasst, umfassend ein oder mehrere magensaftresistente Überzugsmittel, ein oder mehrere Antibackmittel, gegebenenfalls einen oder mehrere Porenbildner; und gegebenenfalls einen oder mehrere pharmazeutisch annehmbare Hilfsstoffe.

3. Orale Mehrfacheinheitsdosierungsform modifizierter Freisetzung nach einem der Ansprüche 1 oder 2, wobei:
die Darreichungsform einen pharmazeutisch annehmbaren inerten Keim der ersten und der zweiten Vielzahl von Pellets umfasst, die eine Teilchengröße aufweisen, sodass mindestens 90 % der inerten Keime eine Teilchengröße von 300 µm bis 1400 µm, gemessen durch analytisches Sieben, aufweisen und mindestens die 90 % der pharmazeutisch annehmbaren inerten Keime eine Teilchengrößenvariabilität von nicht mehr als 150 µm von einem gegebenen Wert aus 450 µm und 1250 µm, gemessen durch analytisches Sieben, aufweisen; wobei die Teilchengrößenvariabilität bedeutet, dass von einem gegebenen Wert mindestens 90 % der pharmazeutisch annehmbaren inerten Keime der ersten und der zweiten Vielzahl von Pellets eine Teilchengröße von ±150 µm von dem gegebenen Wert aufweisen;
die Darreichungsform insbesondere einen pharmazeutisch annehmbaren inerten Keim der ersten und der zweiten Vielzahl von Pellets umfasst, die eine Teilchengröße aufweisen, sodass mindestens 90 % der inerten Keime eine Teilchengröße von 600 µm bis 1180 µm, gemessen durch analytisches Sieben, aufweisen und mindestens die 90 % der pharmazeutisch annehmbaren inerten Keime eine Teilchengrößenvariabilität von nicht mehr als 100 µm von einem gegebenen Wert aus 710 µm und 1000 µm, gemessen durch analytisches Sieben, aufweisen; wobei die Teilchengrößenvariabilität bedeutet, dass von einem gegebenen Wert mindestens 90 % der pharmazeutisch annehmbaren inerten Keime der ersten und der zweiten Vielzahl von Pellets eine Teilchengröße von ±100 µm von dem gegebenen Wert aufweisen;
die Darreichungsform insbesondere einen pharmazeutisch annehmbaren inerten Keim der ersten und der zweiten Vielzahl von Pellets umfasst, die eine Teilchengröße aufweisen, sodass mindestens 90 % der inerten Keime eine Teilchengröße von 710 µm bis 1000 µm, gemessen durch analytisches Sieben, aufweisen und mindestens die 90 % der pharmazeutisch annehmbaren inerten Keime eine Teilchengrößenvariabilität von nicht mehr als 75 µm von einem gegebenen Wert aus 800 µm und 900 µm, gemessen durch analytisches Sieben, aufweisen;
wobei die Teilchengrößenvariabilität bedeutet, dass von einem gegebenen Wert mindestens 90 % der pharmazeutisch annehmbaren inerten Keime der ersten und der zweiten Vielzahl von Pellets eine Teilchengröße von ±75 µm von dem gegebenen Wert aufweisen;

4. Orale Mehrfacheinheitsdosierungsform modifizierter Freisetzung nach einem der Ansprüche 1 bis 3, wobei:
die Teilchengröße der Pellets der ersten Vielzahl von Doxylamin-Pellets modifizierter Freisetzung oder eines pharmazeutisch annehmbaren Salzes davon derart ist, dass mindestens 90 % der Pellets eine durch analytisches Sieben gemessene Teilchengröße von 400 µm bis 2000 µm aufweisen und mindestens die 90 % der Pellets eine Teilchengrößenvariabilität von nicht mehr als 200 µm von einem gegebenen Wert aus 600 µm und 1800 µm, gemessen durch analytisches Sieben, aufweisen; wobei die Teilchengrößenvariabilität bedeutet, dass ab einem gegebenen Wert die mindestens 90 % der ersten Vielzahl von Pellets modifizierter Freisetzung eine Teilchengröße von ±200 µm ab dem gegebenen Wert aufweisen; insbesondere die Teilchengröße der Pellets der ersten Vielzahl von Doxylamin-Pellets modifizierter Freisetzung oder einem pharmazeutisch annehmbaren Salz davon derart ist, dass mindestens 90 % der Pellets eine durch analytisches Sieben gemessene Teilchengröße von 600 µm bis 1600 µm aufweisen und mindestens 90 % der Pellets eine Teilchengrößenvariabilität von nicht mehr als 150 µm von einem gegebenen Wert, der von 800 µm bis 1400 µm umfasst, gemessen durch analytisches Sieben; wobei die Teilchengrößenvariabilität bedeutet, dass von einem gegebenen Wert die mindestens 90 % der ersten Vielzahl von Pellets modifizierter Freisetzung eine Teilchengröße von ±150 µm von dem gegebenen Wert aufweisen;
insbesondere die Teilchengröße der Pellets der ersten Vielzahl von Doxylamin-Pellets modifizierter Freisetzung oder einem pharmazeutisch annehmbaren Salz davon derart ist, dass mindestens 90 % der Pellets eine durch analytisches Sieben gemessene Teilchengröße von 710 µm bis 1400 µm aufweisen und mindestens 90 % der Pellets eine Teilchengrößenvariabilität von nicht mehr als 100 µm von einem gegebenen Wert, der von 850 µm bis 1250 µm umfasst, gemessen durch analytisches Sieben; wobei die Teilchengrößenvariabilität bedeutet, dass von einem gegebenen Wert die mindestens 90 % der ersten Vielzahl von Pellets modifizierter Freisetzung eine Teilchengröße von ±100 µm von dem gegebenen Wert aufweisen; und
insbesondere die Teilchengröße der Pellets der ersten Vielzahl von Doxylamin-Pellets modifizierter Freisetzung oder einem pharmazeutisch annehmbaren Salz davon derart ist, dass mindestens 90 % der Pellets eine durch analytisches Sieben gemessene Teilchengröße von 800 µm bis 1400 um aufweisen und mindestens 90 % der Pellets eine Teilchengrößenvariabilität von nicht mehr als 75 µm von einem gegebenen Wert, der von 900 µm bis 1180 µm umfasst, gemessen durch analytisches Sieben; wobei die Teilchengrößenvariabilität bedeutet, dass von einem gegebenen Wert die mindestens 90 % der ersten Vielzahl von Pellets modifizierter Freisetzung eine Teilchengröße von ±75 µm von dem gegebenen Wert aufweisen; und
die Teilchengröße der Pellets der zweiten Vielzahl von Pyridoxin-Pellets modifizierter Freisetzung oder eines pharmazeutisch annehmbaren Salzes davon derart ist, dass mindestens 90 % der Pellets eine durch analytisches Sieben gemessene Teilchengröße von 400 µm bis 2000 µm aufweisen und mindestens die 90 % der Pellets eine Teilchengrößenvariabilität von nicht mehr als 200 µm von einem gegebenen Wert aus 600 µm und 1800 µm, gemessen durch analytisches Sieben, aufweisen; wobei die Teilchengrößenvariabilität bedeutet, dass ab einem gegebenen Wert die mindestens 90 % der zweiten Vielzahl von Pellets modifizierter Freisetzung eine Teilchengröße von ±200 µm ab dem gegebenen Wert aufweisen;
insbesondere die Teilchengröße der Pellets der zweiten Vielzahl von Pyridoxin-Pellets modifizierter Freisetzung oder einem pharmazeutisch annehmbaren Salz davon derart ist, dass mindestens 90 % der Pellets eine durch analytisches Sieben gemessene Teilchengröße von 600 µm bis 1600 µm aufweisen und mindestens 90 % der Pellets eine Teilchengrößenvariabilität von nicht mehr als 150 µm von einem gegebenen Wert, der von 800 µm bis 1400 µm umfasst, gemessen durch analytisches Sieben; wobei die Teilchengrößenvariabilität bedeutet, dass von einem gegebenen Wert die mindestens 90 % der zweiten Vielzahl von Pellets modifizierter Freisetzung eine Teilchengröße von ±150 µm von dem gegebenen Wert aufweisen;
insbesondere die Teilchengröße der Pellets der zweiten Vielzahl von Pyridoxin-Pellets modifizierter Freisetzung oder einem pharmazeutisch annehmbaren Salz davon derart ist, dass mindestens 90 % der Pellets eine durch analytisches Sieben gemessene Teilchengröße von 710 µm bis 1400 µm aufweisen und mindestens 90 % der Pellets eine Teilchengrößenvariabilität von nicht mehr als 100 µm von einem gegebenen Wert, der von 850 µm bis 1250 µm umfasst, gemessen durch analytisches Sieben; wobei die Teilchengrößenvariabilität bedeutet, dass von einem gegebenen Wert die mindestens 90 % der zweiten Vielzahl von Pellets modifizierter Freisetzung eine Teilchengröße von ±100 µm von dem gegebenen Wert aufweisen; und
insbesondere die Teilchengröße der Pellets der zweiten Vielzahl von Pyridoxin-Pellets modifizierter Freisetzung oder einem pharmazeutisch annehmbaren Salz davon derart ist, dass mindestens 90 % der Pellets eine durch analytisches Sieben gemessene Teilchengröße von 800 µm bis 1400 µm aufweisen und mindestens 90 % der Pellets eine Teilchengrößenvariabilität von nicht mehr als 75 µm von einem gegebenen Wert, der von 900 µm bis 1180 µm umfasst, gemessen durch analytisches Sieben; wobei die Teilchengrößenvariabilität bedeutet, dass von einem gegebenen Wert die mindestens 90 % der zweiten Vielzahl von Pellets modifizierter Freisetzung eine Teilchengröße von ±75 µm von dem gegebenen Wert aufweisen.

5. Orale Mehrfacheinheitsdosierungsform modifizierter Freisetzung nach einem der Ansprüche 1 bis 4, wobei:
die Teilchengröße von Doxylamin oder einem pharmazeutisch annehmbaren Salz davon **dadurch gekennzeichnet ist, dass** sie einen D90 von 250 µm oder weniger aufweist; und die Teilchengröße von Pyridoxin oder einem pharmazeutisch annehmbaren Salz davon **dadurch gekennzeichnet ist, dass** sie einen D90 von 250 µm oder weniger aufweist;
oder alternativ
die Teilchengröße von Doxylamin oder einem pharmazeutisch annehmbaren Salz **dadurch gekennzeichnet ist, dass** sie einen D90 von 250 µm oder weniger aufweist; und die Teilchengröße von Pyridoxin oder einem pharmazeutisch annehmbaren Salz davon **dadurch gekennzeichnet ist, dass** sie einen D90 von 250 µm oder weniger aufweist; die Teilchengröße des einen oder der mehreren Antibackmittel **dadurch gekennzeichnet, ist dass** sie einen D90 von 250 µm oder weniger aufweist wobei
der D90 der Teilchengröße von Doxylamin oder einem pharmazeutisch annehmbaren Salz davon durch Volumen ausgedrückt wird; der D90 der Teilchengröße von Pyridoxin oder einem pharmazeutisch annehmbaren Salz davon durch Volumen ausgedrückt wird; und der D90 der Teilchengröße der Antibackmittel durch Gewicht ausgedrückt wird.

6. Orale Mehrfacheinheitsdosierungsform modifizierter Freisetzung nach einem der Ansprüche 1 bis 5, die ein pharmazeutisch annehmbares Salz von Doxylamin und ein pharmazeutisch annehmbares Salz von Pyridoxin umfasst; vorzugsweise Doxylaminsuccinat und Pyridoxinhydrochlorid umfasst.

7. Orale Mehrfacheinheitsdosierungsform modifizierter Freisetzung nach einem der Ansprüche 1 bis 6, die Folgendes umfasst:
von 5 mg bis 50 mg pro oraler Darreichungsform von Doxylamin oder einem pharmazeutisch annehmbaren Salz davon; und
von 5 mg bis 50 mg pro oraler Darreichungsform von Pyridoxin oder einem pharmazeutisch annehmbaren Salz davon;
insbesondere
von 10 mg bis 20 mg pro oraler Darreichungsform von Doxylamin oder einem pharmazeutisch annehmbaren Salz davon; und
von 10 mg bis 20 mg pro oraler Darreichungsform von Pyridoxin oder einem pharmazeutisch annehmbaren Salz davon.

8. Orale Mehrfacheinheit-Darreichungsform modifizierter Freisetzung nach einem der Ansprüche 1 bis 7,
wobei die orale Mehrfacheinheitsdosierungsform modifizierter Freisetzung ein Auflösungsprofil aufweist, gemäß dem:
5 bis 35 Gewichtsprozent Doxylamingehalt in Stunde 1 in 0,1 N HCI-Medium (pH = 1) gelöst werden;
dann wird das Medium durch ein Medium mit pH = 4,5 (0,05 M Acetatpuffer) ersetzt und in Stunde 4 von einem akkumulierten Ausgangsgehalt von mehr als 35 Gewichtsprozent bis 75 Gewichtsprozent Doxylamin gelöst;
dann wird das Medium durch ein Medium mit pH = 6,8 (0,05 M Phosphatpuffer) ersetzt und in Stunde 7 wird mindestens ein akkumulierter Anfangsgehalt von mehr als 75 Gewichtsprozent Doxylamin gelöst; und
5 bis 35 Gewichtsprozent Pyridoxingehalt in Stunde 1 in 0,1 N HCI-Medium (pH = 1) gelöst werden;
dann wird das Medium durch ein Medium mit pH = 4,5 (0,05 M Acetatpuffer) ersetzt und in Stunde 4 von einem akkumulierten Ausgangsgehalt von mehr als 35 Gewichtsprozent bis 75 Gewichtsprozent Pyridoxin gelöst;
dann wird das Medium durch ein Medium mit pH = 6,8 (0,05 M Phosphatpuffer) ersetzt und in Stunde 7 wird mindestens ein akkumulierter Anfangsgehalt von mehr als 75 Gewichtsprozent Pyridoxin gelöst;
wobei das Auflösungsprofil unter Verwendung einer USP-2-Vorrichtung (Korb) gemessen wird, wobei die Zusammensetzung in 900 ml des entsprechenden Mediums / gepufferten 37 °C ±0,5 °C und 100 U/min platziert wird.

9. Orale Mehrfacheinheitsdosierungsform modifizierter Freisetzung nach einem der Ansprüche 1 bis 8, die eine Kapsel ist; insbesondere eine Hartkapsel; insbesondere ist die Hartkapsel ausgewählt aus der Gruppe, bestehend aus Hartgelatinekapsel und einer Harthydroxypropylmethylcellulosekapsel.

10. Verfahren zur Herstellung der oralen Mehrfacheinheitsdosierungsform modifizierter Freisetzung nach einem der Ansprüche 1 bis 9, umfassend:
(a1) Herstellen der ersten Vielzahl von Doxylamin-Pellets modifizierter Freisetzung oder eines pharmazeutisch annehmbaren Salzes davon durch Beschichten der Doxylamin-Pellets oder eines pharmazeutisch annehmbaren Salzes davon mit der inneren aktiven Überzugsschicht und gegebenenfalls der magensaftresistenten Überzugszwischenschicht durch Hinzufügen des einen oder der mehreren magensaftresistenten Überzugsmittel, des einen oder der mehreren Überzugsmittel modifizierter Freisetzung, des einen oder der mehreren Antibackmittel, gegebenenfalls eines oder mehrerer Porenbildner und gegebenenfalls eines oder mehrerer pharmazeutisch annehmbarer Hilfsstoffe, wobei die Teilchengröße des pharmazeutisch annehmbaren inerten Keims derart ist, dass mindestens 90 % der inerten Keime eine Teilchengröße von 300 µm bis 1700 um, gemessen durch analytisches Sieben, aufweisen und mindestens die 90 % der inerten Keime eine Teilchengrößenvariabilität von nicht mehr als 200 um, gemessen durch analytisches Sieben, aufweisen; insbesondere von nicht mehr als 150 um; insbesondere von nicht mehr als 100 um; und insbesondere von nicht mehr als 75 um; und
(b1) Herstellen der zweiten Vielzahl von Pyridoxin-Pellets modifizierter Freisetzung oder eines pharmazeutisch annehmbaren Salzes davon durch Beschichten der Pyridoxin-Pellets oder eines pharmazeutisch annehmbaren Salzes davon mit der inneren aktiven Überzugsschicht durch Hinzufügen des einen oder der mehreren magensaftresistenten Überzugsmittel, des einen oder der mehreren Überzugsmittel modifizierter Freisetzung, gegebenenfalls des einen oder der mehreren Porenbildner und gegebenenfalls eines oder mehrerer pharmazeutisch annehmbarer Hilfsstoffe, wobei die Teilchengröße des pharmazeutisch annehmbaren inerten Kerns derart ist, dass mindestens 90 % der inerten Keime eine Teilchengröße von 300 µm bis 1700 um, gemessen durch analytisches Sieben, aufweisen und mindestens 90 % der inerten Keime eine Teilchengrößenvariabilität von nicht mehr als 200 um, gemessen durch analytisches Sieben, aufweisen; insbesondere von nicht mehr als 150 um; insbesondere von nicht mehr als 100 um; und insbesondere von nicht mehr als 75 um.

11. Verfahren nach Anspruch 10, wobei das Verfahren Folgendes umfasst:
(a1) Herstellen der ersten Vielzahl von Doxylamin-Pellets modifizierter Freisetzung oder eines pharmazeutisch annehmbaren Salzes davon durch Beschichten der Doxylamin-Pellets oder eines pharmazeutisch annehmbaren Salzes davon mit der inneren aktiven Überzugsschicht und gegebenenfalls der magensaftresistenten Überzugszwischenschicht durch kontinuierliches oder diskontinuierliches Sprühen einer flüssigen Mischung, umfassend das eine oder die mehreren magensaftresistenten Überzugsmittel, das eine oder die mehreren Überzugsmittel modifizierter Freisetzung und gegebenenfalls einen oder mehrere pharmazeutisch annehmbare Hilfsstoffe; und
(b1) Herstellen der zweiten Vielzahl von Pyridoxin-Pellets modifizierter Freisetzung oder eines pharmazeutisch annehmbaren Salzes davon durch Beschichten der Pyridoxin-Pellets oder eines pharmazeutisch annehmbaren Salzes davon mit der inneren aktiven Überzugsschicht durch kontinuierliches oder diskontinuierliches Sprühen einer flüssigen Mischung, umfassend das eine oder die mehreren magensaftresistenten Überzugsmittel, das eine oder die mehreren Überzugsmittel modifizierter Freisetzung und gegebenenfalls einen oder mehrere pharmazeutisch annehmbare Hilfsstoffe;
oder alternativ
(a1) Herstellen der ersten Vielzahl von Doxylamin-Pellets modifizierter Freisetzung oder eines pharmazeutisch annehmbaren Salzes davon durch Beschichten der Doxylamin-Pellets oder eines pharmazeutisch annehmbaren Salzes davon mit der inneren aktiven Überzugsschicht und gegebenenfalls der magensaftresistenten Überzugszwischenschicht durch kontinuierliches oder diskontinuierliches Sprühen einer flüssigen Mischung, umfassend das eine oder die mehreren magensaftresistenten Überzugsmittel, das eine oder die mehreren Überzugsmittel modifizierter Freisetzung und gegebenenfalls einen oder mehrere pharmazeutisch annehmbare Hilfsstoffe; und gleichzeitiges oder abwechselndes Hinzufügen einer Mischung in Pulverform, umfassend das eine oder die mehreren Antibackmittel, gegebenenfalls einen oder mehrere Porenbildner und gegebenenfalls einen oder mehrere pharmazeutisch annehmbare Hilfsstoffe; und
(b1) Herstellen der zweiten Vielzahl von Pyridoxin-Pellets modifizierter Freisetzung oder eines pharmazeutisch annehmbaren Salzes davon durch Beschichten der Pyridoxin-Pellets oder eines pharmazeutisch annehmbaren Salzes davon mit der inneren aktiven Überzugsschicht durch kontinuierliches oder diskontinuierliches Sprühen einer flüssigen Mischung, umfassend das eine oder die mehreren magensaftresistenten Überzugsmittel, das eine oder die mehreren Überzugsmittel modifizierter Freisetzung und gegebenenfalls einen oder mehrere pharmazeutisch annehmbare Hilfsstoffe; und gleichzeitiges oder abwechselndes Hinzufügen einer Mischung in Pulverform, umfassend das eine oder die mehreren Antibackmittel, gegebenenfalls einen oder mehrere Porenbildner und gegebenenfalls einen oder mehrere pharmazeutisch annehmbare Hilfsstoffe.

12. Verfahren nach Anspruch 11, umfassend:
(a1) Herstellen der ersten Vielzahl von Doxylamin-Pellets modifizierter Freisetzung oder eines pharmazeutisch annehmbaren Salzes davon durch Beschichten der Doxylamin-Pellets oder eines pharmazeutisch annehmbaren Salzes davon mit der inneren aktiven Überzugsschicht und gegebenenfalls der magensaftresistenten Überzugszwischenschicht durch Hinzufügen des einen oder der mehreren magensaftresistenten Überzugsmittel, des einen oder der mehreren Überzugsmittel modifizierter Freisetzung, des einen oder der mehreren Antibackmittel, gegebenenfalls eines oder mehrerer Porenbildner und gegebenenfalls eines oder mehrerer pharmazeutisch annehmbarer Hilfsstoffe, wobei: die Summe der magensaftresistenten Überzugsmittel und der Überzugsmittel modifizierter Freisetzung in der Sprühflüssigkeitsmischung 10 bis 49 Gewichtsprozent ist, bezogen auf das Gewicht der flüssigen Mischung, und (b1) Herstellen der zweiten Vielzahl von Pyridoxin-Pellets modifizierter Freisetzung oder eines pharmazeutisch annehmbaren Salzes davon durch Beschichten der Pyridoxin-Pellets oder eines pharmazeutisch annehmbaren Salzes davon mit der inneren aktiven Überzugsschicht durch Hinzufügen des einen oder der mehreren magensaftresistenten Überzugsmittel, des einen oder der mehreren Überzugsmittel modifizierter Freisetzung, des einen oder der mehreren Antibackmittel, gegebenenfalls des eine oder der mehreren Porenbildner und gegebenenfalls eines oder mehrere pharmazeutisch annehmbarer Hilfsstoffe, wobei: die Summe der magensaftresistenten Überzugsmittel und der Überzugsmittel modifizierter Freisetzung in der Sprühflüssigkeitsmischung 10 bis 49 Gewichtsprozent, bezogen auf das Gewicht der Flüssigkeitsmischung, ist.

13. Verfahren nach einem der Ansprüche 11 oder 12, wobei das Verfahren Folgendes umfasst:
(a1) Herstellen der ersten Vielzahl von Doxylamin-Pellets modifizierter Freisetzung oder eines pharmazeutisch annehmbaren Salzes davon durch Beschichten der Doxylamin-Pellets oder eines pharmazeutisch annehmbaren Salzes davon mit der inneren aktiven Überzugsschicht und gegebenenfalls der magensaftresistenten Überzugszwischenschicht durch kontinuierliches oder diskontinuierliches Sprühen einer flüssigen Mischung, umfassend von 1,0 bis 7,5 Gewichtsprozent des einen oder der mehreren magensaftresistenten Überzugsmittel, 10,0 bis 35,0 Gewichtsprozent des einen oder der mehreren Überzugsmittel modifizierter Freisetzung in einem Gewichtsverhältnis untereinander von 5:95 bis 30:70 und gegebenenfalls einen oder mehrere pharmazeutisch annehmbare Hilfsstoffe; und gleichzeitiges oder abwechselndes Hinzufügen einer Mischung in Pulverform, umfassend das eine oder die mehreren Antibackmittel, gegebenenfalls einen oder die mehreren Porenbildner und gegebenenfalls einen oder mehrere pharmazeutisch annehmbare Hilfsstoffe,
wobei: die durchschnittliche Sprühflussrate der Mischung, umfassend die Beschichtung, 0,30 bis 5,00 g/Min pro kg pharmazeutisch annehmbarer inerter Keime ist; die durchschnittliche Feststoffzugaberate der Mischung in fester Form 0,05 bis 1,50 g/Min pro kg pharmazeutisch annehmbarer inerter Keime ist; und die Beziehung zwischen der durchschnittlichen Sprühflussrate der Mischung, umfassend die Überzugsmittel, und der durchschnittlichen Feststoffzugaberate der Mischung in fester Form 90:10 bis 60:40, insbesondere 90:10 bis 70:30 ist; und
(b1) Herstellen der zweiten Vielzahl von Pyridoxin-Pellets modifizierter Freisetzung oder eines pharmazeutisch annehmbaren Salzes davon durch Beschichten der Pyridoxin-Pellets oder eines pharmazeutisch annehmbaren Salzes davon mit der inneren aktiven Überzugsschicht durch kontinuierliches oder diskontinuierliches Versprühen einer flüssigen Mischung, umfassend 1,0 bis 7,5 Gewichtsprozent des einen oder der mehreren magensaftresistenten Überzugsmittel, 10,0 bis 35,0 Gewichtsprozent des einen oder der mehreren Überzugsmittel modifizierter Freisetzung in einem Gewichtsverhältnis von 5:95 bis 30:70 und gegebenenfalls einen oder mehrere pharmazeutisch annehmbare Hilfsstoffe; und gleichzeitiges oder abwechselndes Hinzufügen einer Mischung in Pulverform, umfassend ein oder mehrere Antibackmittel, gegebenenfalls einen oder mehrere Porenbildner und gegebenenfalls einen oder mehrere pharmazeutisch annehmbare Hilfsstoffe,
wobei: die durchschnittliche Sprühflussrate der Mischung, umfassend die Überzugsmittel, 0,30 bis 5,00 g/Min pro kg pharmazeutisch annehmbarer inerter Keime ist; die durchschnittliche Feststoffzugaberate der Mischung in fester Form 0,10 bis 2,25 g/Min pro kg pharmazeutisch annehmbarer inerter Keime ist; und die Beziehung zwischen der durchschnittlichen Sprühflussrate der Mischung, umfassend die Überzugsmittel, und der durchschnittlichen Feststoffzugaberate der Mischung in fester Form 90:10 bis 60:40, insbesondere 80:20 bis 60:40 ist.

14. Verfahren nach einem der Ansprüche 10 bis 13, wobei das Verfahren ferner einen vorherigen Schritt des separaten Beschichtens von Folgendem umfasst:
gegebenenfalls (a2) der Doxylamin-Pellets oder eines pharmazeutisch annehmbaren Salzes davon mit der inneren aktiven Überzugsschicht durch kontinuierliches oder diskontinuierliches Sprühen einer flüssigen Mischung, umfassend das eine oder die mehreren magensaftresistenten Überzugsmittel und gegebenenfalls einen oder mehrere pharmazeutisch annehmbare Hilfsstoffe; und
(b2) des pharmazeutisch annehmbaren inerten Kerns durch kontinuierliches oder diskontinuierliches Versprühen einer flüssigen Mischung, umfassend ein oder mehrere Überzugsmittel, die therapeutisch wirksame Menge an Pyridoxin oder ein pharmazeutisch annehmbares Salz davon und gegebenenfalls einen oder mehrere pharmazeutisch annehmbare Hilfsstoffe; oder alternativ,
gegebenenfalls (a2) der Doxylamin-Pellets oder eines pharmazeutisch annehmbaren Salzes davon mit der inneren aktiven Überzugsschicht durch kontinuierliches oder diskontinuierliches Versprühen einer flüssigen Mischung, umfassend das eine oder die mehreren magensaftresistenten Überzugsmittel und gegebenenfalls einen oder mehrere pharmazeutisch annehmbare Hilfsstoffe; und gleichzeitiges oder abwechselndes Hinzufügen einer Mischung in fester Form, umfassend das eine oder die mehreren Antibackmittel, gegebenenfalls den einen oder die mehreren Porenbildner und gegebenenfalls einen oder mehrere pharmazeutisch annehmbare Hilfsstoffe; und
(b2) des pharmazeutisch annehmbaren inerten Kerns durch kontinuierliches oder diskontinuierliches Versprühen einer flüssigen Mischung, umfassend ein oder mehrere Überzugsmittel und gegebenenfalls einen oder mehrere pharmazeutisch annehmbare Hilfsstoffe; und gleichzeitiges oder abwechselndes Hinzufügen der therapeutisch wirksamen Menge an Pyridoxin oder eines pharmazeutisch annehmbaren Salzes davon und gegebenenfalls eines oder mehrerer pharmazeutisch annehmbarer Hilfsstoffe in Pulverform.

15. Verfahren nach Anspruch 14, wobei das Verfahren Folgendes umfasst:
gegebenenfalls (a2) die Doxylamin-Pellets oder einem pharmazeutisch annehmbaren Salz davon mit der inneren aktiven Überzugsschicht durch kontinuierliches oder diskontinuierliches Versprühen einer flüssigen Mischung, umfassend 5 bis 15 Gewichtsprozent des einen oder der mehreren magensaftresistenten Überzugsmittel und gegebenenfalls einen oder mehrere pharmazeutisch annehmbare Hilfsstoffe; und gleichzeitiges oder abwechselndes Hinzufügen einer Mischung in Pulverform von 5,0 bis 6,5 g pro kg pharmazeutisch annehmbarer inerter Keime der Mischung in fester Form, umfassend das eine oder die mehreren Antibackmittel, gegebenenfalls den einen oder die mehreren Porenbildner und gegebenenfalls einen oder mehrere pharmazeutisch annehmbare Hilfsstoffe; wobei: der Durchschnitt der Sprühflussrate der Mischung, umfassend die magensaftresistenten Überzugsmittel, 0,30 bis 3,00 g/Min pro kg pharmazeutisch annehmbarer inerter Keime ist; der Durchschnitt der Feststoffzugaberate der Mischung in fester Form 0,025 bis 0,400 g/Min pro kg pharmazeutisch annehmbarer inerter Keime ist; und die Beziehung zwischen der durchschnittlichen Sprühflussrate der Mischung, umfassend die Beschichtung und der durchschnittlichen Feststoffzugaberate der Mischung in fester Form 85:15 bis 95:5 ist; und
(b2) den pharmazeutisch annehmbaren inerten Kern mit einem gleichzeitigen oder abwechselnden Sprühen einer flüssigen Mischung, umfassend 20 bis 45 Gewichtsprozent eines oder mehrerer Überzugsmittel und gegebenenfalls einen oder mehrere pharmazeutisch annehmbare Hilfsstoffe; und gleichzeitiges oder abwechselndes Hinzufügen der therapeutisch wirksamen Menge an Pyridoxin oder eines pharmazeutisch annehmbaren Salzes davon und gegebenenfalls eines oder mehrerer pharmazeutisch annehmbarer Hilfsstoffe in Pulverform: wobei die durchschnittliche Sprühflussrate der Mischung, die die Überzugsmittel umfasst, 0,30 bis 4,50 g/Min pro kg pharmazeutisch annehmbarer inerter Kerne ist; die durchschnittliche Feststoffzugaberate des Pulvers 0,50 bis 9,00 g/Min pro kg pharmazeutisch annehmbarer inerter Kerne ist; und die Beziehung zwischen der durchschnittlichen Sprühflussrate der Mischung, die die Überzugsmittel umfasst, und der durchschnittlichen Feststoffzugaberate 25:75 bis 40:60 ist.

16. Verfahren nach einem der Ansprüche 14 oder 15, wobei das Verfahren ferner einen zusätzlichen Schritt umfasst, der Folgendes umfasst:
(a3) Beschichten des pharmazeutisch annehmbaren inerten Kerns mit einem kontinuierlichen oder diskontinuierlichen Sprühen einer flüssigen Mischung, umfassend ein oder mehrere Überzugsmittel, die therapeutisch wirksame Menge an Pyridoxin oder ein pharmazeutisch annehmbares Salz davon und gegebenenfalls einen oder mehrere pharmazeutisch annehmbare Hilfsstoffe; oder alternativ,
(a3) Beschichten des pharmazeutisch annehmbaren inerten Kerns mit einem kontinuierlichen oder diskontinuierlichen Sprühen einer flüssigen Mischung, umfassend ein oder mehrere Überzugsmittel und gegebenenfalls einen oder mehrere pharmazeutisch annehmbare Hilfsstoffe; und gleichzeitiges oder abwechselndes Hinzufügen einer Mischung in Pulverform, umfassend die therapeutisch wirksame Menge an Doxylamin oder ein pharmazeutisch annehmbares Salz davon, einen oder mehrere Antibackmittel, gegebenenfalls einen oder mehrere Porenbildner und gegebenenfalls einen oder mehrere pharmazeutisch annehmbare Hilfsstoffe.

17. Verfahren nach Anspruch 16, wobei das Verfahren Folgendes umfasst:
(a3) Beschichten des pharmazeutisch annehmbaren inerten Kerns mit einem kontinuierlichen oder diskontinuierlichen Sprühen einer flüssigen Mischung, umfassend 15 bis 40 Gewichtsprozent eines oder mehrerer Überzugsmittel und gegebenenfalls einen oder mehrere pharmazeutisch annehmbare Hilfsstoffe; und gleichzeitiges oder abwechselndes Hinzufügen einer Mischung in Pulverform, umfassend die therapeutisch wirksame Menge an Doxylamin oder ein pharmazeutisch annehmbares Salz davon, 18 bis 36 Gewichtsprozent des einen oder der mehreren Antibackmittel, gegebenenfalls einen oder mehrere Porenbildner und gegebenenfalls einen oder mehrere pharmazeutisch annehmbare Hilfsstoffe;
wobei: die durchschnittliche Sprühflussrate der Mischung, umfassend die Überzugsmittel, 0,30 bis 4,50 g/Min pro kg pharmazeutisch annehmbarer inerter Keime ist; die durchschnittliche Feststoffzugaberate der Mischung in Pulverform 0,95 bis 18,00 g/Min pro kg pharmazeutisch annehmbarer inerter Keime ist; und die Beziehung zwischen der durchschnittlichen Sprühflussrate der Mischung, die die Überzugsmittel umfasst, und der durchschnittlichen Feststoffzugaberate der Mischung in Pulverform 15:85 bis 30:70 ist.

18. Verfahren nach einem der Ansprüche 10 bis 17, wobei in jedem der Sprühschritte (a1), (a2), (a3), (b1) und (b2) die flüssige Mischung bei einem Pistolenzerstäubungsdruck von 0,6 bis 2,2 bar und einem Druck mit offenem Muster von 0,6 bis 2,5 bar gesprüht wird.

## Revendications

1. Forme posologique par voie orale à unité multiples à libération modifiée comprenant :
une première pluralité de pastilles à libération modifiée de doxylamine ou son sel pharmaceutiquement acceptable comprenant :
- un noyau inerte pharmaceutiquement acceptable ;
- une couche de revêtement interne active comprenant une quantité thérapeutiquement efficace de doxylamine ou son sel pharmaceutiquement acceptable, un ou plusieurs agents de revêtement, un ou plusieurs agents anti-agglomérants, éventuellement un ou plusieurs agents porogènes ; et éventuellement un ou plusieurs excipients pharmaceutiquement acceptables ;
- éventuellement une couche de revêtement à libération entérique intermédiaire comprenant un ou plusieurs agents de revêtement entérique, un ou plusieurs agents anti-agglomérants, éventuellement un ou plusieurs agents porogènes ; et éventuellement un ou plusieurs excipients pharmaceutiquement acceptables, et
- une couche de revêtement à libération modifiée externe comprenant un ou plusieurs agents de revêtement entérique, un ou plusieurs agents de revêtement à libération modifiée, un ou plusieurs agents anti-agglomérants, éventuellement un ou plusieurs agents porogènes, et éventuellement un ou plusieurs excipients pharmaceutiquement acceptables ; et
une seconde pluralité de pastilles à libération modifiée de pyridoxine ou son sel pharmaceutiquement acceptable comprenant :
- un noyau inerte pharmaceutiquement acceptable ;
- une couche de revêtement interne active comprenant une quantité thérapeutiquement efficace de pyridoxine ou son sel pharmaceutiquement acceptable, un ou plusieurs agents de revêtement, et éventuellement un ou plusieurs excipients pharmaceutiquement acceptables ; et
- une couche de revêtement à libération modifiée externe comprenant un ou plusieurs agents de revêtement entérique, un ou plusieurs agents de revêtement à libération modifiée, éventuellement un ou plusieurs agents porogènes, éventuellement un ou plusieurs excipients pharmaceutiquement acceptables ;
dans laquelle :
la taille de particule du noyau inerte pharmaceutiquement acceptable de la première et de la seconde pluralité de pastilles est telle qu'au moins 90 % des noyaux inertes ont une taille de particule de 300 um à 1700 µm mesurée par tamisage analytique et qu'au moins 90 % des noyaux inertes ont une variabilité de taille de particule non supérieure à 200 um à partir d'une valeur donnée comprise entre 500 µm et 1400 µm mesurée par tamisage analytique ;
dans laquelle la variabilité de taille de particule signifie qu'à partir d'une valeur donnée, au moins 90 % des noyaux inertes pharmaceutiquement acceptables de la première et de la seconde pluralité de pastilles ont une taille de particule comprise à partir de ± 200 um à partir de la valeur donnée.

2. Forme posologique par voie orale à unité multiples à libération modifiée selon la revendication 1, dans laquelle la première pluralité de pastilles à libération modifiée de doxylamine ou son sel pharmaceutiquement acceptable comprend la couche de revêtement à libération entérique intermédiaire comprenant un ou plusieurs agents de revêtement entérique, un ou plusieurs agents anti-agglomérants, éventuellement un ou plusieurs agents porogènes ; et éventuellement un ou plusieurs excipients pharmaceutiquement acceptables.

3. Forme posologique par voie orale à unité multiples à libération modifiée selon l'une quelconque des revendications 1 ou 2, dans laquelle :
la forme posologique comprend un noyau inerte pharmaceutiquement acceptable de la première et de la seconde pluralité de pastilles ayant une taille de particule telle qu'au moins 90 % des noyaux inertes ont une taille de particule de 300 µm à 1400 um mesurée par tamisage analytique, et au moins 90 % des noyaux inertes pharmaceutiquement acceptables ont une variabilité de taille de particule non supérieure à 150 um à partir d'une valeur donnée comprise entre 450 µm et 1250 um mesurée par tamisage analytique ; dans laquelle la variabilité de taille de particule signifie qu'à partir d'une valeur donnée, au moins 90 % des noyaux inertes pharmaceutiquement acceptables de la première et de la seconde pluralité de pastilles ont une taille de particule comprise entre ± 150 um à partir de la valeur donnée ;
en particulier, la forme posologique comprend un noyau inerte pharmaceutiquement acceptable de la première et de la seconde pluralité de pastilles ayant une taille de particule telle qu'au moins 90 % des noyaux inertes ont une taille de particule de 600 µm à 1180 um mesurée par tamisage analytique, et au moins 90 % des noyaux inertes pharmaceutiquement acceptables ont une variabilité de taille de particule non supérieure à 100 um à partir d'une valeur donnée comprise entre 710 µm et 1000 µm mesurée par tamisage analytique ; dans laquelle la variabilité de taille de particule signifie qu'à partir d'une valeur donnée, au moins 90 % des noyaux inertes pharmaceutiquement acceptables de la première et de la seconde pluralité de pastilles ont une taille de particule comprise entre ± 100 um à partir de la valeur donnée ; et
en particulier la forme posologique comprend un noyau inerte pharmaceutiquement acceptable de la première et de la seconde pluralité de pastilles ayant une taille de particule telle qu'au moins 90 % des noyaux inertes ont une taille de particule de 710 µm à 1000 um mesurée par tamisage analytique et au moins 90 % des noyaux inertes pharmaceutiquement acceptables ont une variabilité de taille de particule non supérieure à 75 um à partir d'une valeur donnée comprise entre 800 µm et 900 um mesurée par tamisage analytique ; dans laquelle la variabilité de taille de particule signifie qu'à partir d'une valeur donnée, au moins 90 % des noyaux inertes pharmaceutiquement acceptables de la première et de la seconde pluralité de pastilles ont une taille de particule comprise entre ± 75 um à partir de la valeur donnée.

4. Forme posologique par voie orale à unité multiples à libération modifiée selon l'une quelconque des revendications 1 à 3, dans laquelle :
la taille de particule des pastilles de la première pluralité de pastilles à libération modifiée de doxylamine ou son sel pharmaceutiquement acceptable est telle qu'au moins 90 % des pastilles ont une taille de particule de 400 µm à 2000 um mesurée par tamisage analytique et au moins 90 % des pastilles ont une variabilité de taille de particule non supérieure à 200 um à partir d'une valeur donnée comprise entre 600 µm et 1800 um mesurée par tamisage analytique ; dans laquelle la variabilité de taille de particule signifie qu'à partir d'une valeur donnée, l'au moins 90 % de la première pluralité de pastilles à libération modifiée ont une taille de particule de ± 200 um à partir de la valeur donnée ; en particulier, la taille de particule des pastilles de la première pluralité de pastilles à libération modifiée de doxylamine ou son sel pharmaceutiquement acceptable est telle qu'au moins 90 % des pastilles ont une taille de particule de 600 µm à 1600 um mesurée par tamisage analytique et au moins 90 % des pastilles ont une variabilité de taille de particule non supérieure à 150 um à partir d'une valeur donnée comprise entre 800 µm et 1400 um mesurée par tamisage analytique ;
dans laquelle la variabilité de taille de particule signifie qu'à partir d'une valeur donnée, l'au moins 90 % de la première pluralité de pastilles à libération modifiée ont une taille de particule comprise entre ± 150 um à partir de la valeur donnée ;
en particulier, la taille de particule des pastilles de la première pluralité de pastilles à libération modifiée de doxylamine ou son sel pharmaceutiquement acceptable est telle qu'au moins 90 % des pastilles ont une taille de particule de 710 µm à 1400 um mesurée par tamisage analytique et au moins 90 % des pastilles ont une variabilité de taille de particule non supérieure à 100 um à partir d'une valeur donnée comprise entre 850 µm et 1250 µm mesurée par tamisage analytique ; dans laquelle la variabilité de taille de particule signifie qu'à partir d'une valeur donnée, l'au moins 90 % de la première pluralité de pastilles à libération modifiée ont une taille de particule comprise entre ± 100 um à partir de la valeur donnée ; et
en particulier, la taille de particule des pastilles de la première pluralité de pastilles à libération modifiée de doxylamine ou son sel pharmaceutiquement acceptable est telle qu'au moins 90 % des pastilles ont une taille de particule de 800 µm à 1400 um mesurée par tamisage analytique et au moins 90 % des pastilles ont une variabilité de taille de particule non supérieure à 75 um à partir d'une valeur donnée comprise entre 900 µm et 1180 um mesurée par tamisage analytique ; dans laquelle la variabilité de taille de particule signifie qu'à partir d'une valeur donnée, l'au moins 90 % de la première pluralité de pastilles à libération modifiée ont une taille de particule comprise entre ± 75 um à partir de la valeur donnée ; et
la taille de particule des pastilles de la seconde pluralité de pastilles à libération modifiée de pyridoxine ou son sel pharmaceutiquement acceptable est telle qu'au moins 90 % des pastilles ont une taille de particule de 400 µm à 2000 um mesurée par tamisage analytique et au moins 90 % des pastilles ont une variabilité de taille de particule non supérieure à 200 µm à partir d'une valeur donnée comprise entre 600 µm et 1800 µm mesurée par tamisage analytique ;
dans laquelle la variabilité de taille de particule signifie qu'à partir d'une valeur donnée, l'au moins 90 % de la seconde pluralité de pastilles à libération modifiée ont une taille de particule comprise entre ± 200 um à partir de la valeur donnée ;
en particulier, la taille de particule des pastilles de la seconde pluralité de pastilles à libération modifiée de pyridoxine ou son sel pharmaceutiquement acceptable est telle qu'au moins 90 % des pastilles ont une taille de particule de 600 µm à 1600 µm mesurée par tamisage analytique et au moins 90 % des pastilles ont une variabilité de taille de particule non supérieure à 150 µm à partir d'une valeur donnée comprise entre 800 µm et 1400 um mesurée par tamisage analytique ; dans laquelle la variabilité de taille de particule signifie qu'à partir d'une valeur donnée, l'au moins 90 % de la seconde pluralité de pastilles à libération modifiée ont une taille de particule comprise entre ± 150 um à partir de la valeur donnée ;
en particulier, la taille de particule des pastilles de la seconde pluralité de pastilles à libération modifiée de pyridoxine ou son sel pharmaceutiquement acceptable est telle qu'au moins 90 % des pastilles ont une taille de particule de 710 µm à 1400 um mesurée par tamisage analytique et au moins 90 % des pastilles ont une variabilité de taille de particule non supérieure à 100 um à partir d'une valeur donnée comprise entre 850 µm et 1250 um mesurée par tamisage analytique ; dans laquelle la variabilité de taille de particule signifie qu'à partir d'une valeur donnée, l'au moins 90 % de la seconde pluralité de pastilles à libération modifiée ont une taille de particule comprise entre ± 100 um à partir de la valeur donnée ; et
en particulier, la taille de particule des pastilles de la seconde pluralité de pastilles à libération modifiée de pyridoxine ou son sel pharmaceutiquement acceptable est telle qu'au moins 90 % des pastilles ont une taille de particule de 800 µm à 1400 um mesurée par tamisage analytique et au moins 90 % des pastilles ont une variabilité de taille de particule non supérieure à 75 um à partir d'une valeur donnée comprise entre 900 µm et 1180 µm mesurée par tamisage analytique ; dans laquelle la variabilité de taille de particule signifie qu'à partir d'une valeur donnée, l'au moins 90 % de la seconde pluralité de pastilles à libération modifiée ont une taille de particule comprise entre ± 75 um à partir de la valeur donnée.

5. Forme posologique par voie orale à unité multiples à libération modifiée selon l'une quelconque des revendications 1 à 4, dans laquelle :
la taille de particule de doxylamine ou son sel pharmaceutiquement acceptable est caractérisée pour avoir un D90 égal ou inférieur à 250 um ; et la taille de particule de pyridoxine ou son sel pharmaceutiquement acceptable est **caractérisée par** le fait d'avoir un D90 égal ou inférieur à 250 µm ;
ou alternativement,
la taille de particule de doxylamine ou son sel pharmaceutiquement acceptable est caractérisée pour avoir un D90 égal ou inférieur à 250 um ; et la taille de particule de pyridoxine ou son sel pharmaceutiquement acceptable est caractérisée pour avoir un D90 égal ou inférieur à 250 um ;
la taille de particule d'un ou plusieurs agents anti-agglomérants est **caractérisée par** le fait d'avoir un D90 égal ou inférieur à 250 um
dans laquelle
le D90 de la taille de particule de doxylamine ou son sel pharmaceutiquement acceptable est exprimé en volume ; le D90 de la taille de particule de pyridoxine ou son sel pharmaceutiquement acceptable est exprimé en volume ; et le D90 de la taille de particule des agents anti-agglomérants est exprimé en poids.

6. Forme posologique par voie orale à unité multiples à libération modifiée selon l'une quelconque des revendications 1 à 5, qui comprend un sel pharmaceutiquement acceptable de doxylamine et un sel pharmaceutiquement acceptable de pyridoxine ; de préférence, comprend du succinate de doxylamine et du chlorhydrate de pyridoxine.

7. Forme posologique par voie orale à unité multiples à libération modifiée selon l'une quelconque des revendications 1 à 6, qui comprend :
de 5 mg à 50 mg par forme posologique par voie orale de doxylamine ou son sel pharmaceutiquement acceptable ; et
de 5 mg à 50 mg par forme posologique par voie orale de pyridoxine ou son sel pharmaceutiquement acceptable ;
en particulier,
de 10 mg à 20 mg par forme posologique par voie orale de doxylamine ou son sel pharmaceutiquement acceptable ; et
de 10 mg à 20 mg par forme posologique par voie orale de pyridoxine ou son sel pharmaceutiquement acceptable.

8. Forme posologique par voie orale à unité multiples à libération modifiée selon l'une quelconque des revendications 1 à 7,
dans laquelle la forme posologique par voie orale à unité multiples à libération modifiée présente un profil de dissolution selon lequel :
de 5 % à 35 % en poids de la teneur en doxylamine sont dissous à la 1^{ère} heure dans un milieu HCI de 0,1 N (pH = 1) ;
puis, le milieu est remplacé par un milieu à pH = 4,5 (tampon acétate de 0,05 M) et à la 4^{ème} heure à partir de plus de 35 % à 75 % en poids de la teneur initiale en doxylamine accumulée sont dissous ;
puis, le milieu est remplacé par un milieu à pH = 6,8 (tampon phosphate de 0,05 M) et à la 7^{ème} heure au moins plus de 75 % en poids de la teneur initiale en doxylamine accumulée sont dissous ; et
de 5 % à 35 % en poids de la teneur en pyridoxine sont dissous à la 1^{ère} heure dans un milieu HCI de 0,1 N (pH = 1) ;
puis, le milieu est remplacé par un milieu à pH = 4,5 (tampon acétate de 0,05 M) et à la 4^{ème} heure à partir de plus de 35 % à 75 % en poids de la teneur initiale de pyridoxine accumulée sont dissous ;
puis, le milieu est remplacé par un milieu à pH = 6,8 (tampon phosphate de 0,05 M) et à la 7^{ème} heure, au moins plus de 75 % en poids de la teneur initiale en pyridoxine sont dissous ;
dans laquelle le profil de dissolution est mesuré à l'aide d'un appareil USP de type 2 (panier), en plaçant la composition dans 900 ml du milieu correspondant/ tamponné à 37 °C ± 0,5 °C et 100 tr/min.

9. Forme posologique par voie orale à unité multiples à libération modifiée selon l'une quelconque des revendications 1 à 8, qui est une capsule ; en particulier une capsule dure ; plus particulièrement une capsule dure est choisie dans le groupe constitué d'une capsule de gélatine dure et d'une capsule d'hydroxypropylméthylcellulose dure.

10. Procédé pour la préparation de la forme posologique par voie orale à unité multiples à libération modifiée telle que définie dans l'une quelconque des revendications 1 à 9, comprenant les étapes consistant à :
(a1) préparer la première pluralité de pastilles à libération modifiée de doxylamine ou son sel pharmaceutiquement acceptable en enrobant les pastilles de doxylamine ou son sel pharmaceutiquement acceptable ayant la couche de revêtement interne active et éventuellement la couche de revêtement entérique intermédiaire en ajoutant l'un ou plusieurs agents de revêtement entérique, l'un ou plusieurs agents de revêtement à libération modifiée, l'un ou plusieurs agents anti-agglomérants, éventuellement un ou plusieurs agents porogènes, et éventuellement un ou plusieurs excipients pharmaceutiquement acceptables, dans laquelle la taille de particule du noyau inerte pharmaceutiquement acceptable est telle qu'au moins 90 % des noyaux inertes ont une taille de particule de 300 µm à 1700 µm mesurée par tamisage analytique et au moins 90 % des noyaux inertes ont une variabilité de taille de particule non supérieure à 200 um mesurée par tamisage analytique ; en particulier non supérieure à 150 um ; en particulier non supérieure à 100 um ; et en particulier non supérieure à 75 µm ; et
(b1) préparer la seconde pluralité de pastilles à libération modifiée de pyridoxine ou son sel pharmaceutiquement acceptable en enrobant les pastilles de pyridoxine ou son sel pharmaceutiquement acceptable ayant la couche de revêtement interne active en ajoutant l'un ou plusieurs agents de revêtement entérique, l'un ou plusieurs agents de revêtement à libération modifiée, éventuellement l'un ou plusieurs agents porogènes, et éventuellement un ou plusieurs excipients pharmaceutiquement acceptables, dans laquelle la taille de particule du noyau inerte pharmaceutiquement acceptable est telle qu'au moins 90 % des noyaux inertes ont une taille de particule de 300 µm à 1700 µm mesurée par tamisage analytique et au moins 90 % des noyaux inertes ont une variabilité de taille de particule non supérieure à 200 um mesurée par tamisage analytique ; en particulier non supérieure à 150 um ; en particulier non supérieure à 100 um ; et en particulier non supérieure à 75 µm.

11. Procédé selon la revendication 10, dans lequel le procédé comprend :
(a1) préparer la première pluralité de pastilles à libération modifiée de doxylamine ou son sel pharmaceutiquement acceptable en enrobant les pastilles de doxylamine ou son sel pharmaceutiquement acceptable ayant la couche de revêtement interne active et éventuellement la couche de revêtement entérique intermédiaire en pulvérisant de manière continue ou discontinue un mélange liquide comprenant l'un ou plusieurs agents de revêtement entérique, l'un ou plusieurs agents de revêtement à libération modifiée et éventuellement un ou plusieurs excipients pharmaceutiquement acceptables ; et
(b1) préparer la seconde pluralité de pastilles à libération modifiée de pyridoxine ou son sel pharmaceutiquement acceptable en enrobant les pastilles de pyridoxine ou son sel pharmaceutiquement acceptable ayant la couche de revêtement interne active en pulvérisant de manière continue ou discontinue un mélange liquide comprenant l'un ou plusieurs agents de revêtement entérique, l'un ou plusieurs agents de revêtement à libération modifiée et éventuellement un ou plusieurs excipients pharmaceutiquement acceptables ;
ou alternativement,
(a1) préparer la première pluralité de pastilles à libération modifiée de doxylamine ou son sel pharmaceutiquement acceptable en enrobant les pastilles de doxylamine ou son sel pharmaceutiquement acceptable ayant la couche de revêtement interne active et éventuellement la couche de revêtement entérique intermédiaire en pulvérisant de manière continue ou discontinue un mélange liquide comprenant l'un ou plusieurs agents de revêtement entérique, l'un ou plusieurs agents de revêtement à libération modifiée et éventuellement un ou plusieurs excipients pharmaceutiquement acceptables ; et ajouter simultanément ou alternativement un mélange sous forme de poudre comprenant l'un ou plusieurs agents anti-agglomérants, éventuellement un ou plusieurs agents porogènes, et éventuellement un ou plusieurs excipients pharmaceutiquement acceptables ; et
(b1) préparer la seconde pluralité de pastilles à libération modifiée de pyridoxine ou son sel pharmaceutiquement acceptable en enrobant les pastilles de pyridoxine ou son sel pharmaceutiquement acceptable ayant la couche de revêtement interne active en pulvérisant de manière continue ou discontinue un mélange liquide comprenant l'un ou plusieurs agents de revêtement entérique, l'un ou plusieurs agents de revêtement à libération modifiée et éventuellement un ou plusieurs excipients pharmaceutiquement acceptables ;
et ajouter simultanément ou alternativement un mélange sous forme de poudre comprenant l'un ou plusieurs agents anti-agglomérants, éventuellement un ou plusieurs agents porogènes, et éventuellement un ou plusieurs excipients pharmaceutiquement acceptables.

12. Procédé selon la revendication 11, comprenant :
(a1) préparer la première pluralité de pastilles à libération modifiée de doxylamine ou son sel pharmaceutiquement acceptable en enrobant les pastilles de doxylamine ou son sel pharmaceutiquement acceptable ayant la couche de revêtement interne active et éventuellement la couche de revêtement entérique intermédiaire en ajoutant l'un ou plusieurs agents de revêtement entérique, l'un ou plusieurs agents de revêtement à libération modifiée, l'un ou plusieurs agents anti-agglomérants, éventuellement un ou plusieurs agents porogènes, et éventuellement un ou plusieurs excipients pharmaceutiquement acceptables, dans lequel : la somme des agents de revêtement entérique et des agents de revêtement à libération modifiée dans le mélange liquide de pulvérisation est de 10 % à 49 % en poids par rapport au poids du mélange liquide, et (b1) préparer la seconde pluralité de pastilles à libération modifiée de pyridoxine ou son sel pharmaceutiquement acceptable en enrobant les pastilles de pyridoxine ou son sel pharmaceutiquement acceptable ayant la couche de revêtement interne active en ajoutant l'un ou plusieurs agents de revêtement entérique, l'un ou plusieurs agents de revêtement à libération modifiée, l'un ou plusieurs agents anti-agglomérants, éventuellement l'un ou plusieurs agents porogènes, et éventuellement un ou plusieurs excipients pharmaceutiquement acceptables, dans lequel : la somme des agents de revêtement entérique et des agents de revêtement à libération modifiée dans le mélange liquide de pulvérisation est de 10 % à 49 % en poids par rapport au poids du mélange liquide.

13. Procédé selon l'une quelconque des revendications 11 ou 12, dans lequel le procédé comprend les étapes consistant à
(a1) préparer la première pluralité de pastilles à libération modifiée de doxylamine ou son sel pharmaceutiquement acceptable en enrobant les pastilles de doxylamine ou son sel pharmaceutiquement acceptable ayant la couche de revêtement interne active et éventuellement la couche de revêtement entérique intermédiaire en pulvérisant de manière continue ou discontinue un mélange liquide comprenant de 1,0 à 7,5 % en poids de l'un ou plusieurs agents de revêtement entérique, de 10,0 à 35,0 % en poids de l'un ou plusieurs agents de revêtement à libération modifiée dans un rapport de poids entre ceux-ci allant de 5:95 à 30:70, et éventuellement un ou plusieurs excipients pharmaceutiquement acceptables ; et ajouter simultanément ou alternativement un mélange sous forme de poudre comprenant l'un ou plusieurs agents anti-agglomérants, éventuellement un ou plusieurs agents porogènes, et éventuellement un ou plusieurs excipients pharmaceutiquement acceptables,
dans lequel : le débit moyen de pulvérisation du mélange comprenant le revêtement est de 0,30 à 5,00 g/min par kg de noyaux inertes pharmaceutiquement acceptables ; la moyenne du taux d'addition solide du mélange sous forme solide est de 0,05 à 1,50 g/min par kg de noyaux inertes pharmaceutiquement acceptables ; et la relation entre le débit moyen de pulvérisation du mélange comprenant les agents de revêtement et la moyenne du taux d'addition solide du mélange sous forme solide est de 90:10 à 60:40, en particulier de 90:10 à 70:30 ; et
(b1) préparer la seconde pluralité de pastilles à libération modifiée de pyridoxine ou son sel pharmaceutiquement acceptable en enrobant les pastilles de pyridoxine ou son sel pharmaceutiquement acceptable ayant la couche de revêtement interne active en pulvérisant de manière continue ou discontinue un mélange liquide comprenant de 1,0 à 7,5 % en poids de l'un ou plusieurs agents de revêtement entérique, de 10,0 à 35,0 % en poids de l'un ou plusieurs agents de revêtement à libération modifiée dans un rapport de poids allant de 5:95 à 30:70 ; et éventuellement un ou plusieurs excipients pharmaceutiquement acceptables ; et ajouter simultanément ou alternativement un mélange sous forme de poudre comprenant un ou plusieurs agents anti-agglomérants, éventuellement un ou plusieurs agents porogènes, et éventuellement un ou plusieurs excipients pharmaceutiquement acceptables,
dans lequel : le débit moyen de pulvérisation du mélange comprenant les agents de revêtement est de 0,30 à 5,00 g/min par kg de noyaux inertes pharmaceutiquement acceptables ; la moyenne du taux d'addition solide du mélange sous forme solide est de 0,10 à 2,25 g/min par kg de noyaux inertes pharmaceutiquement acceptables ; et la relation entre le débit moyen de pulvérisation du mélange comprenant les agents de revêtement et la moyenne du taux d'addition solide du mélange sous forme solide est de 90:10 à 60:40, en particulier de 80:20 à 60:40.

14. Procédé selon l'une quelconque des revendications 10 à 13, dans lequel le procédé comprend en outre une étape précédente consistant à enrober séparément :
éventuellement (a2) les pastilles de doxylamine ou son sel pharmaceutiquement acceptable ayant la couche de revêtement interne active en pulvérisant de manière continue ou discontinue un mélange liquide comprenant l'un ou plusieurs agents de revêtement entérique, et éventuellement un ou plusieurs excipients pharmaceutiquement acceptables ; et
(b2) le noyau inerte pharmaceutiquement acceptable avec une pulvérisation continue ou discontinue d'un mélange liquide comprenant un ou plusieurs agents de revêtement, la quantité thérapeutiquement efficace de pyridoxine ou son sel pharmaceutiquement acceptable, et éventuellement un ou plusieurs excipients pharmaceutiquement acceptables ; ou alternativement,
éventuellement (a2) les pastilles de doxylamine ou son sel pharmaceutiquement acceptable ayant la couche de revêtement interne active en pulvérisant de manière continue ou discontinue un mélange liquide comprenant l'un ou plusieurs agents de revêtement entérique, et éventuellement un ou plusieurs excipients pharmaceutiquement acceptables ; et
ajouter simultanément ou alternativement un mélange sous forme solide comprenant l'un ou plusieurs agents anti-agglomérants, éventuellement l'un ou plusieurs agents porogènes, et éventuellement un ou plusieurs excipients pharmaceutiquement acceptables ; et
(b2) le noyau inerte pharmaceutiquement acceptable en pulvérisant de manière continue ou discontinue un mélange liquide comprenant un ou plusieurs agents de revêtement, et éventuellement un ou plusieurs excipients pharmaceutiquement acceptables ; et ajouter simultanément ou alternativement sous forme de poudre la quantité thérapeutiquement efficace de pyridoxine ou son sel pharmaceutiquement acceptable, et éventuellement un ou plusieurs excipients pharmaceutiquement acceptables.

15. Procédé selon la revendication 14, dans lequel le procédé comprend :
éventuellement (a2) les pastilles de doxylamine ou son sel pharmaceutiquement acceptable ayant la couche de revêtement interne active en pulvérisant de manière continue ou discontinue un mélange liquide comprenant de 5 à 15 % en poids d'un ou plusieurs agents de revêtement entérique, et éventuellement un ou plusieurs excipients pharmaceutiquement acceptables ; et ajouter simultanément ou alternativement un mélange sous forme de poudre de 5,0 à 6,5 g par kg de noyaux inertes pharmaceutiquement acceptables du mélange sous forme solide comprenant l'un ou plusieurs agents anti-agglomérants, éventuellement l'un ou plusieurs agents porogènes, et éventuellement un ou plusieurs excipients pharmaceutiquement acceptables ; dans lequel : la moyenne du débit de pulvérisation du mélange comprenant les agents de revêtement entérique est de 0,30 à 3,00 g/min par kg de noyaux inertes pharmaceutiquement acceptables ; la moyenne du taux d'addition solide du mélange sous forme solide est de 0,025 à 0,400 g/min par kg de noyaux inertes pharmaceutiquement acceptables ; et la relation entre le débit moyen de pulvérisation du mélange comprenant les agents de revêtement et la moyenne du taux d'addition solide du mélange sous forme solide est de 85:15 à 95:5 ; et
(b2) le noyau inerte pharmaceutiquement acceptable avec une pulvérisation simultanée ou alternée d'un mélange liquide comprenant de 20 % à 45 % en poids d'un ou plusieurs agents de revêtement, et éventuellement un ou plusieurs excipients pharmaceutiquement acceptables ; et ajouter simultanément ou alternativement sous forme de poudre la quantité thérapeutiquement efficace de pyridoxine ou son sel pharmaceutiquement acceptable, et éventuellement un ou plusieurs excipients pharmaceutiquement acceptables : dans lequel le débit moyen de pulvérisation du mélange comprenant les agents de revêtement est de 0,30 à 4,50 g/min par kg de noyaux inertes pharmaceutiquement acceptables ; la moyenne du taux d'addition solide de la poudre est de 0,50 à 9,00 g/min par kg de noyaux inertes pharmaceutiquement acceptables ; et la relation entre le débit moyen de pulvérisation du mélange comprenant les agents de revêtement et la moyenne du taux d'addition solide est de 25:75 à 40:60.

16. Procédé selon l'une quelconque des revendications 14 ou 15, dans lequel le procédé comprend en outre une étape supplémentaire qui comprend les étapes consistant à :
(a3) enrober le noyau inerte pharmaceutiquement acceptable avec une pulvérisation continue ou discontinue d'un mélange liquide comprenant un ou plusieurs agents de revêtement, la quantité thérapeutiquement efficace de doxylamine ou son sel pharmaceutiquement acceptable, et éventuellement un ou plusieurs excipients pharmaceutiquement acceptables ; ou alternativement,
(a3) enrober le noyau inerte pharmaceutiquement acceptable avec une pulvérisation continue ou discontinue d'un mélange liquide comprenant un ou plusieurs agents de revêtement, et éventuellement un ou plusieurs excipients pharmaceutiquement acceptables ; et ajouter simultanément ou alternativement un mélange sous forme de poudre comprenant la quantité thérapeutiquement efficace de doxylamine ou son sel pharmaceutiquement acceptable, un ou plusieurs agents anti-agglomérants, éventuellement un ou plusieurs agents porogènes, et éventuellement un ou plusieurs excipients pharmaceutiquement acceptables.

17. Procédé selon la revendication 16, dans lequel le procédé comprend l'étape consistant à :
(a3) enrober le noyau inerte pharmaceutiquement acceptable avec une pulvérisation continue ou discontinue d'un mélange liquide comprenant de 15 % à 40 % en poids d'un ou plusieurs agents de revêtement, et éventuellement un ou plusieurs excipients pharmaceutiquement acceptables ; et ajouter simultanément ou alternativement un mélange sous forme de poudre comprenant la quantité thérapeutiquement efficace de doxylamine ou son sel pharmaceutiquement acceptable, de 18 à 36 % en poids de l'un ou plusieurs agents anti-agglomérants, éventuellement un ou plusieurs agents porogènes, et éventuellement un ou plusieurs excipients pharmaceutiquement acceptables ;
dans lequel : le débit moyen de pulvérisation du mélange comprenant les agents de revêtement est de 0,30 à 4,50 g/min par kg de noyaux inertes pharmaceutiquement acceptables ; la moyenne du taux d'addition solide du mélange sous forme de poudre est de 0,95 à 18,00 g/min par kg de noyaux inertes pharmaceutiquement acceptables ; et la relation entre le débit moyen de pulvérisation du mélange comprenant les agents de revêtement et la moyenne du taux d'addition solide du mélange sous forme de poudre est de 15:85 à 30:70.

18. Procédé selon l'une quelconque des revendications 10 à 17, dans lequel dans chacune des étapes de pulvérisation (a1), (a2), (a3), (b1) et (b2), le mélange liquide est pulvérisé à une pression d'atomisation de pistolet de 0,6 à 2,2 bars et à une pression de motif ouvert de 0,6 à 2,5 bars.
